(19)

Europäisches
Patentamt
European
Patent Office
Office européen
des brevets

(11) **EP 4 497 756 A2**

(12) **EUROPEAN PATENT APPLICATION**

(43) Date of publication:
**29.01.2025 Bulletin 2025/05**

(21) Application number: **24190963.9**

(22) Date of filing: **25.07.2024**

(51) International Patent Classification (IPC):
***C07K 16/26*** (2006.01)    ***A61P 5/00*** (2006.01)

(52) Cooperative Patent Classification (CPC):
**C07K 16/26; A61P 5/00;** C07K 2317/33;
C07K 2317/565; C07K 2317/90; C07K 2317/92

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:  **25.07.2023   CN 202310920447**

(71) Applicants:
- **Shenzhen Mindray Bio-Medical Electronics Co., Ltd.**
  **Shenzhen, Guangdong 518057 (CN)**
- **Hytest Ltd.**
  **20520 Turku (FI)**

(72) Inventors:
- **SEFERIAN, Karina R.**
  **20520 Turku (FI)**
- **KUZICHKINA, Evgenia O.**
  **20520 Turku (FI)**
- **ALTAEVA, Erzhena G.**
  **20520 Turku (FI)**
- **ROZOV, Fedor N.**
  **20520 Turku (FI)**
- **KOZLOVSKY, Stanislav V.**
  **20520 Turku (FI)**
- **LEZHNIN, Yury N.**
  **20520 Turku (FI)**
- **GAYNOVA, Kristina M.**
  **20520 Turku (FI)**

- **AGEEVA, Liudmila V.**
  **20520 Turku (FI)**
- **GRUZDEVA, Natalia A.**
  **20520 Turku (FI)**
- **TAMM, Natalia N.**
  **20520 Turku (FI)**
- **KULIEVA, Elizaveta D.**
  **20520 Turku (FI)**
- **TRUFANOVA, Anna A.**
  **20520 Turku (FI)**
- **KOTLOV, Sergei A.**
  **20520 Turku (FI)**
- **ZHAN, Chengxiong**
  **Shenzhen, 518057 (CN)**
- **SONG, Qixue**
  **Shenzhen, 518057 (CN)**
- **TANG, Tao**
  **Shenzhen, 518057 (CN)**
- **YAN, Yufeng**
  **Shenzhen, 518057 (CN)**
- **LI, Ke**
  **Shenzhen, 518057 (CN)**

(74) Representative: **KIPA AB**
**Drottninggatan 11**
**252 21 Helsingborg (SE)**

Remarks:
The complete document including Reference Table(s) and the Sequence Listing(s) can be downloaded from the EPO website

(54)    **ANTIBODY, KIT, DETECTION METHOD AND SAMPLE ANALYZER FOR DETECTING THYROID STIMULATING HORMONE**

(57)    The present application relates to an antibody, kit, detection method, and sample analyzer for detecting thyroid stimulating hormone. Specifically, the present application relates to a monoclonal antibody or antigen-binding antibody fragment thereof that specifically binds to thyroid stimulating hormone (TSH), wherein the antibody can recognize TSH variant R55G and does not cross-react with an α-subunit shared by human luteinizing hormone (LH), follicle stimulating hormone (FSH), human chorionic gonadotropin (hCG), and free glycoprotein hormones, and is particularly suitable for developing TSH detection kit with a functional sensitivity of ≤0.0015 μIU/ml. The present application also relates to a thyroid stimulating hormone detection kit, detection method, and sample analyzer.

**EP 4 497 756 A2**

## Description

### Technical Field

The present application belongs to the field of molecular immunology, and specifically relates to a thyroid stimulating hormone (TSH)-specific antibody, and a kit, detection method and sample analyzer for detecting TSH.

### Background

Thyroid-dysfunction is common in adults, and mainly includes hypothyroidism and hyperthyroidism. Thyroid stimulating hormone (TSH) in blood is an accurate marker reflecting thyroid function. The normal range of TSH in serum is 0.58 to 4.1 μIU/ml. TSH concentration in blood increases in patients with hypothyroidism and decreases in patients with hyperthyroidism.

Sandwich-immunoassay method is used for the determination of TSH concentration in human serum samples. Most of the current TSH assays are third-generation assays with a functional sensitivity of 0.01 to 0.02 μIU/mL. These assays reliably distinguish patients with hyperthyroidism from those with euthyroidism. However, in some patients, TSH concentration in serum is below the limit of detection of third-generation TSH assay. The fourth-generation TSH assays provide 10-fold increase in the functional sensitivity (0.001 to 0.002 μIU/mL) and therefore make it possible to quantify the concentration of TSH in serum from these patients. Functional sensitivity is determined by the affinity of the antibodies used and the sensitivity that the detection system can provide. For the fourth-generation TSH assays high affinity antibodies are needed which can provide such a high sensitivity.

TSH is a member of glycoprotein hormones family consisting of thyroid-stimulating hormone, luteinizing hormone (LH), follicle-stimulating hormone (FSH), and human chorionic gonadotropin (hCG). TSH is a heterodimer consisting of α- and β-subunits. The α-subunit of TSH is similar to the α-subunits of LH, FSH, and hCG, and the β-subunit is specific for TSH. However, high degree of homology between human TSH, LH, FSH, and CG β-subunit sequences exists. In order to accurately determine the concentration of TSH in serum samples, it is required that the pair of antibodies used in the assays does not recognize other glycoprotein hormones. In particular, for one-step TSH sandwich immunoassay in which capture and detection antibodies are incubated with serum sample simultaneously, both antibodies used in the immunoassay should not have cross-reaction with LH, FSH, and hCG. Therefore, antibodies specific to TSH dimer or TSH β-subunit without cross-reactivity to other glycoprotein hormones should be developed.

To accurately measure TSH concentration in serum, the assay must be able to measure all forms of TSH in the blood. In 2014, a novel point mutation in the TSH β subunit resulting in the replacement of arginine with glycine at position 55 (R55G) was reported (Drees JC et al. Falsely undetectable TSH in a cohort of South Asian euthyroid patients. J Clin Endocrinol Metab. 2014 Apr; 99(4): 1171-9). This mutation altered an epitope on TSH, preventing antibodies specific to that epitope from binding. In patients homozygous for R55G was undetectable by four widely used US Food and Drug Administration (FDA) - approved TSH immunoassays. When using such assays, patients carrying TSH R55G variant cannot be distinguished from patients with subclinical hyperthyroidism. Patients with falsely undetectable TSH levels have been incorrectly diagnosed with hyperthyroidism and treated. There is still a need to develop a kit that can accurately measure TSH in patients with the R55G mutation.

### Summary

An object of the present application is to provide a new monoclonal antibody or antigen-binding antibody fragment thereof that is capable of specifically binding to TSH dimer or TSH β-subunit and does not cross-react with the α-subunit shared by human LH, FSH, CG and free glycoprotein hormone. It is suitable for developing a fourth-generation functionally sensitive TSH assay. Another object of the present application is to provide a monoclonal antibody or antigen-binding antibody fragment thereof that recognizes TSH variant R55G.

Antibody, nucleic acid molecule, expression vector, host cell

Specifically, in one aspect, the present application provides an antibody or antigen-binding fragment thereof that specifically binds to TSH or a mutant thereof, which comprises:

a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or,

a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof.

In some embodiments, the variant comprise an amino acid mutation as compared to the amino acid sequence from which it is derived, and the amino acid mutation is a substitution, deletion or addition of one or several amino acids (e.g., a substitution, deletion or addition of 1, 2, 3, 4 or 5 amino acids). In some embodiments, the substitution is a conservative substitution.

In some embodiments, the variant has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which it is derived.

In some embodiments, the three CDRs comprised in the heavy chain variable region and/or the three CDRs contained in the light chain variable region are defined by the Kabat, Chothia or IMGT numbering system.

In some embodiments, the antibody or antigen-binding fragment thereof comprises:

(1a) a heavy chain variable region comprising the following three CDRs: CDR-H1 having an amino acid sequence of SEQ ID NO: 1 or a variant thereof, CDR-H2 having an amino acid sequence of SEQ ID NO: 2 or a variant thereof, and CDR-H3 having an amino acid sequence of SEQ ID NO: 3 or a variant thereof; and, a light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;

(1b) a heavy chain variable region comprising the following three CDRs: CDR-H1 having an amino acid sequence of SEQ ID NO: 7 or a variant thereof, CDR-H2 having an amino acid sequence of SEQ ID NO: 8 or a variant thereof, and CDR-H3 having an amino acid sequence of SEQ ID NO: 9 or a variant thereof; and, a light chain variable region comprising the following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;

(1c) a heavy chain variable region comprising the following three CDRs: CDR-H1 having an amino acid sequence of SEQ ID NO: 13 or a variant thereof, CDR-H2 having an amino acid sequence of SEQ ID NO: 14 or a variant thereof, and CDR-H3 having an amino acid sequence of SEQ ID NO: 15 or a variant thereof; and, a light chain variable region comprising the following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;

(1d) a heavy chain variable region comprising the following three CDRs: CDR-H1 having an amino acid sequence of SEQ ID NO: 19 or a variant thereof, CDR-H2 having an amino acid sequence of SEQ ID NO: 20 or a variant thereof, and CDR-H3 having an amino acid sequence of SEQ ID NO: 21 or a variant thereof; and, a light chain variable region comprising the following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;

(1e) a heavy chain variable region comprising the following three CDRs: CDR-H1 having an amino acid sequence of SEQ ID NO: 25 or a variant thereof, CDR-H2 having an amino acid sequence of SEQ ID NO: 26 or a variant thereof, and CDR-H3 having an amino acid sequence of SEQ ID NO: 27 or a variant thereof; and, a light chain variable region comprising the following three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof;

(1f) a heavy chain variable region comprising the following three CDRs: CDR-H1 having an amino acid sequence of SEQ ID NO: 31 or a variant thereof, CDR-H2 having an amino acid sequence of SEQ ID NO: 32 or a variant thereof, and CDR-H3 having an amino acid sequence of SEQ ID NO: 33 or a variant thereof; and, a light chain variable region comprising the following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof;

(1g) a heavy chain variable region comprising the following three CDRs: CDR-H1 having an amino acid sequence of SEQ ID NO: 37 or a variant thereof, CDR-H2 having an amino acid sequence of SEQ ID NO: 38 or a variant thereof, and CDR-H3 having an amino acid sequence of SEQ ID NO: 39 or a variant thereof; and, a light chain variable region comprising the following three CDRs: CDR-L1 having an amino acid sequence of SEQ ID NO: 40 or a variant thereof, CDR-L2 having an amino acid sequence of SEQ ID NO: 41 or a variant thereof, and CDR-L3 having an amino acid sequence of SEQ ID NO: 42 or a variant thereof;

(1h) a heavy chain variable region comprising the following three CDRs: CDR-H1 having an amino acid sequence of SEQ ID NO: 43 or a variant thereof, CDR-H2 having an amino acid sequence of SEQ ID NO: 44 or a variant thereof, and CDR-H3 having an amino acid sequence of SEQ ID NO: 45 or a variant thereof; and, a light chain variable region comprising the following three CDRs: CDR-L1 having an amino acid sequence of SEQ ID NO: 46 or a variant thereof, CDR-L2 having an amino acid sequence of SEQ ID NO: 47 or a variant thereof, and CDR-L3 having an amino acid sequence of SEQ ID NO: 48 or a variant thereof;

(1i) a heavy chain variable region comprising the following three CDRs: CDR-H1 having an amino acid sequence of SEQ ID NO: 49 or a variant thereof, CDR-H2 having an amino acid sequence of SEQ ID NO: 50 or a variant thereof, and CDR-H3 having an amino acid sequence of SEQ ID NO: 51 or a variant thereof; and, a light chain variable region comprising the following three CDRs: CDR-L1 having an amino acid sequence of SEQ ID NO: 52 or a variant thereof, CDR-L2 having an amino acid sequence of SEQ ID NO: 53 or a variant thereof, and CDR-L3 having an amino acid sequence of SEQ ID NO: 54 or a variant thereof;

(1j) a heavy chain variable region comprising the following three CDRs: CDR-H1 having an amino acid sequence of SEQ ID NO: 55 or a variant thereof, CDR-H2 having an amino acid sequence of SEQ ID NO: 56 or a variant thereof, and CDR-H3 having an amino acid sequence of SEQ ID NO: 57 or a variant thereof; and, a light chain variable region comprising the following three CDRs: CDR-L1 having an amino acid sequence of SEQ ID NO: 58 or a variant thereof, CDR-L2 having an amino acid sequence of SEQ ID NO: 59 or a variant thereof, and CDR-L3 having an amino acid sequence of SEQ ID NO: 60 or a variant thereof;

(1k) a heavy chain variable region comprising the following three CDRs: CDR-H1 having an amino acid sequence of SEQ ID NO: 61 or a variant thereof, CDR-H2 having an amino acid sequence of SEQ ID NO: 62 or a variant thereof, and CDR-H3 having an amino acid sequence of SEQ ID NO: 63 or a variant thereof; and, a light chain variable region comprising the following three CDRs: CDR-L1 having an amino acid sequence of SEQ ID NO: 64 or a variant thereof, CDR-L2 having an amino acid sequence of SEQ ID NO: 65 or a variant thereof, and CDR-L3 having an amino acid sequence of SEQ ID NO: 66 or a variant thereof;

(11) a heavy chain variable region comprising the following three CDRs: CDR-H1 having an amino acid sequence of SEQ ID NO: 67 or a variant thereof, CDR-H2 having an amino acid sequence of SEQ ID NO: 68 or a variant thereof, and CDR-H3 having an amino acid sequence of SEQ ID NO: 69 or a variant thereof; and, a light chain variable region comprising the following three CDRs: CDR-L1 having an amino acid sequence of SEQ ID NO: 70 or a variant thereof, CDR-L2 having an amino acid sequence of SEQ ID NO: 71 or a variant thereof, and CDR-L3 having an amino acid sequence of SEQ ID NO: 72 or a variant thereof;

(1m) a heavy chain variable region comprising the following three CDRs: CDR-H1 having an amino acid sequence of SEQ ID NO: 73 or a variant thereof, CDR-H2 having an amino acid sequence of SEQ ID NO: 74 or a variant thereof, and CDR-H3 having an amino acid sequence of SEQ ID NO: 75 or a variant thereof; and, a light chain variable region comprising the following three CDRs: CDR-L1 having an amino acid sequence of SEQ ID NO: 76 or a variant thereof, CDR-L2 having an amino acid sequence of SEQ ID NO: 77 or a variant thereof, and CDR-L3 having an amino acid sequence of SEQ ID NO: 78 or a variant thereof;

(1n) a heavy chain variable region comprising the following three CDRs: CDR-H1 having an amino acid sequence of SEQ ID NO: 79 or a variant thereof, CDR-H2 having an amino acid sequence of SEQ ID NO: 80 or a variant thereof, and CDR-H3 having an amino acid sequence of SEQ ID NO: 81 or a variant thereof; and, a light chain variable region comprising the following three CDRs: CDR-L1 having an amino acid sequence of SEQ ID NO: 82 or a variant thereof, CDR-L2 having an amino acid sequence of SEQ ID NO: 83 or a variant thereof, and CDR-L3 with an amino acid sequence of SEQ ID NO: 84 or a variant thereof;

(1o) a heavy chain variable region comprising the following three CDRs: CDR-H1 having an amino acid sequence of SEQ ID NO: 85 or a variant thereof, CDR-H2 having an amino acid sequence of SEQ ID NO: 86 or a variant thereof, and CDR-H3 having an amino acid sequence of SEQ ID NO: 87 or a variant thereof; and, a light chain variable region comprising the following three CDRs: CDR-L1 having an amino acid sequence of SEQ ID NO: 88 or a variant thereof, CDR-L2 having an amino acid sequence of SEQ ID NO: 89 or a variant thereof, and CDR-L3 having an amino acid sequence of SEQ ID NO: 90 or a variant thereof;

(1p) a heavy chain variable region comprising the following three CDRs: CDR-H1 having an amino acid sequence of SEQ ID NO: 91 or a variant thereof, CDR-H2 having an amino acid sequence of SEQ ID NO: 92 or a variant thereof, and CDR-H3 having an amino acid sequence of SEQ ID NO: 93 or a variant thereof; and, a light chain variable region comprising the following three CDRs: CDR-L1 having an amino acid sequence of SEQ ID NO: 94 or a variant thereof, CDR-L2 having an amino acid sequence of SEQ ID NO: 95 or a variant thereof, and CDR-L3 having an amino acid sequence of SEQ ID NO: 96 or a variant thereof; or

(1q) a heavy chain variable region comprising the following three CDRs: CDR-H1 having an amino acid sequence of SEQ ID NO: 97 or a variant thereof, CDR-H2 having an amino acid sequence of SEQ ID NO: 98 or a variant thereof, and CDR-H3 having an amino acid sequence of SEQ ID NO: 99 or a variant thereof; and, a light chain variable region comprising the following three CDRs: CDR-L1 having an amino acid sequence of SEQ ID NO: 100 or a variant thereof, CDR-L2 having an amino acid sequence of SEQ ID NO: 101 or a variant thereof, and CDR-L3 having an amino acid sequence of SEQ ID NO: 102 or a variant thereof.

In some embodiments, the heavy chain variable region further comprises a heavy chain framework region (FR). In some embodiments, the light chain variable region further comprises a light chain framework region (FR). The framework region is highly conserved, and it provides a scaffold for the six CDRs in three-dimensional space to form an antigen binding surface. The variable domains of naturally occurring heavy and light chains each comprises four FR regions (FR1, FR2, FR3, and FR4), and these four FR regions are mainly based on a $\beta$-folded conformation, and three complementary determining regions are connected in the form of FR1-CDR1-FR2-CDR2-FR3-CDR3-FR4, wherein the CDR regions are mainly based on loop conformation, and in some cases form a part of $\beta$-folded structure. The complementary determining regions in each chain are closely together through FR, and together with the complementary determining regions from another chain, thereby contributing to the formation of the antigen binding side end (see, Kabat et al., loc. cit.). The heavy chain framework region and/or the light chain framework region can be independently derived from the framework region of any species immunoglobulin. In some embodiments, the heavy chain framework region and the light chain framework region are each independently derived from the heavy chain framework region and the light chain framework region of a rabbit or sheep immunoglobulin. In some embodiments, the heavy chain framework region and the light chain framework region are each independently derived from the heavy chain framework region and the light chain framework region of a human or mouse immunoglobulin. In some embodiments, the heavy chain framework region and the light chain framework region each independently comprise a sequence derived from the heavy chain framework region and the light chain framework region of a mouse immunoglobulin, the heavy chain framework region and the light chain framework region of a human immunoglobulin, or a combination thereof. In some embodiments, the heavy chain framework region and the light chain framework region each independently comprise a sequence derived from the heavy chain framework region and the light chain framework region of a rabbit or sheep immunoglobulin, the heavy chain framework region and the light chain framework region of a human immunoglobulin, or a combination thereof. In some embodiments, the heavy chain framework region and the light chain framework region each independently comprise a sequence derived from the heavy chain framework region and the light chain framework region of a human germline antibody.

In some embodiments, the antibody or antigen-binding fragment thereof comprises a heavy chain constant region (CH) and a light chain constant region (CL). In some embodiments, the antibody or antigen-binding fragment thereof comprises a rabbit or sheep heavy chain constant region and a rabbit or sheep light chain constant region. In some embodiments, the antibody or antigen-binding fragment thereof comprises a human heavy chain constant region and a human light chain constant region. In some embodiments, the antibody or antigen-binding fragment thereof is an IgG, IgM, IgE, IgD or IgA antibody. In some embodiments, the heavy chain constant region is an IgG heavy chain constant region, such as an IgG1, IgG2, IgG3 or IgG4 heavy chain constant region. In some embodiments, the light chain constant region is a $\kappa$ or $\lambda$ light chain constant region (e.g., a human $\lambda$ light chain constant region).

In some embodiments, the antigen-binding fragment is selected from the group consisting of scFv, Fab, Fab', (Fab')$_2$, Fd, Fv, CDR fragment, nanobody, disulfide-linked Fv (dsFv), diabody, bispecific antibody and multispecific antibody; and/or, the antibody is a rabbit or sheep antibody, a chimeric antibody or a humanized antibody.

In some embodiments, the antibody or antigen-binding fragment thereof further has a detectable label.

In the present application, the detectable label can be any substance that can be detected by fluorescent, spectrospic, photochemical, biochemical, immunological, electrical, optical or chemical means. It is particularly preferred that such labels are suitable for immunological detection (e.g., enzyme-linked immunosorbent assay, radioimmunoassay, fluorescent immunoassay, chemiluminescent immunoassay, etc.). Such labels are well known in the art and include, but are not limited to, enzymes (e.g., oxidase, microperoxidase, horseradish peroxidase, alkaline phosphatase, β-galactosidase, urease, glucose oxidase, etc.), radionuclides (e.g., $^{3}H$, $^{125}I$, $^{35}S$, $^{14}C$ or $^{32}P$), fluorescent dyes (e.g., fluorescein isothiocyanate (FITC), fluorescein, tetramethylrhodamine isothiocyanate (TRITC), phycoerythrin (PE), Texas Red, rhodamine, quantum dots or cyanine dye derivatives (e.g., Cy7, Alexa 750), europium and green fluorescent protein, etc.), chemiluminescence (e.g., luminol, isoluminol, phenanthridinium, acridinium esters, etc.), and biotin for binding to avidin modified with the above-mentioned labels (e.g., streptavidin). The labels covered in the present application can be detected by methods known in the art. For example, radioactive labels can be detected using photographic film or a scintillation counter, and fluorescent labels can be detected using a photodetector to detect the emitted light. Enzyme labels are generally detected by providing a substrate to the enzyme and detecting the reaction product produced by the action of the enzyme on the substrate. Chemiluminescent substances (e.g., acridinium esters) are generally detected by providing an excitation liquid and/or a catalyst to the luminescent substance to detect the emitted light. Biotin is generally detected by providing biotin with avidin (e.g., streptavidin) modified with the above-mentioned label and detecting the label carried by the avidin linked to biotin. In certain embodiments, the detectable labels as described above can be linked to the antibody or antigen-binding fragment thereof of the present application through linkers of different lengths to reduce potential steric hindrance.

In some embodiments, the label is selected from the group consisting of fluorescein, chemiluminescence (e.g., acridinium esters), enzyme (e.g., horseradish peroxidase, alkaline phosphatase), radioactive isotope, biotin, colloidal gold, and magnetic particle.

In some embodiments, the antibody or antigen-binding fragment thereof specifically recognizes TSH dimer or TSH β-subunit. In some embodiments, the antibody or antigen-binding fragment thereof can also recognize TSH R55G mutant.

In another aspect, the present application provides an isolated nucleic acid molecule, which encodes the antibody or antigen-binding fragment thereof as described in any of the foregoing.

In another aspect, the present application provides an expression vector, which comprises the isolated nucleic acid molecule as described in any of the foregoing.

In some embodiments, the vector is a plasmid, a virus, a phage, a bacterium or a viroid.

In another aspect, the present application provides a host cell, which comprises the isolated nucleic acid molecule or the expression vector as described in any of the foregoing.

In some embodiments, the host cell is a eukaryotic cell, preferably a mammalian cell.

In some embodiments, the host cell is a prokaryotic cell, preferably *Escherichia coli.*

In another aspect, the present application provides an antibody or antigen-binding fragment thereof that specifically binds to TSH or a mutant thereof, wherein the antibody or antigen-binding fragment thereof is:

produced based on a plasmid in *Escherichia coli* Rosetta™ (DE3) pLysS hTS01 VH deposited at the All-Russian National Collection of Industrial Microorganisms (VKPM) with Accession Number B-14885 and a plasmid in Escherichia coli RosettaTM (DE3) pLysS hTS01 VL deposited at VKPM with Accession Number B-14884;

produced based on a plasmid in *Escherichia coli* Rosetta™ (DE3) pLysS hTS02 VH deposited at the VKPM with Accession Number B-14887 and a plasmid in Escherichia coli RosettaTM (DE3) pLysS hTS02 VL deposited at VKPM with Accession Number B-14886;

produced based on a plasmid in *Escherichia coli* Rosetta™ (DE3) pLysS hTS03 VH deposited at the VKPM with Accession Number B-14889 and a plasmid in Escherichia coli RosettaTM (DE3) pLysS hTS03 VL deposited at VKPM with Accession Number B-14888;

produced based on a plasmid in *Escherichia coli* Rosetta™ (DE3) pLysS hTS04 VH deposited at the VKPM with Accession Number B-14891 and a plasmid in Escherichia coli RosettaTM (DE3) pLysS hTS04 VL deposited at VKPM with Accession Number B-14890;

produced based on a plasmid in *Escherichia coli* Rosetta™ (DE3) pLysS hTS05 VH deposited at the VKPM with Accession Number B-14893 and a plasmid in Escherichia coli RosettaTM (DE3) pLysS hTS05 VL deposited at VKPM with Accession Number B-14892;

produced based on a plasmid in *Escherichia coli* Rosetta™ (DE3) pLysS hTS06 VH deposited at the VKPM with Accession Number B-14895 and a plasmid in Escherichia coli RosettaTM (DE3) pLysS hTS06 VL deposited at VKPM with Accession Number B-14896;

produced based on a plasmid in *Escherichia coli* Rosetta™ (DE3) pLysS hTS07 VH deposited at the VKPM with Accession Number B-14897 and a plasmid in Escherichia coli RosettaTM (DE3) pLysS hTS07 VL deposited at VKPM with Accession Number B-14896;

produced based on a plasmid in *Escherichia coli* Rosetta™ (DE3) pLysS hTS08 VH deposited at the VKPM with Accession Number B-14899 and a plasmid in Escherichia coli RosettaTM (DE3) pLysS hTS08 VL deposited at VKPM with Accession Number B-14898;

produced based on a plasmid in *Escherichia coli* Rosetta™ (DE3) pLysS hTS09 VH deposited at the VKPM with Accession Number B-14901 and a plasmid in Escherichia coli RosettaTM (DE3) pLysS hTS09 VL deposited at VKPM with Accession Number B-14900;

produced based on a plasmid in *Escherichia coli* Rosetta™ (DE3) pLysS hTS10 VH deposited at the VKPM with Accession Number B-14903 and a plasmid in Escherichia coli RosettaTM (DE3) pLysS hTS10 VL deposited at VKPM with Accession Number B-14902;

produced based on a plasmid in *Escherichia coli* Rosetta™ (DE3) pLysS hTS11 VH deposited at the VKPM with Accession Number B-14905 and a plasmid in Escherichia coli RosettaTM (DE3) pLysS hTS11 VL deposited at VKPM with Accession Number B-14904;

produced based on a plasmid in *Escherichia coli* Rosetta™ (DE3) pLysS hTS12 VH deposited at the VKPM with Accession Number B-14907 and a plasmid in Escherichia coli RosettaTM (DE3) pLysS hTS13 VL deposited at VKPM with Accession Number B-14906;

produced based on a plasmid in *Escherichia coli* Rosetta™ (DE3) pLysS hTS13 VH deposited at the VKPM with Accession Number B-14909 and a plasmid in Escherichia coli RosettaTM (DE3) pLysS hTS13 VL deposited at VKPM with Accession Number B-14908;

produced based on a plasmid in *Escherichia coli* Rosetta™ (DE3) pLysS hTS14 VH deposited at the VKPM with Accession Number B-14911 and a plasmid in Escherichia coli RosettaTM (DE3) pLysS hTS14 VL deposited at VKPM with Accession Number B-14910;

produced based on a plasmid in *Escherichia coli* Rosetta™ (DE3) pLysS hTS15 VH deposited at the VKPM with Accession Number B-14913 and a plasmid in Escherichia coli RosettaTM (DE3) pLysS hTS15 VL deposited at VKPM with Accession Number B-14912;

produced based on a plasmid in *Escherichia coli* Rosetta™ (DE3) pLysS hTS16 VH deposited at the VKPM with Accession Number B-14915 and a plasmid in Escherichia coli RosettaTM (DE3) pLysS hTS16 VL deposited at VKPM with Accession Number B-14914; or

produced based on a plasmid in *Escherichia coli* Rosetta™ (DE3) pLysS hTS17 VH deposited at the VKPM with Accession Number B-14917 and a plasmid in Escherichia coli RosettaTM (DE3) pLysS hTS17 VL deposited at VKPM with Accession Number B-14916.

Kit

Currently, mainstream TSH detection is still based on the third generation of detection reagents, and the functional sensitivity for TSH is 0.01 to 0.02 μIU/mL. There are still obvious deficiencies in some special clinical scenarios:

1. For patients with severe hyperthyroidism, accurate TSH measurement is particularly important in the doctor's initial judgment and treatment monitoring of patients, and the TSH content in the circulation of these patients is often lower than 0.01 μIU/mL, so the sensitivity of existing detection reagents may not meet this specific clinical need;

2. For patients with thyroid cancer, after complete removal of thyroid gland, they need to take levothyroxine tablets to inhibit the synthesis and secretion of TSH in the body. In order to ensure better inhibition of the recurrence of thyroid cancer, the optimization of this inhibition therapy is to suppress TSH to below 0.01 μIU/mL, so the sensitivity of existing detection reagents cannot meet this specific clinical need.

The antibody of the present application has excellent specificity and affinity for TSH. On the one hand, it does not cross-react with the α-subunit shared by LH, FSH, CG and free glycoprotein hormones, and on the other hand, it can recognize the TSH variant R55G. The kit developed based on the antibody of the present application can achieve a fourth-generation functional sensitivity of <0.002 μIU/mL.

Therefore, in one aspect, the present application provides a TSH detection kit for detecting the presence or level of TSH or a mutant thereof in a sample.

In some embodiments, the detection sensitivity of the kit for TSH is ≤0.0015 μIU/mL, preferably ≤0.001 μIU/mL.

In some embodiments, the TSH or a mutant thereof is selected from the group consisting of TSH dimer, β subunit of TSH and/or R55G mutant of TSH.

In some embodiments, the kit comprises a first reagent and a second reagent, wherein the first reagent comprises magnetic beads coated with a first antibody capable of recognizing the TSH or a mutant thereof; and the second reagent comprises a second antibody connected with a chemiluminescent label, wherein a sandwich complex of the first antibody-an antigen-the second antibody can be formed after the sample is mixed with the first reagent and the second reagent, and the antigen is the TSH or mutant thereof.

The label is well known to those skilled in the art, and may be, for example, an enzyme such as oxidase, microperoxidase, horseradish peroxidase, alkaline phosphatase (ALP), β-galactosidase, glucose oxidase, and glucose 6-phosphate dehydrogenase; a fluorescent substance such as fluorescein isothiocyanate, tetramethylrhodamine isothiocyanate, fluorescein, rhodamine, europium, and green fluorescent protein; a chemiluminescence such as luminol, isoluminol, phenanthridinium, and acridinium ester.

In some embodiments, the first antibody and/or the second antibody is selected from an antibody or antigen-binding fragment thereof that is that specifically binds to TSH or a mutant thereof, comprising:

a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or,

a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof.

In some embodiments, the first antibody and/or the second antibody comprises:

a heavy chain variable region comprising the following three CDRs: CDR-H1 having an amino acid sequence of SEQ ID NO: 1 or a variant thereof, CDR-H2 having an amino acid sequence of SEQ ID NO: 2 or a variant thereof, and CDR-H3 having an amino acid sequence of SEQ ID NO: 3 or a variant thereof; and, a light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;

a heavy chain variable region comprising the following three CDRs: CDR-H1 having an amino acid sequence of SEQ ID NO: 7 or a variant thereof, CDR-H2 having an amino acid sequence of SEQ ID NO: 8 or a variant thereof, and CDR-H3 having an amino acid sequence of SEQ ID NO: 9 or a variant thereof; and, a light chain variable region comprising the following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;

a heavy chain variable region comprising the following three CDRs: CDR-H1 having an amino acid sequence of SEQ ID NO: 13 or a variant thereof, CDR-H2 having an amino acid sequence of SEQ ID NO: 14 or a variant thereof, and CDR-H3 having an amino acid sequence of SEQ ID NO: 15 or a variant thereof; and, a light chain variable region comprising the following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;

a heavy chain variable region comprising the following three CDRs: CDR-H1 having an amino acid sequence of SEQ ID NO: 19 or a variant thereof, CDR-H2 having an amino acid sequence of SEQ ID NO: 20 or a variant thereof, and CDR-H3 having an amino acid sequence of SEQ ID NO: 21 or a variant thereof; and, a light chain variable region comprising the following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;

a heavy chain variable region comprising the following three CDRs: CDR-H1 having an amino acid sequence of SEQ ID NO: 25 or a variant thereof, CDR-H2 having an amino acid sequence of SEQ ID NO: 26 or a variant thereof, and CDR-H3 having an amino acid sequence of SEQ ID NO: 27 or a variant thereof; and, a light chain variable region comprising the following three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof;

a heavy chain variable region comprising the following three CDRs: CDR-H1 having an amino acid sequence of SEQ ID NO: 31 or a variant thereof, CDR-H2 having an amino acid sequence of SEQ ID NO: 32 or a variant thereof, and CDR-H3 having an amino acid sequence of SEQ ID NO: 33 or a variant thereof; and, a light chain variable region comprising the following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof;

a heavy chain variable region comprising the following three CDRs: CDR-H1 having an amino acid sequence of SEQ ID NO: 37 or a variant thereof, CDR-H2 having an amino acid sequence of SEQ ID NO: 38 or a variant thereof, CDR-H3 having an amino acid sequence of SEQ ID NO: 39 or a variant thereof; and, a light chain variable region comprising the following three CDRs: CDR-L1 having an amino acid sequence of SEQ ID NO: 40 or a variant thereof, CDR-L2 having an amino acid sequence of SEQ ID NO: 41 or a variant thereof, and CDR-L3 having an amino acid sequence of SEQ ID NO: 42 or a variant thereof;

a heavy chain variable region comprising the following three CDRs: CDR-H1 having an amino acid sequence of SEQ ID NO: 43 or a variant thereof, CDR-H2 having an amino acid sequence of SEQ ID NO: 44 or a variant thereof, and CDR-H3 having an amino acid sequence of SEQ ID NO: 45 or a variant thereof; and, a light chain variable region comprising the following three CDRs: CDR-L1 having an amino acid sequence of SEQ ID NO: 46 or a variant thereof, CDR-L2 having an amino acid sequence of SEQ ID NO: 47 or a variant thereof, and CDR-L3 having an amino acid sequence of SEQ ID NO: 48 or a variant thereof;

a heavy chain variable region comprising the following three CDRs: CDR-H1 having an amino acid sequence of SEQ ID NO: 49 or a variant thereof, CDR-H2 having an amino acid sequence of SEQ ID NO: 50 or a variant thereof, and CDR-H3 having an amino acid sequence of SEQ ID NO: 51 or a variant thereof; and, a light chain variable region comprising the following three CDRs: CDR-L1 having an amino acid sequence of SEQ ID NO: 52 or a variant thereof, CDR-L2 having an amino acid sequence of SEQ ID NO: 53 or a variant thereof, and CDR-L3 having an amino acid sequence of SEQ ID NO: 54 or a variant thereof;

a heavy chain variable region comprising the following three CDRs: CDR-H1 having an amino acid sequence of SEQ ID NO: 55 or a variant thereof, CDR-H2 having an amino acid sequence of SEQ ID NO: 56 or a variant thereof, and CDR-H3 having an amino acid sequence of SEQ ID NO: 57 or a variant thereof; and, a light chain variable region comprising the following three CDRs: CDR-L1 having an amino acid sequence of SEQ ID NO: 58 or a variant thereof, CDR-L1 having an amino acid sequence of SEQ ID NO: 59 or a variant thereof, and CDR-L3 having an amino acid sequence of SEQ ID NO: 60 or a variant thereof;

a heavy chain variable region comprising the following three CDRs: CDR-H1 having an amino acid sequence of SEQ ID NO: 61 or a variant thereof, CDR-H2 having an amino acid sequence of SEQ ID NO: 62 or a variant thereof, and CDR-H3 having an amino acid sequence of SEQ ID NO: 63 or a variant thereof; and, a light chain variable region comprising the following three CDRs: CDR-L1 having an amino acid sequence of SEQ ID NO: 64 or a variant thereof, CDR-L2 having an amino acid sequence of SEQ ID NO: 65 or a variant thereof, and CDR-L3 having an amino acid sequence of SEQ ID NO: 66 or a variant thereof;

a heavy chain variable region comprising the following three CDRs: CDR-H1 having an amino acid sequence of SEQ ID NO: 67 or a variant thereof, CDR-H2 having an amino acid sequence of SEQ ID NO: 68 or a variant thereof, CDR-H3 having an amino acid sequence of SEQ ID NO: 69 or a variant thereof; and, a light chain variable region comprising the following three CDRs: CDR-L1 having an amino acid sequence of SEQ ID NO: 70 or a variant thereof, CDR-L2 having an amino acid sequence of SEQ ID NO: 71 or a variant thereof, and CDR-L3 having an amino acid sequence of SEQ ID NO: 72 or a variant thereof;

a heavy chain variable region comprising the following three CDRs: CDR-H1 having an amino acid sequence of SEQ ID NO: 73 or a variant thereof, CDR-H2 having an amino acid sequence of SEQ ID NO: 74 or a variant thereof, and CDR-H3 having an amino acid sequence of SEQ ID NO: 75 or a variant thereof; and, a light chain variable region comprising the following three CDRs: CDR-L1 having an amino acid sequence of SEQ ID NO: 76 or a variant thereof, CDR-L2 having an amino acid sequence of SEQ ID NO: 77 or a variant thereof, and CDR-L3 having an amino acid sequence of SEQ ID NO: 78 or a variant thereof;

a heavy chain variable region comprising the following three CDRs: CDR-H1 having an amino acid sequence of SEQ ID NO: 79 or a variant thereof, CDR-H2 having an amino acid sequence of SEQ ID NO: 80 or a variant thereof, and CDR-H3 having an amino acid sequence of SEQ ID NO: 81 or a variant thereof; and, a light chain variable region comprising the following three CDRs: CDR-L1 having an amino acid sequence of SEQ ID NO: 82 or a variant thereof, CDR-L2 having an amino acid sequence of SEQ ID NO: 83 or a variant thereof, and CDR-L3 having an amino acid sequence of SEQ ID NO: 84 or a variant thereof;

a heavy chain variable region comprising the following three CDRs: CDR-H1 having an amino acid sequence of SEQ ID NO: 85 or a variant thereof, CDR-H2 having an amino acid sequence of SEQ ID NO: 86 or a variant thereof, and CDR-H3 having an amino acid sequence of SEQ ID NO: 87 or a variant thereof; and, a light chain variable region comprising the following three CDRs: CDR-L1 having an amino acid sequence of SEQ ID NO: 88 or a variant thereof, CDR-L2 having an amino acid sequence of SEQ ID NO: 89 or a variant thereof, and CDR-L3 having an amino acid sequence of SEQ ID NO: 90 or a variant thereof;

a heavy chain variable region comprising the following three CDRs: CDR-H1 having an amino acid sequence of SEQ ID NO: 91 or a variant thereof, CDR-H2 having an amino acid sequence of SEQ ID NO: 92 or a variant thereof, and CDR-H3 having an amino acid sequence of SEQ ID NO: 93 or a variant thereof; and, a light chain variable region comprising the following three CDRs: CDR-L1 having an amino acid sequence of SEQ ID NO: 94 or a variant thereof, CDR-L2 having an amino acid sequence of SEQ ID NO: 95 or a variant thereof, and CDR-L3 having an amino acid sequence of SEQ ID NO: 96 or a variant thereof; or

a heavy chain variable region comprising the following three CDRs: CDR-H1 having an amino acid sequence of SEQ ID NO: 97 or a variant thereof, CDR-H2 having an amino acid sequence of SEQ ID NO: 98 or a variant thereof, CDR-H3 having an amino acid sequence of SEQ ID NO: 99 or a variant thereof; and a light chain variable region comprising the following three CDRs: CDR-L1 having an amino acid sequence of SEQ ID NO: 100 or a variant thereof, CDR-L2 having an amino acid sequence of SEQ ID NO: 101 or a variant thereof, and CDR-L3 having an amino acid sequence of SEQ ID NO: 102 or a variant thereof.

In some embodiments, the first antibody is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof; and,
the second antibody is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof.

In some embodiments, the first antibody is an antibody or antigen-binding fragment thereof having a heavy chain variable region comprising the following three CDRs: CDR-H1 having an amino acid sequence selected from SEQ ID NO: 37 and variants thereof, CDR-H2 having an amino acid sequence selected from SEQ ID NO: 38 and variants thereof, and CDR-H3 having an amino acid sequence selected from SEQ ID NO: 39 and variants thereof; and, a light chain variable region comprising the following three CDRs: CDR-L1 having an amino acid sequence selected from SEQ ID NO: 40 and variants thereof, CDR-L2 having an amino acid sequence selected from SEQ ID NO: 41 and variants thereof, and CDR-L3 having an amino acid sequence selected from SEQ ID NO: 42 and variants thereof; and,

the second antibody is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-H3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof.

In some embodiments, the first antibody is an antibody or antigen-binding fragment thereof having a heavy chain variable region comprising the following three CDRs: CDR-H1 having an amino acid sequence selected from SEQ ID NO: 25 and variants thereof, CDR-H2 having an amino acid sequence selected from SEQ ID NO: 26 and variants thereof, and CDR-L3 having an amino acid sequence selected from SEQ ID NO: 27 and variants thereof; and, a light chain variable region comprising the following three CDRs: CDR-L1 having an amino acid sequence selected from SEQ ID NO: 28 and variants thereof, CDR-L2 having an amino acid sequence selected from SEQ ID NO: 29 and variants thereof, and CDR-L3 having an amino acid sequence selected from SEQ ID NO: 30 and variants thereof; and,

the second antibody is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof.

In some embodiments, the first antibody is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 having an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: 23 and variants thereof, and CDR-L3 having an amino acid sequence selected from SEQ ID NO: 24 and variants thereof; and,

the second antibody is an antibody or antigen-binding fragment thereof having a heavy chain variable region comprising the following three CDRs: CDR-H1 having an amino acid sequence selected from SEQ ID NO: 61 and variants thereof, CDR-H2 having an amino acid sequence selected from SEQ ID NO: 62 and variants thereof, and CDR-H3 having an amino acid sequence selected from SEQ ID NO: 63 and variants thereof; and, a light chain variable region comprising the following three CDRs: CDR-L1 having an amino acid sequence selected from SEQ ID NO: 64 and variants thereof, CDR-L2 having an amino acid sequence selected from SEQ ID NO: 65 and variants thereof, and CDR-L3 having an amino acid sequence selected from SEQ ID NO: 66 and variants thereof.

In some embodiments, the first antibody is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 with an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: ID NO: 23 and variants thereof, and CDR-L3 having an amino acid sequence selected from SEQ ID NO: 24 and variants thereof; and,

the second antibody is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-H3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof.

In another aspect, the present application provides a composition or TSH detection kit, which comprises the antibody or antigen-binding fragment thereof as described in any of the foregoing.

In some embodiments, the composition or TSH detection kit comprises:

a first antibody, which is at least one selected from the antibody or antigen-binding fragment as described in any of the foregoing, and,

a second antibody, which is at least one selected from the antibody or antigen-binding fragment as described in any of the foregoing;

wherein, the first antibody and the second antibody are directed to different epitopes of TSH or a mutant thereof, respectively.

In some embodiments, in the composition or TSH detection kit,

I) the first antibody is the antibody or antigen-binding fragment thereof as described in (1a) of the first aspect, or a combination thereof with any one or more of (1b) to (1q) as defined in the first aspect;
the second antibody is one or more selected from (1b) to (1q), preferably (1c) or (1f), as defined in the first aspect;
II) the first antibody is the antibody or antigen-binding fragment thereof as described in (1b) of the first aspect, or a combination thereof with any one or more of (1a), (1c) to (1q) as defined in the first aspect;
the second antibody is one or more selected from (1a), (1c) to (1q), preferably (1i) or (1j), as defined in the first aspect;
III) the first antibody is the antibody or antigen-binding fragment thereof as described in (1c) of the first aspect, or a combination thereof with any one or more of (1a), (1b), (1d) to (1q) as defined in the first aspect;
the second antibody is one or more selected from (1a), (1b), (1d) to (1q), preferably (1j), as defined in the first aspect;
IV) the first antibody is the antibody or antigen-binding fragment thereof as described in (1d) of the first aspect, or a combination thereof with any one or more of (1a) to (1c), (1e) to (1q) as defined in the first aspect;
The second antibody is one or more selected from (1a) to (1c), (1e) to (1q), preferably (1h), (1k) or (1o), as defined in the first aspect;
V) the first antibody is the antibody or antigen-binding fragment thereof as described in (1e) of the first aspect, or a combination thereof with any one or more of (1a) to (1d), (1f) to (1q) as defined in the first aspect;
the second antibody is one or more selected from (1a) to (1d), (1f) to (1q), preferably (1m) or (1o), as defined in the first aspect;
VI) the first antibody is the antibody or antigen-binding fragment thereof as described in (1f) of the first aspect, or a combination thereof with any one or more of (1a) to (1e), (1g) to (1q) as defined in the first aspect;
the second antibody is one or more selected from (1a) to (1e), (1g) to (1q), preferably (1i), as defined in the first aspect;
VII) the first antibody is the antibody or antigen-binding fragment thereof as described in (1g) of the first aspect, or a combination thereof with any one or more of (1a) to (1f), (1h) to (1q) as defined in the first aspect;
the second antibody is one or more selected from (1a) to (1f), (1h) to (1q), preferably (1h) or (1j), as defined in the first aspect;
VIII) the first antibody is the antibody or antigen-binding fragment thereof as described in (1h) of the first aspect, or a combination thereof with any one or more of (1a) to (1g), (1i) to (1q) as defined in the first aspect;
the second antibody is one or more selected from (1a) to (1g), (1i) to (1q), preferably (1f), as defined in the first aspect;
IX) the first antibody is the antibody or antigen-binding fragment thereof as described in (1i) of the first aspect, or a combination thereof with any one or more of (1a) to (1h), (1j) to (1q) as defined in the first aspect;
the second antibody is one or more selected from (1a) to (1h), (1j) to (1q), preferably (1f), as defined in the first aspect;
X) the first antibody is the antibody or antigen-binding fragment thereof as described in (1j) of the first aspect, or a combination thereof with any one or more of (1a) to (1i), (1k) to (1q) as defined in the first aspect;
the second antibody is one or more selected from (1a) to (1i), (1k) to (1q) as defined in the first aspect;
XI) the first antibody is the antibody or antigen-binding fragment thereof as described in (1k) of the first aspect, or a combination thereof with any one or more of (1a) to (1j), (11) to (1q) as defined in the first aspect;
the second antibody is one or more selected from (1a) to (1j), (11) to (1q) as defined in the first aspect;
XII) the first antibody is the antibody or antigen-binding fragment thereof as described in (11) of the first aspect, or a combination thereof with any one or more of (1a) to (1k), (1m) to (1q) as defined in the first aspect;
the second antibody is one or more selected from (1a) to (1k), (1m) to (1q), preferably (1f), as defined in the first aspect;
XIII) the first antibody is the antibody or antigen-binding fragment thereof as described in (1m) of the first aspect, or a combination thereof with any one or more of (1a) to (11), (1n) to (1q) as defined in the first aspect;
the second antibody is one or more selected from (1a) to (11), (1n) to (1q) as defined in the first aspect;
XIV) the first antibody is the antibody or antigen-binding fragment thereof as described in (1n) of the first aspect, or a combination thereof with any one or more of (1a) to (1m), (1o) to (1q) as defined in the first aspect;
the second antibody is one or more selected from (1a) to (1m), (1o) to (1q), preferably (1f), as defined in the first aspect; or
XV) the first antibody is the antibody or antigen-binding fragment thereof as described in (1o) of the first aspect, or a combination thereof with any one or more of (1a) to (1n), (1p) to (1q) as defined in the first aspect;
the second antibody is one or more selected from (1a) to (1n), (1p) to (1q) as defined in the first aspect;
XVI) the first antibody is the antibody or antigen-binding fragment thereof as described in (1p) of the first aspect, or a combination thereof with any one or more of (1a) to (1o), (1q) as defined in the first aspect;
the second antibody is one or more selected from (1a) to (1o), (1q), preferably (1c) or (1f), as defined in the first aspect;
or
XVII) the first antibody is the antibody or antigen-binding fragment thereof as described in (1q) of the first aspect, or a combination thereof with any one or more of (1a) to (1p) as defined in the first aspect;
the second antibody is one or more selected from (1a) to (1p) as defined in the first aspect.

In some embodiments, in the composition or TSH detection kit:

the first antibody is the antibody or antigen-binding fragment thereof as described in any one of (1b) to (1g) of the first aspect;

the second antibody is the antibody or antigen-binding fragment thereof as described in any one of (1a), (1h) to (1q) of the first aspect, or a combination thereof.

In some embodiments, the composition or TSH detection kit comprises:

the first antibody as shown in (1a) of the first aspect, and the second antibody as shown in (1c) of the first aspect;

the first antibody as shown in (1a) of the first aspect, and the second antibody as shown in (1f) of the first aspect;

the first antibody as shown in (1h) of the first aspect, and the second antibody as shown in (1f) of the first aspect;

the first antibody as shown in (1i) of the first aspect, and the second antibody as shown in (1f) of the first aspect;

the first antibody as shown in (1b) of the first aspect, and the second antibody as shown in (1i) of the first aspect;

the first antibody as shown in the first aspect (1b), and the second antibody as shown in the first aspect (1j);

The first antibody as shown in the first aspect (1c), and the second antibody as shown in the first aspect (1j);
The first antibody as shown in (1d) of the first aspect, and the second antibody as shown in (1o) of the first aspect;

the first antibody as shown in (11) of the first aspect, and the second antibody as shown in (1f) of the first aspect;

the first antibody as shown in (1n) of the first aspect, and the second antibody as shown in (1f) of the first aspect;

the first antibody as shown in (1e) of the first aspect, and the second antibody as shown in (1m) of the first aspect;

the first antibody as shown in (1e) of the first aspect, and the second antibody as shown in (1o) of the first aspect;

the first antibody as shown in (1g) of the first aspect, and the second antibody as shown in (1j) of the first aspect;

the first antibody as shown in (1p) of the first aspect, and the second antibody as shown in (1c) of the first aspect;

the first antibody as shown in (1p) of the first aspect, and the second antibody as shown in (1f) of the first aspect;

the first antibody as shown in (1f) of the first aspect, and the second antibody as shown in (1i) of the first aspect;

the first antibody as shown in (1d) of the first aspect, and the second antibody as shown in (1h) of the first aspect;

the first antibody as shown in (1d) of the first aspect, and the second antibody as shown in (1k) of the first aspect;

the first antibody as shown in (1g) of the first aspect, and the second antibody as shown in (1h) of the first aspect; or

the first antibody as shown in (1e) of the first aspect, and the second antibody as shown in (1i) of the first aspect.

In some embodiments, the composition or TSH detection kit comprises:

the first antibody as shown in (1b) of the first aspect, and the second antibody as shown in (1i) of the first aspect;

the first antibody as shown in (1b) of the first aspect, and the second antibody as shown in (1j) of the first aspect;

the first antibody as shown in (1c) of the first aspect, and the second antibody as shown in (1j) of the first aspect;

the first antibody as shown in (1d) of the first aspect, and the second antibody as shown in (1o) of the first aspect;

the first antibody as shown in (1e) of the first aspect, and the second antibody as shown in (1m) of the first aspect;

the first antibody as shown in (1e) of the first aspect, and the second antibody as shown in (1o) of the first aspect;

the first antibody as shown in (1g) of the first aspect, and the second antibody as shown in (1j) of the first aspect;

the first antibody as shown in (1f) of the first aspect, and the second antibody as shown in (1i) of the first aspect;

the first antibody as shown in (1d) of the first aspect, and the second antibody as shown in (1h) of the first aspect;

the first antibody as shown in (1d) of the first aspect, and the second antibody as shown in (1k) of the first aspect;

the first antibody as shown in (1g) of the first aspect, and the second antibody as shown in (1h) of the first aspect; or

the first antibody as shown in (1e) of the first aspect, and the second antibody as shown in (1i) of the first aspect.

In some embodiments, the first antibody is a capture antibody and the second antibody is a detection antibody.
In some embodiments, the immunoassay kit further comprises a solid phase carrier.
In some embodiments, the solid phase carrier is selected from the group consisting of magnetic particles or microtiter plates (e.g., microtiter plates or ELISA plates).
In some embodiments, the first antibody is coated on the surface of the solid phase carrier, preferably fixed on the surface of the magnetic particles.
In some embodiments, the second antibody has a detectable label.
In some embodiments, the label is selected from the group consisting of fluorescein, chemiluminescent label (e.g., acridinium ester compound), enzyme (e.g., horseradish peroxidase, alkaline phosphatase), radioisotope, biotin and colloidal gold.
In some embodiments, the second antibody is labeled with alkaline phosphatase.
In some embodiments, the kit further comprises a first antibody diluent and a second antibody diluent, wherein,

the first antibody diluent comprises one or more of the following components: a buffer solution, an inorganic salt, a preservative, a surfactant and a stabilizer; and/or
the second reagent further comprises one or more of the following components: a buffer solution, an inorganic salt, a preservative, a surfactant and a stabilizer.

In some embodiments, the first antibody diluent and the first antibody are placed in the same or different formulation units.
In some embodiments, the second antibody diluent and the second antibody are placed in the same or different formulation units.
In some embodiments, the buffer solution is selected from the group consisting of Tris, Hepes and PBS buffer solutions.
In some embodiments, the inorganic salt is selected from the group consisting of NaCl, KCl, $CaCl_2$, $MgCl_2$ and $ZnCl_2$.
In some embodiments, the preservative is selected from the group consisting of Proclin300, PC-300, BND (e.g., BND-10 or BND-99) and BIT (e.g., BIT-10).
In some embodiments, the surfactant is selected from the group consisting of Tween-20, TritonX-100 and S9.
In some embodiments, the stabilizer is selected from the group consisting of calf serum, bovine serum albumin and gelatin.
In some embodiments, the immunoassay kit further comprises one or more of the following: a TSH calibrator, a substrate of the detection label, a washing solution, a stop solution and an instruction for use.
In some embodiments, the immunoassay kit is used to detect the presence or level of TSH or a mutant thereof in a sample.

In some embodiments, the TSH or mutant thereof is selected from the group consisting of TSH dimer, TSH β-subunit or TSH R55G mutant.

In some embodiments, the sample is from a human, such as a human body fluid (e.g., blood, serum or plasma).

In some embodiments, the immunoassay kit has a detection sensitivity of ≤0.0015 μIU/mL, preferably ≤0.001 μIU/mL, for TSH or mutant thereof.

Detection method

Based on the aforementioned antibody and kit, the present application further provides a method for detecting the presence or level of TSH or a mutant thereof in a sample, which comprises the step of using the antibody or antigen-binding fragment thereof or the immunoassay kit as described in any one of the above items for detection.

In some embodiments, the method comprises the following steps:

contacting the sample with two or more kinds of the antibody or antigen-binding fragment thereof, and

qualitatively or quantitatively detecting the binding of the two or more kinds of the antibody or antigen-binding fragment thereof to the TSH or mutant thereof;

using the binding to indicate the presence or concentration of TSH or a mutant thereof in the sample, wherein,

the two or more kinds of the antibody or antigen-binding fragment thereof are directed to two or more epitopes of the TSH or mutant thereof.

In some embodiments, at least one of the two or more kinds of the antibody or antigen-binding fragment thereof is a capture antibody, and at least another is a detection antibody.

In some embodiments, the capture antibody is the first antibody as defined in any one of the preceding items.

In some embodiments, the detection antibody is the second antibody as defined in any one of the preceding items.

In some embodiments, the method comprises the following steps:

(1) contacting the sample with the capture antibody to form an antibody-antigen complex;

(2) contacting the antibody-antigen complex with the detection antibody to form an antibody-antigen-antibody complex; and

(3) determining the amount of the antibody-antigen-antibody complex.

The antibody or antigen-binding antibody fragment thereof of the present application does not have cross-reactions with other glycoprotein hormones and can be used in a single-step sandwich immunoassay, in which the capture and detection antibodies are incubated with a human blood sample simultaneously. Therefore, in some embodiments, step (1) and step (2) can be performed separately or simultaneously.

In some embodiments, the method is an ELISA immunoassay, such as a double antibody sandwich ELISA immunoassay, preferably a one-step double antibody sandwich ELISA immunoassay.

Use

In another aspect, the present application provides a use of the antibody or antigen-binding fragment thereof in the manufacture of a kit, or a use of the immunoassay kit as described in any one of the preceding items, wherein the kit is used for:

(1) detection of the presence or level of TSH or a mutant thereof in a sample;

(2) diagnosis or auxiliary diagnosis of a thyroid dysfunction (e.g., hypothyroidism or hyperthyroidism);

(3) diagnosis or auxiliary diagnosis of a thyroid-related disease; and/or

(4) evaluation or auxiliary evaluation of a therapeutic effect on a thyroid-related disease.

In another aspect, the present application provides a method, which comprises steps of detecting a sample from a subject using the antibody or antigen-binding fragment thereof or the kit described in any one of the preceding items, or detecting a sample from a subject using the method as described in any one of the preceding items, and quantifying the TSH or mutant thereof in the sample, wherein the method is used for:

(1) diagnosis or auxiliary diagnosis of a thyroid dysfunction (e.g., hypothyroidism or hyperthyroidism);

(2) diagnosis or auxiliary diagnosis of a thyroid-related disease; and/or

(3) evaluation or auxiliary evaluation of a therapeutic effect on a thyroid-related disease.

In some embodiments, the thyroid-related disease is hyperthyroidism or thyroid cancer.

In some embodiments, the sample is from a human, such as a human body fluid (e.g., blood, serum, or plasma).

Detection device

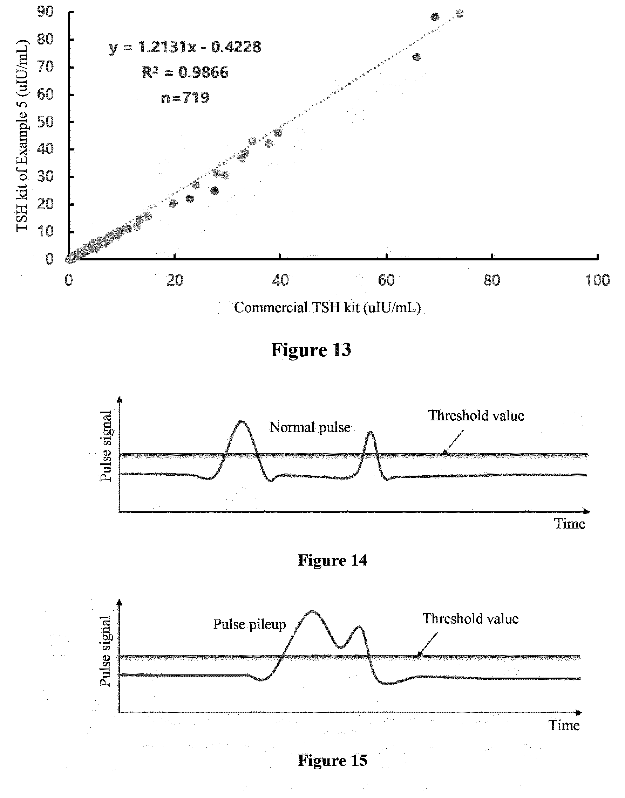

It is well known that the basic principle of photomultiplier tube is that a photon is used to output a beam of electrons through a photomultiplier tube, corresponding to an electrical pulse signal. However, since the electrical pulse signal caused by the photon entering the photomultiplier tube is randomly distributed, as the intensity of the photosignal to be measured increases, the electrical pulse signal caused by the photon entering the photomultiplier tube increases, resulting in a pulse accumulation phenomenon in which two or more electrical pulses are connected together. As shown in Figures 14 and 15, Figure 14 shows an electrical pulse signal under normal conditions, while Figure 15 shows an electrical pulse signal under pulse accumulation conditions, wherein the photon is considered to be identified when the peak value of the identified pulse is greater than a threshold. The photon counting method of the existing photometric device can accurately detect the number of photons in the case of Figure 14, but in the case of Figure 15, the accumulated multiple electrical pulses will be identified as one electrical pulse, resulting in a low counting result in the higher light intensity segment, and the upper limit of the linear range of the photometric device is insufficient. For example, the upper limit of the linear range of the existing photometric device is only 30 million photons per second. When the measured luminescence value of the sample (e.g., a high-value sample of TSH) is greater than 30 million photons per second, the test result is unreliable, and the sample usually needs to be diluted and retested, but this seriously affects the measurement speed, resulting in a longer reporting time for the sample.

The immunoassay kit of the present application can achieve ultra-sensitive detection of samples. However, due to the linear range limitation of the measurement signal of the device, one sample measurement cannot simultaneously meet the ultra-sensitive detection of low-value samples and the detection of high-value samples.

Based on the aforementioned antibody and kit, the present application further provides a sample analyzer with a high linear range, which can detect photosignals in a high linear range and can be adapted to the kit of the present application, so that the sample can be detected ultra-sensitively and accurately in one detection.

Specifically, the sample analyzer comprises the use of the antibody or antigen-binding fragment thereof or the immunoassay kit as described in any one of the preceding items.

In some embodiments, the sample analyzer comprises:

a loading device, which is configured to load a sample to be tested and a reagent required for chemiluminescent reaction into a reaction container, wherein the reagent comprises the antibody or antigen-binding fragment thereof, or the immunoassay kit;

a reaction incubation device, which is configured to provide a reaction and incubation site for the reaction container containing the sample to be tested and the reagent required for the chemiluminescent reaction, so that the sample to be tested and the reagent required for the chemiluminescent reaction in the reaction container form a sample liquid to be tested; and

a photometric device, which is configured to perform photometry on the sample liquid to be tested; wherein the photometric device comprises:

a receiving component, which is configured to receive a photosignal emitted by the sample to be tested through the chemiluminescent reaction and convert the photosignal into a corresponding electrical signal; and

a processing component, which is configured to be electrically connected to the receiving component and receiving the electrical signal from the receiving component, the processing component comprising a first photon counting module and a second photon counting module, the first photon counting module being configured to detect the number of pulses of the electrical signal using a pulse recognition method to obtain a first photon counting result, the second photon counting module being configured to process the electrical signal to obtain a parameter for characterizing photon number, and perform calculation according to the parameter for characterizing photon number and a preset calibration function to obtain a second photon counting result, wherein the calibration function represents a mapping relationship between the parameter for characterizing photon number and the photon counting result, and the processing component is further configured to output a final photon counting result of the sample to be tested based on the first photon counting result and the second photon counting result.

In some embodiments, the processing component is further configured to: output the first photon counting result as the final photon counting result when the first photon counting result is lower than a first threshold; output the second photon counting result as the final photon counting result when the first photon counting result is higher than a second threshold, wherein the second threshold is greater than or equal to the first threshold.

In some embodiments, the second photon counting module comprises an integration circuit and a second counting circuit which are electrically connected to each other, the integration circuit is configured to integrate the electrical signal within a predetermined time period to obtain a DC component signal, the parameter for characterizing photon number comprises a parameter related to the DC component signal, and the second counting circuit is configured to calculate the second photon counting result based on the parameter related to the DC component signal and the preset calibration function.

In some embodiments, the sample analyzer comprises:

a loading device, which is configured to load a sample to be tested and a reagent required for chemiluminescent reaction into a reaction container, wherein the reagent comprises the antibody or antigen-binding fragment thereof, or the immunoassay kit;

a reaction incubation device, which is configured to provide a reaction and incubation site for the reaction container containing the sample to be tested and the reagent required for chemiluminescent reaction, so that the sample to be tested and the reagent required for chemiluminescent reaction in the reaction container form a sample liquid to be tested; and

a photometric device, which is configured to measure the sample liquid to be tested in the reaction container after the reaction and incubation, wherein the linear detection range of the photometric device is [A1, A2] photons/second, wherein the linear range represents the range of output photon number of the photometric device, within which the intensity of the luminescent signal generated in the chemiluminescent reaction is linear with the output photon number of the detection device, wherein A1 is less than or equal to 2000 and A2 is greater than or equal to $10^8$.

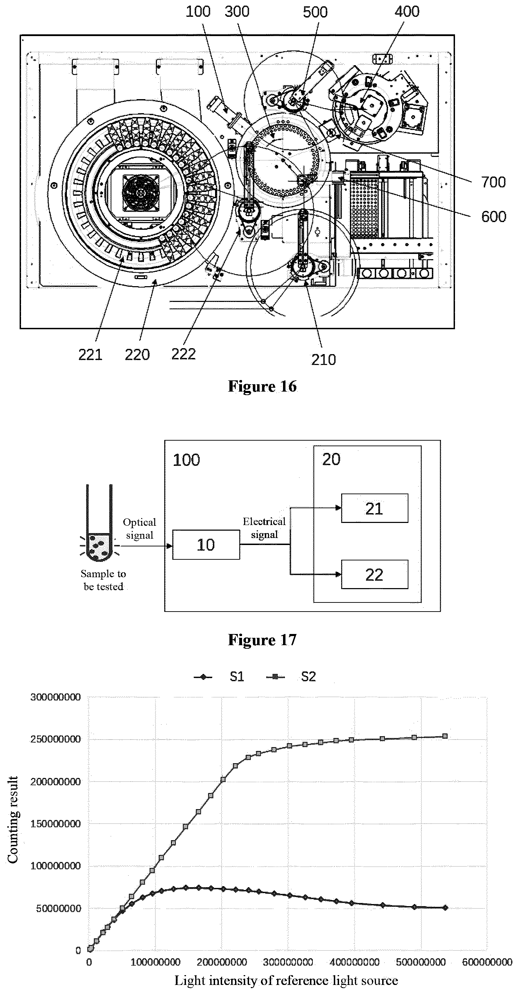

As shown in Figure 16, an embodiment of the present application further provides a sample analyzer 1, and the sample analyzer 1 comprises a loading device 200, a reaction incubation device 300, and a photometric device 100 according to any of the above embodiments.

The loading device 200 is configured to load a sample to be tested, such as a blood sample, and a reagent required for chemiluminescent reaction into a reaction container. The reaction incubation device 300 is configured to provide a reaction and incubation site for the reaction container containing the sample to be tested and the reagent required for chemiluminescent reaction, so that the sample to be tested in the reaction container and the reagent required for chemiluminescent reaction form a sample liquid to be tested. The photometric device 100 is configured to perform photometry on the sample liquid to be tested in the reaction container that has completed the reaction and incubation.

In a specific embodiment, as shown in Figure 16, the sample analyzer 1 is configured as a chemiluminescent immunoassay analyzer for analyzing a sample, such as a blood sample. The loading device 200 comprises a sample supply unit 210 and a reagent supply unit 220, the sample supply unit 210 is configured to pipette the sample to be tested and supply it to the reaction container, and the reagent supply unit 220 is configured to pipette an immune reagent (e.g., a labeled antigen or antibody) and a magnetic bead reagent and supply them to the reaction container. The chemiluminescent immunoassay analyzer further comprises a magnetic separation device 400, a substrate supply device (not shown) and a transport device 500, the magnetic separation device 400 is configured to perform a magnetic separation operation on the reaction container containing the sample to be tested, the immune reagent and the magnetic bead reagent, the substrate supply device is configured to supply a luminescent substrate to the reaction container that has completed the magnetic separation operation, and the transport device 500 is configured to transport the reaction container that has completed the magnetic separation operation from the magnetic separation device 500 to the reaction incubation device 300. After incubation, the reaction container is transported to the photometric device 100 for photometry.

In some embodiments, the reagent supply unit 220 comprises a reagent tray 221 and a reagent pipette 222, the reagent tray 221 is used to place reagent bottles containing the immune reagent and the magnetic bead reagent and has the function of refrigerating the reagent bottles. The reagent pipette 222 is used to pipette a reagent from a reagent bottle at a reagent pipetting position and discharge the pipetted reagent into the reaction container at a sample loading position.

In some embodiments, the chemiluminescent immunoassay analyzer further comprises a mixer 700 disposed between a reaction tray and a magnetic separation tray, the mixer is configured to perform mixing in the reaction container into which the reagent and sample have been added. The mixer can be configured, for example, as a vortex mixer for non-contact mixing, which can effectively avoid cross contamination.

In some embodiments, the chemiluminescent immunoassay analyzer further comprises a first manipulator 600 for transporting the reaction container, the first manipulator is configured to be able to move in three dimensions and to be able to clamp the reaction container. The first manipulator is configured to be able to load a new reaction container into the sample loading position so that the sample pipette can inject the sample. The first manipulator is further configured to be able to discard an empty reaction cup. The first manipulator is further configured to be able to transport the reaction container between the sample loading position, the reaction incubation device 300, and a dilution position.

In some embodiments, the upper limit of the linear range of photosignal detected by the photometric device 100 is greater than or equal to $10^8$ (i.e., 100 million) photons/second; under the condition that the upper limit of the linear range of the photometric device 100 is greater than or equal to $10^8$ photons/second, the upper limit of the detection range of the aforementioned kit can reach 100μIU/mL, which can achieve accurate results for high-value samples in one measurement; preferably, the linear range of photosignal detected by the photometric device 100 is greater than or equal to 120 million photons/second; further preferably, the linear range of photosignal detected by the photometric device 100 is greater than or equal to 150 million photons/second; more preferably, the linear range of photosignal detected by the photometric device 100 is greater than or equal to 200 million photons/second, and the linear detection range refers to the linear detection range of a single detection, that is, the linear detection range that can be achieved in one detection of a sample.

According to some embodiments of the photometric device 100 of the present application, as shown in Figure 17, the photometric device 100 comprises a receiving component 10 and a processing component 20.

The receiving component 10 is configured to receive the photosignal emitted by the sample to be tested, such as the blood sample to be tested, after the chemiluminescent reaction, and convert the photosignal into a corresponding electrical signal. The receiving component 10 can be configured, for example, as a photomultiplier tube that converts a weak photosignal into an electrical signal.

The processing component 20 is configured to be electrically connected to the receiving component 10 so as to receive the electrical signal from the receiving component 10, and to process the received electrical signal to obtain the number of photons.

According to the present application, the processing component 20 comprises a first photon counting module 21 and a second photon counting module 22, wherein the first photon counting module 21 is configured to process the electrical signal using a first photon counting method to obtain a first photon counting result, and the second photon counting module 22 is configured to process the electrical signal using a second photon counting method different from the first photon counting method to obtain a second photon counting result. The processing component 20 is further configured to output a final photon counting result of the sample to be tested according to the first photon counting result and the second photon counting result. The final photon counting result can then be used to calculate a content of a substance to be tested in the sample to be tested.

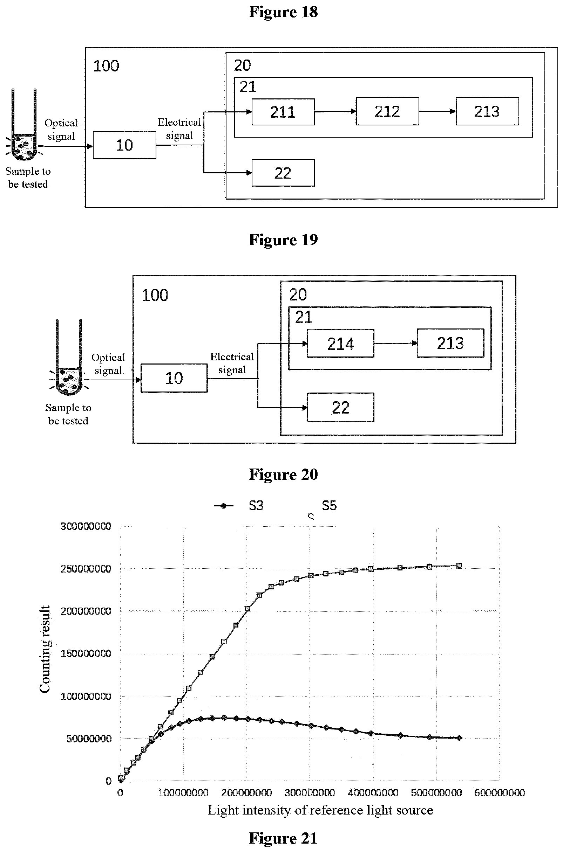

In the embodiment of the present application, two photon counting modules are used to count photons simultaneously, wherein the first photon counting module adopts a first counting method for weak light segments to obtain a first photon counting result, and the second photon counting module adopts a second counting method for strong light segments to obtain a second photon counting result, and finally the first photon counting result and the second photon counting result are integrated and a final counting result is output. This can greatly expand the linear photometric range of the photometric device. As shown in Figure 18, curve S1 represents the photometric linear characteristics of the photometric device of the prior art, and curve S2 represents the photometric linear characteristics of the photometric device according to the present application. The present application can realize the expansion of the linear photometric range of the photometric device from the original linear photometric range of 0 to 30 million photons/second (i.e., 0 to 30 million photons per second) to 0 to 200 million photons/second (i.e., 0 to 200 million photons per second).

After obtaining the first photon counting result and the second photon counting result, the processing component 20 is further configured to output a final photon counting result according to one of the following embodiments.

In some embodiments, the processing component 20 can be configured to: output the first photon counting result as the final photon counting result when the first photon counting result is lower than a first threshold; output the second photon counting result as the final photon counting result when the first photon counting result is higher than a second threshold, wherein the second threshold is greater than or equal to the first threshold. For example, when the first photon counting result is lower than 30 million photons/second, the first photon counting result is output; and when the first photon counting result is not lower than 30 million photons/second, the second photon counting result is output.

Further, the processing component can further be configured to: calculate a final photon counting result according to the first counting photon result and the second photon counting result by a preset fusion function when the first photon counting result is between the first threshold and the second threshold, wherein the second threshold is greater than the first threshold. The preset fusion function may be, for example, a function for obtaining an average value or a function for obtaining a weighted average value.

In other embodiments, multiple threshold intervals may be set for the first photon counting module. At this time, the processing component 20 can also be configured to: when the first photon counting result is in different threshold intervals, use different fusion functions to calculate the first photon counting result and the second photon counting result, and output the calculation result as the final photon counting result. Here, the fusion function can be, for example, a function for obtaining a weighted average value, and the fusion functions for different threshold intervals have different weights for the first photon counting result and the second photon counting result.

As the photometric device 10 ages and the use environment changes, the first photon counting result and the second photon counting result will show different variations, and the two will deviate near the first threshold point. Therefore, the setting of at least two thresholds can enable the photometric device to meet the linearity or precision requirements in long-term use.

Next, some embodiments of the first photon counting module 21 and the second photon counting module 22 according to the present application are described.

In some embodiments, the first counting method may be a pulse recognition method for calculating the number of photons by identifying the electrical pulses caused by the photons entering the receiving component, while the second counting method does not calculate the number of photons by identifying the electrical pulses caused by the photons entering the receiving component, but estimates the number of photons by processing the electrical signal into a parameter that can characterize the number of photons (i.e., the number of electrical pulses caused by the photons entering the receiving component).

Here, those skilled in the art can understand that in the embodiments of the present application, the pulse recognition method can be understood as a method for calculating the number of photons by identifying the pulses in the electrical signal, that is, when the peak value of the pulse identified from the electrical signal is greater than the threshold, it is considered that the photon is identified.

In some embodiments, the first photon counting module 21 can be configured to detect the number of pulses of the electrical signal using the pulse recognition method to obtain a first photon counting result, and the second photon counting module 22 can be configured to process the electrical signal to obtain a parameter for characterizing photon number, and calculate the second photon counting result according to the parameter for characterizing photon number and a preset calibration function, wherein the calibration function represents a mapping relationship between the parameter for characterizing photon number and the photon counting result.

Some embodiments of the first photon counting module 21 according to the present application are described below in conjunction with Figures 19 and 20.

In some embodiments, as shown in Figure 19, the first photon counting module 21 may comprises a level identification circuit 211, a shaping and frequency division circuit 212, and a first counting circuit 213. The level identification circuit 211 is configured to convert an electrical signal into a square wave signal, the shaping and frequency division circuit 212 is configured to perform frequency division and shaping on the square wave signal, and the first counting circuit 213 is configured to count the signal output by the shaping and frequency division circuit 212, that is, to identify the number of pulses, to obtain a first photon counting result. The first photon counting module 21 according to this embodiment can achieve a linear photometric range of 0 to 30 million photons/second.

In some other alternative embodiments, as shown in Figure 20, the first photon counting module 21 may comprise at least one first AD conversion circuit 214 and a first counting circuit 213. The first AD conversion circuit 214 is configured to collect electrical signals at a sampling frequency greater than 1 GHz/s and convert the collected electrical signals into digital signals and output them to the first counting circuit 213, the first counting circuit 213 is configured to analyze the collected digital signals to identify the number of pulses, and then obtain the first photon counting result. Thus, by high-speed sampling at a sampling frequency of 1 GHz/s, the first counting circuit 213 can more accurately identify the number of pulses in the electrical signals, and then obtain a more accurate first photon counting result.

Further, the first counting circuit 213 can also be configured to correct the first photon counting result by a Poisson distribution compensation algorithm. Thus, a more accurate photon counting result can be obtained.

In some embodiments, the first counting circuit 213 may comprise an FPGA chip and surrounding circuits thereof.

Some embodiments of the second photon counting module 22 according to the present application are described below in conjunction with Figures 21 to 24.

In an embodiment of the present application, the preset calibration function is a conversion function that converts the value of the parameter for characterizing photon number into a photon counting value, which represents an mapping relationship between the parameter for characterizing photon number and the photon counting result (or counting value).

In a specific embodiment, a reference light source, such as an LED light, and the first photon counting module of the photometric device are used to calibrate the calibration function used by the second photon counting module of the photometric device, that is, the calibration method of the conversion function of the parameter for characterizing photon number and the photon counting result is carried out as follows:

an input value of the reference light source is controlled, a photometric device to be calibrated is used to test the photon counting result of the reference light source from the weak light segment to the strong light segment and detect the value of the parameter for characterizing photon number, wherein the weak light segment has photosignals with a photon count not higher than a predetermined threshold value per second, and the strong light segment has photosignals with a photon count higher than the predetermined threshold value per second, and the predetermined threshold value is any value selected from, for example, 20 million to 30 million photons/second, such as, 30 million photons/second;

according to the linear function relationship between the weak light input value of the reference light source and the weak light photon counting result measured by the first photon counting module of the photometric device to be calibrated, a theoretical photon counting result corresponding to the strong light input value of the reference light source is calculated; and under the premise that the input value of the reference light source is fixed, the corresponding value of the parameter for characterizing photon number and the theoretical photon counting result are one-to-one corresponding, a set of values of the parameter for characterizing photon number in the strong light segment and the theoretical photon counting results are fitted, for example, by n-order polynomial weighted least-square fitting, and finally the conversion parameter of the parameter for characterizing photon number and the photon counting result, that is, the calibration function, is obtained.

In another alternative embodiment, a reference light source and a reference photometric device can also be used to calibrate the conversion function of the parameter for characterizing photon number and the photon counting result, which is perform in the following manner: an input value of the reference light source is controlled, the photometric device to be calibrated according to the present application is used to test the value of the parameter for characterizing photon number of pulses and from the weak light segment to the strong light segment of the reference light source, and the reference photometric device is used at the same time to test the counting result of the reference light source from the weak light segment to the strong light segment; according to the linear function relationship between the input value of the reference light source and the counting result of the reference photometric device, the theoretical photon counting result corresponding to the input value of the reference light source is deduced; and under the premise that the input value of the reference light source is fixed, the corresponding value of the parameter for characterizing photon number and the theoretical photon counting result are one-to-one corresponding, and a set of values of the parameter for characterizing photon number in the strong light segment and the theoretical photon counting results are fitted, for example, by n-order polynomial weighted least-square fitting, and finally the conversion parameter of the parameter for characterizing photon number and the photon counting result, i.e., the calibration function, is obtained.

It can be understood that the calibration function can be pre-stored in the photometric device, especially in the second photon counting module 22.

In an embodiment of the present application, the second photon counting module 22 that uses the parameter for characterizing photon number and the predetermined calibration function to obtain the photon counting result can achieve a linear photometric range of 10 million to 200 million photons/second, as shown in Figure 21, wherein S3 represents the photometric linear characteristics of the first photon counting module according to the embodiment shown in Figure 19, and S4 represents the photometric linear characteristics of the second photon counting module according to the embodiment of the present application. Thus, the combination of the first photon counting module 21 and the second photon counting module 22 can achieve a linear photometric range of at least 0 to 200 million photons/second.

In some embodiments, the second photon counting module 22 can be configured to process the electrical signal, for example, by integration processing to obtain a DC component signal, and the parameter for characterizing photon number comprises a parameter related to the DC component signal, such as the DC component signal itself per unit time. The second photon counting module 22 is further configured to calculate the second photon counting result according to the parameter related to the DC component signal and a preset calibration function.

Alternatively or additionally, the second photon counting module 22 is configured to integrate the electrical signal within a predetermined time period to obtain an integration result, such as a DC component signal, and the parameter for characterizing photon number comprises a parameter related to the integration result, such as an integration result per unit time. The second photon counting module 22 is further configured to calculate the second photon counting result according to the parameter related to the integration result and a preset calibration function.

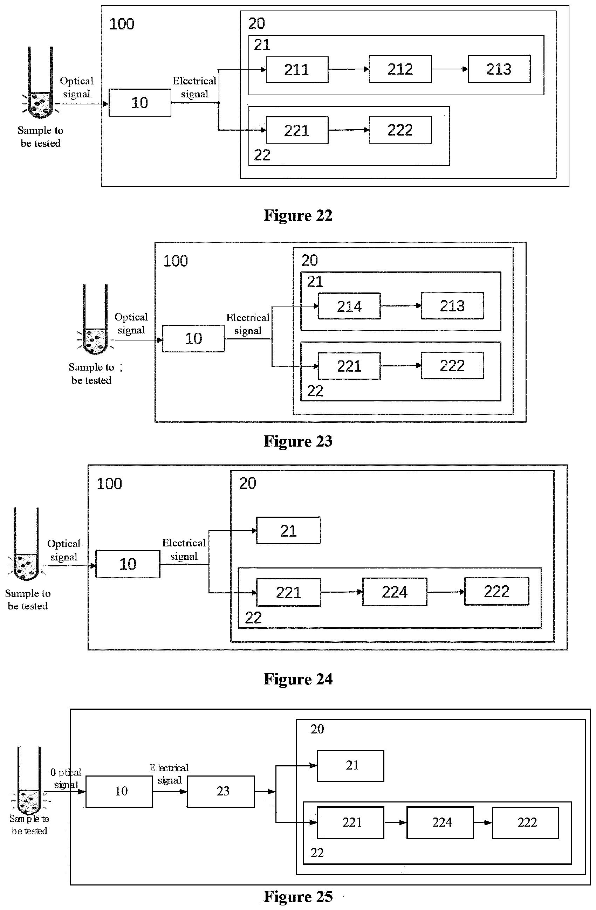

In a specific embodiment, as shown in Figures 22 and 23, the second photon counting module 22 comprises an integration circuit 221 and a second counting circuit 222 electrically connected to each other. The integration circuit 221 is configured to integrate the electrical signal within a predetermined time period to obtain a DC component signal, and the parameter for characterizing photon number comprises a parameter related to the DC component signal. The second counting circuit 222 is configured to calculate the second photon counting result according to the parameter related to the DC component signal and a preset calibration function.

Further, as shown in Figure 24 , an AD conversion circuit 224 is also provided between the integration circuit 221 and the second counting circuit 222, and the AD conversion circuit 224 is configured to convert the DC component signal output by the integration circuit 221 into a digital signal. The sampling frequency of the AD conversion circuit 224 can be set to be less than 1 MHz/s, for example, hundreds of kHz/s.

For example, the second counting circuit 222 can be configured to perform Fourier transform on the digital signal to obtain the DC component signal.

Alternatively or additionally, the second counting circuit 222 can be configured to integrate or sum the digital signal within a predetermined time period to obtain a parameter related to the integration result or the summation result as the parameter for characterizing photon number.

In addition, in some embodiments, as shown in Figure 25 , the processing component 20 ma further comprise a pre-amplifier circuit 23 electrically connected to the receiving component 10, the first photon counting module 21, and the second photon counting module 22. The pre-amplifier circuit 23 is configured to amplify the electrical signal output by the receiving component 10 and transmit it to the first photon counting module 21 and the second photon counting module 22. For example, the pre-amplifier circuit 23 can amplify the electrical signal output by the receiving component 10 by 60 to 90 times, especially by 70 to 80 times.

Of course, in other embodiments, the processing component 20 may comprise a first pre-amplifier circuit electrically connected to the receiving component 10 and the first photon counting module 21 and/or a second pre-amplifier circuit electrically connected to the receiving component 10 and the second photon counting module 22. The first pre-amplifier circuit is configured to amplify the electrical signal output by the receiving component 10 and transmit it to the first photon counting module 22, and the second pre-amplifier circuit is configured to amplify the electrical signal output by the receiving component 10 and transmit it to the second photon counting module 22.

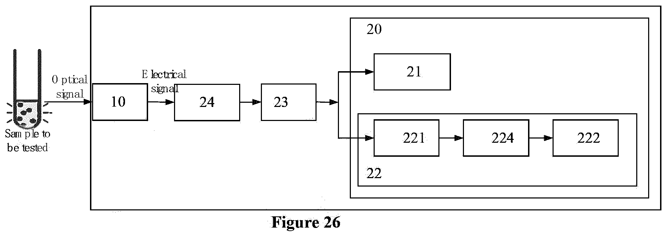

Further, as shown in Figure 26, the processing component 20 may comprise a resistor 24 disposed between the receiving component 10 and the pre-amplifier circuit 23, which is used to convert the electrical signal in the form of current signal output by the receiving component 10 into a voltage signal so that the pre-amplifier circuit 23 amplifies the voltage signal.

According to some embodiments of the photometric device 100 of the present application, the photometric device 100 comprises a receiving component 10 and a switchable light attenuation component (not shown) arranged in front of the receiving component, the light attenuation component is, for example, a filter; at the beginning of the measurement, the photosignal that has not been attenuated by the light attenuation component is received by the receiving component 10, and a weak photosignal can be measured at this time; then the light attenuation component is switched so that the receiving component 10 receives the photosignal that has been attenuated by the light attenuation component, even if it is a strong photosignal at this time, the strong photosignal becomes weak photosignal after light attenuation and is detected by the receiving component 10. The linear range of the photometric device can also be extended by the switchable light attenuation component.

Definition of terms

In the present application, unless otherwise specified, the scientific and technical terms used herein have the meanings commonly understood by those skilled in the art. In addition, the virology, biochemistry, and immunology laboratory operation steps used herein are all conventional steps widely used in the corresponding fields. At the same time, in order to better understand the present application, the definitions and explanations of relevant terms are provided below.

As used herein, the term "specific binding" refers to a non-random binding reaction between two molecules (i.e., a binding molecule and a target molecule), such as a reaction between an antibody and an antigen to which it is directed. The binding affinity between two molecules can be described by a $K_D$ value. The $K_D$ value refers to the dissociation constant obtained by the ratio of kd (dissociation rate of a specific binding molecule-target molecule interaction; also known as koff) to ka (association rate of a specific binding molecule-target molecule interaction; also known as kon), or refers to kd/ka expressed in molar concentration (M). The smaller the $K_D$ value, the tighter the two molecules bind and the higher the affinity. In certain embodiments, an antibody that specifically binds to an antigen (or an antibody that is specific to an antigen) refers to an antibody that binds to the antigen with a $K_D$ of less than about $10^{-5}$ M, such as less than about $10^{-6}$ M, $10^{-7}$ M, $10^{-8}$ M, $10^{-9}$ M or $10^{-10}$ M or less. The $K_D$ value can be determined by methods well known in the art, such as using surface plasmon resonance (SPR) in a BIACORE instrument.

As used herein, the term "immunological detection" refers to a determination made using the specific interaction/binding affinity between an antigen and an antibody, which can generally be used to detect the presence or level of a specific antigen or antibody in a sample. Such immunological detection is well known to those skilled in the art, including but not limited to enzyme immunoassay (EIA), chemiluminescent immunoassay (CLIA), radioimmunoassay (RIA), fluorescent immunoassay (FIA), Western blotting, immunoturbidimetry, surface plasmon resonance, etc. "Enzyme-linked immunosorbent assay" (i.e., ELISA) comprises binding an antigen or antibody to the surface of a solid phase carrier, and cross-linking an antigen- or antibody-related substance with an enzyme to form an enzyme conjugate, which, on the one hand, maintains the immune characteristics of binding to the corresponding antigen or antibody, and on the other hand, has enzyme activity. When the enzyme conjugate binds to the corresponding antigen or antibody, an enzyme-labeled antigen-antibody complex is formed. When the enzyme on the complex encounters the corresponding substrate, it can catalyze the hydrolysis, oxidation or reduction of the product, thereby producing a colored substance. Based on the presence or absence of the colored substance and its concentration, it can be indirectly inferred whether the corresponding antigen or antibody exists in the sample being tested and how much it is, thereby achieving the purpose of qualitative and quantitative determination. Among them, competitive ELISA has a variety of modes for detecting antibodies. The sample and enzyme-labeled antibody can compete with the solid phase antigen for binding, or the sample and antigen are added to the solid phase antibody for competitive binding, and then the enzyme-labeled antibody is added after washing to react with the antigen bound to the solid phase. For a detailed description of immunological detection, see, for example, Fundamental Immunology, Ch. 7 Paul, W., ed., 2nd edition, Raven Press, N.Y. (1989).

As used herein, the terms "antibody" and "monoclonal antibody" are used interchangeably and refer to an immunoglobulin molecule that is usually composed of two pairs of polypeptide chains (each pair having a light chain (LC) and a heavy chain (HC)). Antibody light chains can be classified as $\kappa$ (kappa) and $\lambda$ (lambda) light chains. Heavy chains can be classified as $\mu$, $\delta$, $\gamma$, $\alpha$ or $\varepsilon$, and define the isotypes of antibody as IgM, IgD, IgG, IgA and IgE, respectively. Each heavy chain consists of a heavy chain variable region (VH) and a heavy chain constant region (CH). Each light chain consists of a light chain variable region (VL) and a light chain constant region (CL). The light chain constant region consists of one domain, CL. The constant domain is not directly involved in the binding of antibody to antigen, but exhibits a variety of effector functions, such as mediating the binding of immunoglobulin to host tissues or factors, including various cells of the immune system (e.g., effector cells) and the first component (C1q) of the classical complement system. The VH and VL regions can also be subdivided into regions of high variability, called complementarity determining regions (CDRs), interspersed with more conservative regions called framework regions (FRs). Each $V_H$ and $V_L$ consists of three CDRs and four FRs arranged from the amino terminus to the carboxyl terminus in the following order: FR1, CDR1, FR2, CDR2, FR3, CDR3, FR4. The variable regions (VH and VL) of each heavy chain/light chain pair form antigen binding sites, respectively. The distribution of amino acids in each region or domain can follow the definitions of Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, the term "complementarity determining region" or "CDR" refers to the amino acid residues in an antibody variable region that is responsible for antigen binding. There are three CDRs in each of the variable regions of the heavy and light chains, designated as CDR1, CDR2, and CDR3. The precise boundaries of these CDRs can be defined according to various numbering systems known in the art, such as the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given antibody, a person skilled in the art will readily identify the CDRs defined by each numbering system. Moreover, the correspondence between different numbering systems is well known to those skilled in the art (for example, see, Lefranc et al., Dev. Comparat. Immunol. 27:55-77, 2003).

In the present application, the CDRs contained in the antibody or antigen-binding fragment thereof of the present application can be determined according to various numbering systems known in the art. In certain embodiments, the CDRs contained in the antibody or antigen-binding fragment thereof of the present application are determined by the Kabat, Chothia or IMGT numbering systems.

As used herein, the term "framework region" or "FR" residues refers to those amino acid residues in an antibody variable region other than the CDR residues defined above.

The term "antibody" is not limited to any particular method for producing antibody. For example, it includes recombinant antibody, monoclonal antibody, and polyclonal antibody. The antibody can be an antibody of different isotypes, for example, an IgG (e.g., IgG1, IgG2, IgG3, or IgG4 subtype), an IgA1, an IgA2, an IgD, an IgE, or an IgM antibody.

As used herein, the term "antigen-binding fragment" of antibody refers to a polypeptide comprising a fragment of a full-length antibody that retains the ability to specifically bind to the same antigen to which the full-length antibody binds and/or competes with the full-length antibody for specific binding to the antigen, which is also referred to as an "antigen-binding portion." See generally, Fundamental Immunology, Ch. 7 (Paul, W., ed., 2nd ed., Raven Press, N.Y. (1989), which is incorporated herein by reference in its entirety for all purposes. Antigen-binding fragments of antibodies can be produced by recombinant DNA techniques or by enzymatic or chemical cleavage of intact antibodies. Non-limiting examples of antigen-binding fragments include Fab, Fab', F(ab')$_2$, Fd, Fv, complementarity determining region (CDR) fragments, scFv, diabodies, single domain antibodies, chimeric antibodies, linear antibodies, and polypeptides that contain at least a portion of antibody sufficient to confer specific antigen binding ability on the polypeptide. Engineered antibody variants are reviewed by Holliger et al., 2005; Nat Biotechnol, 23: 1126-1136.

As used herein, the term "Fd" refers to an antibody fragment consisting of VH and CH1 domains; the term "dAb fragment" refers to an antibody fragment consisting of a VH domain (Ward et al., Nature 341:544 546 (1989)); the term "Fab fragment" refers to an antibody fragment consisting of VL, VH, CL and CH1 domains; the term "F(ab')$_2$ fragment" refers to an antibody fragment comprising two Fab fragments connected by a disulfide bridge on the hinge regions; the term "Fab' fragment" refers to a fragment obtained after reducing the disulfide bond connecting the two heavy chain fragments in the F(ab')$_2$ fragment, which consists of a complete light chain and a heavy chain Fd fragment (consisting of VH and CH1 domains).

As used herein, the term "Fv" refers to an antibody fragment consisting of the VL and VH domains of a single arm of antibody. The Fv fragment is generally considered to be the smallest antibody fragment that can form a complete antigen binding site. It is generally believed that the six CDRs confer antigen binding specificity to the antibody. However, even a single variable region (e.g., an Fd fragment, which contains only three CDRs specific for an antigen) can recognize and bind to the antigen, although its affinity may be lower than that of the complete binding site.

As used herein, the term "scFv" refers to a single polypeptide chain comprising VL and VH domains, wherein the VL and VH are connected by a linker (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994)). Such scFv molecules may have the general structure: NH$_2$-VL-linker-VH-COOH or NH$_2$-VH-linker-VL-COOH. Suitable prior art linkers consist of repeated GGGGS amino acid sequences or variants thereof. For example, a linker having the amino acid sequence (GGGGS)$_4$ may be used, but variants thereof may also be used (Holliger et al. (1993), Proc. Natl. Acad. Sci. USA 90: 6444-6448). Other linkers that can be used in the present application are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56, and Roovers et al. (2001), Cancer Immunol. In some cases, a disulfide bond may also exist between the VH and VL of the scFv.

As used herein, the term "diabody" means that its VH and VL domains are expressed on a single polypeptide chain, but using a linker that is too short to allow pairing between the two domains of the same chain, thereby forcing the domains to pair with the complementary domains of another chain and create two antigen binding sites (see, for example, Holliger P. et al., Proc. Natl. Acad. Sci. USA 90:6444-6448 (1993), and Poljak R. J. et al., Structure 2:1121-1123 (1994)).

As used herein, the term "single-domain antibody (sdAb)" has the meaning commonly understood by those skilled in the art, which refers to an antibody fragment composed of a single monomer variable antibody domain (e.g., a single heavy chain variable region) that retains the ability to specifically bind to the same antigen to which the full-length antibody binds. Single-domain antibody is also called nanobody.

Each of the above antibody fragments retains the ability to specifically bind to the same antigen to which the full-length antibody binds, and/or competes with the full-length antibody for specific binding to the antigen.

Antigen-binding fragment of antibody (e.g., the above antibody fragment) can be obtained from a given antibody (e.g., the antibody provided by the present application) using conventional techniques known to those skilled in the art (e.g., recombinant DNA technology or enzymatic or chemical cleavage methods), and the antibody antigen-binding fragment can be screened for specificity in the same manner as for the intact antibody.

In this article, unless the context clearly indicates otherwise, when referring to the term "antibody", it includes not only an intact antibody, but also an antigen-binding fragment of the antibody.

As used herein, the term "chimeric antibody" refers to an antibody in which a portion of its light chain or/and heavy chain is derived from one antibody (which may be derived from a particular species or belong to a particular antibody class or subclass), and another portion of the light chain or/and heavy chain is derived from another antibody (which may be derived from the same or different species or belong to the same or different antibody class or subclass), but in any case, it still retains the activity to bind to the target antigen (U.S.P 4,816,567 to Cabilly et al.; Morrison et al., Proc. Natl. Acad. Sci. USA, 81:6851 6855 (1984)). For example, the term "chimeric antibody" may include such an antibody (e.g., a human-mouse chimeric antibody) in which the heavy chain and light chain variable regions of the antibody are derived from a first antibody (e.g., a murine antibody), and the heavy chain and light chain variable regions of the antibody are derived from a second antibody (e.g., a human antibody).

As used herein, the term "humanized antibody" refers to a genetically engineered non-human antibody whose amino acid sequence is modified to increase homology with the amino acid sequence of a human antibody. Generally speaking, all or part of the CDR regions of a humanized antibody are derived from a non-human antibody (donor antibody), and all or part of the non-CDR regions (e.g., variable region FR and/or constant region) are derived from a human immunoglobulin (recipient antibody). Humanized antibodies generally retain the expected properties of the donor antibody, including, but not limited to, antigen specificity, affinity, reactivity, etc. The donor antibody may be a mouse, rat, rabbit, or non-human primate (e.g., cynomolgus monkey) antibody having the expected properties (e.g., antigen specificity, affinity, reactivity, etc.).

The chimeric antibody or humanized antibody of the present application can be prepared according to the sequence of the mouse monoclonal antibody prepared above. DNA encoding the heavy and light chains can be obtained from the target mouse hybridoma and engineered to contain non-mouse (e.g., human) immunoglobulin sequences using standard molecular biology techniques.

To prepare chimeric antibodies, methods known in the art can be used to connect mouse immunoglobulin variable regions to human immunoglobulin constant region. For example, the DNA encoding VH can be operably connected to another DNA molecule encoding the heavy chain constant region to obtain a full-length heavy chain gene. The sequence of the human heavy chain constant region gene is known in the art (see, for example, Kabat, E.A. et al. (1991) Sequences of Proteins of Immunological Interest, Fifth Edition, U.S. Department of Health and Human Services, NIH Publication No.91-3242), and DNA fragments containing these regions can be obtained by standard PCR amplification. The heavy chain constant region can be IgG1, IgG2, IgG3, IgG4, IgA, IgE, IgM or IgD constant region, but IgG1 or IgG4 constant region is generally preferred. For example, the DNA encoding VL can be operably connected to another DNA molecule encoding the light chain constant region CL to obtain a full-length light chain gene (as well as a Fab light chain gene). The sequence of human light chain constant region gene is known in the art (see, for example, Kabat, E.A. et al. (1991), Sequences of Proteins of Immunological Interest, Fifth Edition, U.S. Department of Health and Human Services, NIH Publication No. 91-3242), and DNA fragments containing these regions can be obtained by standard PCR amplification. The light chain constant region can be a κ or λ constant region, but a κ constant region is generally preferred.

To prepare humanized antibodies, mouse CDR regions can be inserted into human framework sequences using methods known in the art (see Winter's U.S. Patent No. 5,225,539; Queen et al.'s U.S. Patent No.os.5,530,101; 5,585,089; 5,693,762 and 6,180,370; and Lo, Benny, K.C., editor, in Antibody Engineering: Methods and Protocols, volume 248, Humana Press, New Jersey, 2004).

As used herein, the term "vector" refers to a nucleic acid vehicle into which a polynucleotide can be inserted. When a vector can express a protein encoded by the inserted polynucleotide, the vector is called an expression vector. The vector can be introduced into a host cell by transformation, transduction or transfection so that the genetic material elements it carries are expressed in the host cell. Vectors are well known to those skilled in the art, and include but are not limited to: plasmids; phagemids; cosmids; artificial chromosomes, such as yeast artificial chromosome (YAC), bacterial artificial chromosome (BAC) or P1-derived artificial chromosome (PAC); bacteriophages, such as λ phage or M13 phage, as well as animal viruses, etc. Animal viruses that can be used as vectors include, but are not limited to, retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including, but not limited to, promoter sequence, transcription initiation sequence, enhancer sequence, selection element, and reporter gene. In addition, the vector may also contain a replication origin.

As used herein, the term "host cell" refers to a cell that can be used to introduce a vector, including, but not limited to, prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell, or human cell.

As used herein, the term "identity" is used to refer to the matching of sequences between two polypeptides or between two nucleic acids. When a position in both sequences being compared is occupied by the same base or amino acid monomer subunit (e.g., a position in each of the two DNA molecules is occupied by adenine, or a position in each of the two polypeptides is occupied by lysine), then the molecules are identical at that position. The "percent identity" between two sequences is a function of the number of matching positions shared by the two sequences divided by the number of positions being compared $\times 100$. For example, if 6 out of 10 positions in two sequences match, then the two sequences have an identity of 60%. For example, the DNA sequences CTGACT and CAGGTT share 50% identity (3 out of a total of 6 positions match). Typically, two sequences are compared when they are aligned to produce maximum identity. Such an alignment can be achieved by using, for example, the method of Needleman et al. (1970) J. Mol. Biol. 48: 443-453, which can be conveniently performed by a computer program such as the Align program (DNAstar, Inc.). The percent identity between two amino acid sequences can also be determined using the algorithm of E. Meyers and W. Miller (Comput. Appl Biosci., 4:11-17 (1988)), which has been incorporated into the ALIGN program (version 2.0), using a PAM120 weight residue table, a gap length penalty of 12, and a gap penalty of 4. In addition, the percent identity between two amino acid sequences can be determined using the algorithm of Needleman and Wunsch (J Mol Biol. 48:444-453 (1970)), which has been incorporated into the GAP program of the GCG software package (available at www.gcg.com), using a Blossum 62 matrix or a PAM250 matrix, a gap weight of 16, 14, 12, 10, 8, 6, or 4, and a length weight of 1, 2, 3, 4, 5, or 6.

As used herein, the term "conservative substitution" refers to an amino acid substitution that does not adversely affect or change the expected properties of the protein/polypeptide comprising the amino acid sequence. For example, a conservative substitution can be introduced by standard techniques known in the art such as site-directed mutagenesis and PCR-mediated mutagenesis. Conservative amino acid substitutions include substitutions of amino acid residues with amino acid residues having similar side chains, such as substitutions with residues physically or functionally similar to the corresponding amino acid residues (e.g., having similar size, shape, charge, chemical properties, including the ability to form covalent bond or hydrogen bond, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which are incorporated herein by reference).

The twenty conventional amino acids referred to herein are written in accordance with conventional usage. See, for example, Immunology-A Synthesis (2nd Edition, E. S. Golub and D. R. Gren, Eds., Sinauer Associates, Sunderland, Mass. (1991)), which is incorporated herein by reference.

In the present application, the terms "polypeptide" and "protein" have the same meaning and are used interchangeably. And in the present application, amino acids are generally represented by single-letter and three-letter abbreviations known in the art. For example, alanine can be represented by A or Ala.

As used herein, the term "subject" includes, but is not limited to, various animals, particularly mammals, such as human.

In this article, unless otherwise stated, "%" refers to mass percentage.

## Brief Description of the Drawings

The drawings described herein are used to provide a further understanding of the present application and constitute a part of the present application. The schematic examples of the present application and their descriptions are used to explain the present application and do not constitute an improper limitation of the present application. In the drawings:

Figure 1 shows the cross-reactivity test of antibodies with human glycoprotein hormones TSH, FSH, LH and CG, wherein the common α subunit-specific antibodies of glycoprotein hormones were used as positive controls for FSH, LH and CG immunoreactivity.

Figure 2 shows the cross-reactivity test of antibodies with human glycoprotein hormones FSH (10 IU/ml), LH (10 IU/ml) and CG (1000 IU/ml), wherein TSH at a concentration of $0.137\mu$IU/ml was used as a control.

Figure 3 shows the cross-reactivity test of antibodies with human recombinant wild-type TSH and TSH variant R55G.

Figure 4 shows the functional sensitivity test results of the kit of Example 7.

Figure 5 shows the functional sensitivity test results of the kit of Example 8.

Figure 6 shows the functional sensitivity test results of the kit of Example 9.

Figure 7 shows the functional sensitivity test results of the kit of Example 10.

Figure 8 shows the functional sensitivity test results of the kit of Example 11.

Figure 9 shows the methodological comparison results of the kit of Example 7 and the commercial TSH kit.

Figure 10 shows the methodological comparison results of the kit of Example 8 and the commercial TSH kit.

Figure 11 shows the methodological comparison results of the kit of Example 9 and the commercial TSH kit.

Figure 12 shows the methodological comparison results of the kit of Example 10 and the commercial TSH kit.

Figure 13 shows the methodological comparison results of the kit of Example 11 and the commercial TSH kit.

Figure 14 shows the schematic curve graph of a pulse signal with normal pulse.

Figure 15 shows the schematic curve graph of a pulse signal with pile-up pulse.

Figure 16 shows the schematic structural diagram of an example of chemical immunoassay analyzer according to the present application.

Figure 17 shows the curve graphs of the photometric linear characteristics of the photometric device of the prior art and the photometric device according to the present application.

Figure 18 shows the schematic block diagram of the first example of photometric device according to the present application.

Figure 19 shows the schematic block diagram of the second example of photometric device according to the present application.

Figure 20 shows the curve graphs of photometric linear characteristics of the first photon counting module and the second photon counting module according to the present application.

Figure 21 shows the schematic block diagram of the third example of photometric device according to the present application.

Figure 22 shows the schematic block diagram of the fourth example of photometric device according to the present application.

Figure 23 shows the schematic block diagram of the fifth example of photometric device according to the present application.

Figure 24 shows the schematic block diagram of the sixth example of photometric device according to the present application.

Figure 25 shows the schematic block diagram of the seventh example of photometric device according to the present application.

Figure 26 shows the schematic block diagram of the fifth example of photometric device according to the present application.

## Embodiments of the present Application

The following will describe the embodiments of the present application in detail in conjunction with the examples, but those skilled in the art will understand that the following examples are only used to illustrate the present application and should not be regarded as limiting the scope of the present application. If the specific conditions are not specified in the examples, they should be carried out according to conventional conditions or conditions recommended by the manufacturer. The reagents or instruments used without indicating the manufacturer were all conventional products that could be purchased commercially.

Example 1: Preparation of monoclonal antibodies

1. Immunization of rabbits and sheep with human TSH

Recombinant human thyroid stimulating hormone (TSH) (the protein did not contain any tags and its sequence was the same as that of natural TSH (WHO International Standard NIBSC code 81/565)) was used to immunize rabbits and sheep. The immunized hybridoma cells were then screened to obtain hybridoma cells that produce human thyroid stimulating hormone (TSH).

2. Screening for antibodies without cross-reactivity with other glycoprotein hormones

ELISA was used to detect the presence of antibodies specific to the common $\alpha$ subunit of free human glycoprotein hormones, human luteinizing hormone (LH), human follicle stimulating hormone (FSH) and human chorionic gonadotropin (hCG) in the hybridoma culture supernatant. Even hybridomas that only produced antibodies with weak cross-reactivity to free α subunits, LH, FSH or CG were excluded. Rabbit-mouse hybridomas and sheep-mouse hybridomas that secreted antibodies that did not cross-react with other glycoprotein hormones were further selected.

3. Selection of sheep antibodies capable of recognizing TSH with R55G mutation in TSH β subunit

To select antibodies that recognized TSH with R55G mutation in β subunit (TSH R55G mutant), we developed recombinant human TSH containing this mutation. Table 1 provided the sequences encoding the α- and β-subunits. The β-subunit contained glycine instead of arginine at position 55 (R55G mutation).

Table 1: Amino acid sequence of TSH mutant R55G

| TSH α-subunit | APDVQDCPECTLQENPFFSQPGAPILQCMGCCFSRAYPTPLRSKKTM LVQKNVTSESTCCVAKSYNRVTVMGGFKVENHTACHCSTCYYHKS |
|---|---|
| TSH β-subunit containing R55G mutation | FCIPTEYTMHIERRECAYCLTINTTICAGYCMTRDINGKLFLPKYALS QDVCTYGDFIYRTVEIPGCPLHVAPYFSYPVALSCKCGKCNTDYSDC IHEAIKTNYCTKPQKSYLVGFSV |

Culture supernatants from sheep-mouse hybridomas were tested with wild-type TSH and TSH mutant R55G by ELISA. Hybridomas that recognized both wild-type TSH and TSH variant R55G were selected. In the later stages of antibody development, the interaction of rabbit antibodies with TSH R55G variant was studied on purified antibody preparations.

4. Screening of antibodies capable of recognizing TSH with high sensitivity

The hybridoma with the highest signal-to-noise ratio in the chemiluminescent sandwich immunoassay was selected.

TSH-specific high-affinity polyclonal antibodies were separated from the blood of immunized rabbits and sheep for sandwich immunoassay. Among them, rabbit polyclonal antibodies were used as capture antibodies in the screening of sheep antibodies. Sheep polyclonal antibodies were used as capture antibodies in the screening of rabbit antibodies. The hybridomas were graded based on the signal-to-noise ratios. Rabbit-mouse hybridomas and sheep-mouse hybridomas that showed the highest signal-to-noise ratio were selected for the development of recombinant forms. Some antibody sequencing results were shown in the section "5. Antibody sequencing".

5. Antibody sequencing

Total RNA was extracted from about 50 to 500 hybridoma cells using the Norgen Single Cell Purification Kit (Norgen Biotek) and sequenced according to routine procedures in the field. The nucleotide sequences and deduced amino acid sequences of all three CDRs of the VH, VK, and VL domains were given in Table 2 and Table 3. Among them, TS01 was a rabbit antibody and the rest were sheep antibodies.

Table 2: Partial sequencing results of heavy chains (nucleotide sequence + amino acid sequence)

| Antibody name | Kind of sequence | Heavy chain | | |
|---|---|---|---|---|
| | | CDR1 | CDR2 | CDR3 |
| TS01 | Nucleotide sequence | acctactactactacatgtgc | tgtattgaggctggtggtagtggtaccacttactacgcgaactgggcgaaaggc | tttttgggttacgctagtgatggtggtgcttatattaacgccttcaatttg |
| | Amino acid sequence | TYYYYMC | CIEAGGSGTTYYAN WAKG | FLGYASDGGAYIN AFNL |

(continued)

| Antibody name | Kind of sequence | Heavy chain | | |
|---|---|---|---|---|
| | | CDR1 | CDR2 | CDR3 |
| TS02 | Nucleotide sequence | agcaatgctgtaggc | ggcatgagtagtggtggaagt acatactataacccggccctg aaatcc | gttgatgcgactcgtcctgatg atattggttggatcgactac |
| | Amino acid sequence | SNAVG | GMSSGGSTYYNPA LKS | VDATRPDDIGWID Y |
| TS03 | Nucleotide sequence | agcaatgctgtcggc | ggcataagtagtggtggcaca gcatactataatccggccctga aatcc | gttgacgcgactcgtcctgat gatattggttggatcgactat |
| | Amino acid sequence | SNAVG | GISSGGTAYYNPAL KS | VDATRPDDIGWID Y |
| TS04 | Nucleotide sequence | aattatgatgtaggc | gacatctatagtggtggaggt aaaagctataacccggccctg aaatcc | ggggtctatagtggtaatggt attgctgatgctgctgatgtc |
| | Amino acid sequence | NYDVG | DIYSGGGKSYNPAL KS | GVYSGNGIADAAD V |
| TS05 | Nucleotide sequence | atgtatagtataacc | gatatatggggtgatggaagt acgctctataacccggccctc aaatcc | ggtgttgggacgactgtgaca gaagtcgacgac |
| | Amino acid sequence | MYSIT | DIWGDGSTLYNPA LKS | GVGTTVTEVDD |
| TS06 | Nucleotide sequence | agcaatgctgtaggc | ggcataagtagtggtggaagt gcatactataacccggccctg aaatcc | gttgatgcgactcgtcctgatg atattggttgggtcgacttc |
| | Amino acid sequence | SNAVG | GISSGGSAYYNPAL KS | VDATRPDDIGWVD F |
| TS07 | Nucleotide sequence | agcaatgctgtcggc | ggcataagtagtggtggcact acatactataatccggccctga aatcc | gttgatgcgactcgtcctgatg atattggttggatcgactat |
| | Amino acid sequence | SNAVG | GISSGGTTYYNPAL KS | VDATRPDDIGWID Y |
| TS08 | Nucleotide sequence | agctatagtgtaaac | gatataagtacttatggaaaca cagtctataacccgaccctgg aatcc | cttcacttgactgatagtagtc atactcgaaacggtgtggatg tc |
| | Amino acid sequence | SYSVN | DISTYGNTVYNPTL ES | LHLTDSSHTRNGV DV |

(continued)

| Antibody name | Kind of sequence | Heavy chain | | |
|---|---|---|---|---|
| | | CDR1 | CDR2 | CDR3 |
| TS09 | Nucleotide sequence | agctataatgtagtc | gaaatagaacctggtggaagt acattcagtaacccggccctg aaatcc | gcttcgtataccgatgcactgc ga |
| | Amino acid sequence | SYNVV | EIEPGGSTFSNPALK S | ASYTDALR |
| TS10 | Nucleotide sequence | agctatagtgtaaac | gatataagcacttatggaaaca cagtctataacccggccctgg aatcc | cttcgcttgagtgaaggtggtc acactcgaaacggtgtagatg tc |
| | Amino acid sequence | SYSVN | DISTYGNTVYNPAL ES | LRLSEGGHTRNGV DV |
| TS11 | Nucleotide sequence | agcaatgctgtacac | gttatagttagtactgggagca cagcctataacccggccctga atcc | acaataggtggtgatgactgg actagtgatgtagaacatatcg actac |
| | Amino acid sequence | SNAVH | VIVSTGSTAYNPAL KS | TIGGDDWTSDVEHI DY |
| TS12 | Nucleotide sequence | agcgatgctgtaact | atcatatatagtggtggaagta catcttataatcagaccctgaa atcc | agtaattttggttatgattccga tctcgactac |
| | Amino acid sequence | SDAVT | IIYSGGSTSYNQTL KS | SNFGYDSDLDY |
| TS13 | Nucleotide sequence | accaaggcggtacac | gttattgctagtagtgggagca cagcctataacccggccctga atcc | acaataggtggtgatgactgg cgtagtgatgtggagcatatc gactac |
| | Amino acid sequence | TKAVH | VIASSGSTAYNPAL KS | TIGGDDWRSDVEH IDY |
| TS14 | Nucleotide sequence | agctataatttagtc | gaaatagaacctggtggaagt acattctataacccggccctga atcc | gcttcgcataccgatttcctgc ga |
| | Amino acid sequence | SYNLV | EIEPGGSTFYNPAL KS | ASHTDFLR |
| TS15 | Nucleotide sequence | agcaatgctgtacac | ggtatagttagtagtggaagc acagcctataacccggccctg aaatcc | acaataggtggtgatgactgg actagtaatgtagagcatatcg actac |
| | Amino acid sequence | SNAVH | GIVSSGSTAYNPAL KS | TIGGDDWTSNVEHI DY |

(continued)

| Antibody name | Kind of sequence | Heavy chain | | |
|---|---|---|---|---|
| | | CDR1 | CDR2 | CDR3 |
| TS16 | Nucleotide sequence | acctatgctgtaact | atcatatatagtggtggaagta catcttataaccagaccctgaa atcc | agtaattttggttatgattccga tctcgactac |
| | Amino acid sequence | TYAVT | IIYSGGSTSYNQTL KS | SNFGYDSDLDY |
| TS17 | Nucleotide sequence | aacgatggtgtaggc | gcgatttacagtagtggaggt acatactataatccggccctga aatcc | cacttttaccgtgattgggcta gtgggtctgatatggtttctttc gactac |
| | Amino acid sequence | NDGVG | AIYSSGGTYYNPAL KS | HFYRDWASGSDM VSFDY |

Table 3: Partial sequencing results of light chain (nucleotide sequence + amino acid sequence)

| Antibody name | Kind of sequence | Light chain | | |
|---|---|---|---|---|
| | | CDR1 | CDR2 | CDR3 |
| TS01 | Nucleotide sequence | caggccagtcagaacattg gtagtaatttagcc | agggcatccactctggaatct | caatgtactgtttatggtagta gtgatgtttttgct |
| | Amino acid sequence | QASQNIGSNLA | RASTLES | QCTVYGSSDVFA |
| TS02 | Nucleotide sequence | tctggaagcgacatcggta gtagtgcagtaggc | ggtactgtccgtcgaccctca | ggaggtgctgctggtagtaac tatggtgat |
| | Amino acid sequence | SGSDIGSSAVG | GTVRRPS | GGAAGSNYGD |
| TS03 | Nucleotide sequence | tctggaagcgacttcggta gtagtgcagtaggc | ggtactaccaggcgaccctca | ggaggtgctgctggttctgac catggtgat |
| | Amino acid sequence | SGSDFGSSAVG | GTTRRPS | GGAAGSDHGD |
| TS04 | Nucleotide sequence | tctggaagctacatcgatag taggcatgtaggc | tatagtaccaatcgaccctca | ggaagttatgctgcgagtatc gatgagggtatt |
| | Amino acid sequence | SGSYIDSRHVG | YSTNRP | GSYAASIDEGI |
| TS05 | Nucleotide sequence | cagggagacctgctggac gatcaatatacagct | aaagacactgagcggccttca | ctgtcacttgacagcagcaga atgggtgtt |
| | Amino acid sequence | QGDLLDDQYTA | KDTERPS | LSLDSSRMGV |
| TS06 | Nucleotide sequence | tctggaagcgacttctttagt aatgcagtaggc | ggtactacccgtcgaccctca | ggaagtactgcccgtagtaac tatggtgat |
| | Amino acid sequence | SGSDFFSNAVG | GTTRRPS | GSTARSNYGD |

(continued)

| Antibody name | Kind of sequence | Light chain | | |
|---|---|---|---|---|
| | | CDR1 | CDR2 | CDR3 |
| TS07 | Nucleotide sequence | tctggaagcgacttcggtagtagtgcagtaggc | ggtactactaggcgaccctca | ggaggtgctgctggtactaaccatggtgat |
| | Amino acid sequence | SGSDFGSSAVG | GTTRRPS | GGAAGTNHGD |
| TS08 | Nucleotide sequence | cagggagacataggaagctcttatgttgcg | caaaatagtaagaggccctcg | ctgtcagctgacagccgttttgtt |
| | Amino acid sequence | QGDIGSSYVA | QNSKRPS | LSADSRFV |
| TS09 | Nucleotide sequence | tctggaagcagcagcaacatcggggggtggttattatgtgggc | caaaacagcaaacgaccgaca | tcaacttatgacagcagtattagtgctagtgtt |
| | Amino acid sequence | SGSSSNIGGGYYVG | QNSKRPT | STYDSSISASV |
| TS10 | Nucleotide sequence | cagggagacgacataggaaggtcttatgttgcg | caaaatagtaagaggccctcg | ctgtcagctggcagcaggtttgtt |
| | Amino acid sequence | QGDDIGRSYVA | QNSKRPS | LSAGSRFV |
| TS11 | Nucleotide sequence | tctggaagcagcaggaacgttggtgaatatggtgtaggc | ggtactagtagtcgaccctcg | gcaactactgacagcagtagaaggaatgttgtt |
| | Amino acid sequence | SGSSRNVGEYGVG | GTSSRPS | ATTDSSRRNVV |
| TS12 | Nucleotide sequence | tctggaagcagcagcaacgttggatatggtaattatgtgggc | ggtgcaaccaatcgagcctcg | gcatcttatgacagcagtagtagaattgtt |
| | Amino acid sequence | SGSSSNVGYGNYVG | GATNRAS | ASYDSSSRIV |
| TS13 | Nucleotide sequence | tctggaagcagcaggaacgttggtgaatatggtgtagct | ggtactagcaatcgaccctcg | gcagctactgatagcagtagcgcgggatgttgtt |
| | Amino acid sequence | SGSSRNVGEYGVA | GTSNRPS | AATDSSRRDVV |
| TS14 | Nucleotide sequence | tctggaagcagcagcaacatcggggggtggttattatgtgggc | caaaacagcaaacgaccgaca | tcaacttatgacagcagtattagtactactgtt |
| | Amino acid sequence | SGSSSNIGGGYYVG | QNSKRPT | STYDSSISTTV |

(continued)

| Antibody name | Kind of sequence | Light chain | | |
|---|---|---|---|---|
| | | CDR1 | CDR2 | CDR3 |
| TS15 | Nucleotide sequence | tctggaagcagcaggaac gttggtgaatatggtgtagg c | ggtactcgtagtcgaccctcg | gcaactactgacagcagtag aaggaatgttgtt |
| | Amino acid sequence | SGSSRNVGEYGV G | GTRSRPS | ATTDSSRRNVV |
| TS16 | Nucleotide sequence | tcgggaagcagcagcaac gttggatatggtaattatgtg ggc | ggtgcaaccaatcgagcctcg | gcatcttatgacatcaacagta ggattatt |
| | Amino acid sequence | SGSSSNVGYGNY VG | GATNRAS | ASYDINSRII |
| TS17 | Nucleotide sequence | tctggaagcagcggcaac gttggatttggtgattatgtg gcc | cgtgcaacgagtcgagcctcg | gcctcttttgacagcagtgcc agtggtatt |
| | Amino acid sequence | SGSSGNVGFGDY VA | RATSRAS | ASFDSSASGI |

6. Production and affinity purification of antibodies

Antibodies were obtained in recombinant form. Design of appropriate molecular genetic constructs was performed. To develop molecular genetic constructs of rabbit antibodies, variable domains of antibodies and constant domains of rabbit immunoglobulins of heavy chain IgG isotype and light chain λ isotype were used. To develop molecular genetic constructs of sheep antibodies, variable domains of antibodies and constant domains of human immunoglobulins of heavy chain IgG1 isotype and light chain λ isotype were used. Molecular genetic constructs of recombinant antibodies were obtained in preparative amounts in bacterial cells and purified. Expi293F cells were transfected with molecular genetic constructs of recombinant antibodies.

For example, the light chain and heavy chain gene sequences were transferred to the open reading frame (ORF) of the expression vector through homologous recombination or restriction enzyme cleavage, and finally the "promoter-antibody light chain gene-terminator" and "promoter-antibody heavy chain gene-terminator" structures were formed on the two expression vectors, respectively. Then, the two expression vectors (antibody light chain expression vector and heavy chain expression vector) were simultaneously transferred into the mammalian cell line Expi293F for expression through biological, physical and chemical methods. Finally, the two light chains and two heavy chains were reassembled into a complete antibody in the cell and secreted into the culture supernatant. Alternatively, the expression vector was modified so that one expression vector contained two open reading frames, and then the light chain and heavy chain gene sequences were inserted into the two open reading frames (ORFs) of the expression vector by homologous recombination or restriction enzyme cleavage, respectively, to form a "promoter-antibody light chain gene-terminator-vector sequence-promoter-antibody heavy chain gene-terminator" or "promoter-antibody heavy chain gene-terminator-vector sequence-promoter-antibody light chain gene-terminator" structure, and then the expression vector containing both antibody light chain and heavy chain was transferred into the mammalian cell line Expi293F for expression by biological, physical and chemical methods, and finally the two light chains and two heavy chains were reassembled into a complete antibody in the cell and secreted into the culture supernatant.

Antibodies were purified from conditioned culture medium by using protein A affinity chromatography (Mab Select SuRe, Cytiva). The supernatant was loaded onto a column containing Mab Select SuRe equilibrated with PBS. The column was washed with PBS and the antibody was eluted with 0.1 M citric acid (pH 3.0). The eluate was then neutralized and dialyzed against PBS containing 0.09% sodium azide. The antibody solution was concentrated using an Amicon Ultra-15 centrifugal filter 50 kDa MWCO (Millipore). Recombinant rabbit monoclonal antibody rTS01 and recombinant chimeric antibody hTS02-hTS17 were obtained. Antibody purity was analyzed by SDS-PAGE under reducing conditions and size exclusion chromatography using Superdex 200 Increase 5/150 column (Cytiva). The antibodies had a purity of > 90%.

Deposit information:

hTS01: *Escherichia coli* Rosetta™ (DE3)pLysS hTS01 VH was deposited at VKPM on July 10, 2024, having Accession Number B-14885, and the plasmid in the *E.coli* is used to produced heavy chain of hTS01, and *Escherichia coli* Rosetta™(DE3)pLysS hTS01 VL was deposited at VKPM on July 10, 2024, having Accession Number B-14884, and the plasmid in the E.coli is used to produced light chain of hTS01l;

hTS02: *Escherichia coli* Rosetta™ (DE3)pLysS hTS02 VH was deposited at VKPM on July 10, 2024, having Accession Number B-14887, and the plasmid in the *E.coli* is used to produced heavy chain of hTS02, and *Escherichia coli* Rosetta™(DE3)pLysS hTS02 VL was deposited at VKPM on July 10, 2024, having Accession Number B-14886, and the plasmid in the E.coli is used to produced light chain of hTS02;

hTS03 *Escherichia coli* Rosetta™ (DE3)pLysS hTS03 VH was deposited at VKPM on July 10, 2024, having Accession Number B-14889, and the plasmid in the *E.coli* is used to produced heavy chain of hTS03, and *Escherichia coli* Rosetta™(DE3)pLysS hTS03 VL was deposited at VKPM on July 10, 2024, having Accession Number B-14888, and the plasmid in the E.coli is used to produced light chain of hTS03;

hTS04: *Escherichia coli* Rosetta™ (DE3)pLysS hTS04 VH was deposited at VKPM on July 10, 2024, having Accession Number B-14891, and the plasmid in the *E.coli* is used to produced heavy chain of hTS04, and *Escherichia coli* Rosetta™(DE3)pLysS hTS04 VL was deposited at VKPM on July 10, 2024, having Accession Number B-14890, and the plasmid in the E.coli is used to produced light chain of hTS04;

hTS05: *Escherichia coli* Rosetta™ (DE3)pLysS hTS05 VH was deposited at VKPM on July 10, 2024, having Accession Number B-14893, and the plasmid in the *E.coli* is used to produced heavy chain of hTS05, and *Escherichia coli* Rosetta™(DE3)pLysS hTS05 VL was deposited at VKPM on July 10, 2024, having Accession Number B-14892, and the plasmid in the E.coli is used to produced light chain of hTS05;

hTS06: *Escherichia coli* Rosetta™ (DE3)pLysS hTS06 VH was deposited at VKPM on July 10, 2024, having Accession Number B-14895, and the plasmid in the *E.coli* is used to produced heavy chain of hTS06, and *Escherichia coli* Rosetta™(DE3)pLysS hTS06 VL was deposited at VKPM on July 10, 2024, having Accession Number B-14894, and the plasmid in the E.coli is used to produced light chain of hTS06;

hTS07: *Escherichia coli* Rosetta™ (DE3)pLysS hTS07 VH was deposited at VKPM on July 10, 2024, having Accession Number B-14897, and the plasmid in the *E.coli* is used to produced heavy chain of hTS07, and *Escherichia coli* Rosetta™(DE3)pLysS hTS07 VL was deposited at VKPM on July 10, 2024, having Accession Number B-14896, and the plasmid in the E.coli is used to produced light chain of hTS07;

hTS08: *Escherichia coli* Rosetta™ (DE3)pLysS hTS08 VH was deposited at VKPM on July 10, 2024, having Accession Number B-14899, and the plasmid in the *E.coli* is used to produced heavy chain of hTS08, and *Escherichia coli* Rosetta™(DE3)pLysS hTS08 VL was deposited at VKPM on July 10, 2024, having Accession Number B-14898, and the plasmid in the E.coli is used to produced light chain of hTS08;

hTS09: *Escherichia coli* Rosetta™ (DE3)pLysS hTS09 VH was deposited at VKPM on July 10, 2024, having Accession Number B-14901, and the plasmid in the *E.coli* is used to produced heavy chain of hTS09, and *Escherichia coli* Rosetta™(DE3)pLysS hTS09 VL was deposited at VKPM on July 10, 2024, having Accession Number B-14900, and the plasmid in the E.coli is used to produced light chain of hTS09;

hTS10: *Escherichia coli* Rosetta™ (DE3)pLysS hTS10 VH was deposited at VKPM on July 10, 2024, having Accession Number B-14903, and the plasmid in the *E.coli* is used to produced heavy chain of hTS10, and *Escherichia coli* Rosetta™(DE3)pLysS hTS10 VL was deposited at VKPM on July 10, 2024, having Accession Number B-14902, and the plasmid in the E.coli is used to produced light chain of hTS10;

hTS11: *Escherichia coli* Rosetta™ (DE3)pLysS hTS11 VH was deposited at VKPM on July 10, 2024, having Accession Number B-14905, and the plasmid in the *E.coli* is used to produced heavy chain of hTS11, and *Escherichia coli* Rosetta™(DE3)pLysS hTS11 VL was deposited at VKPM on July 10, 2024, having Accession Number B-14904, and the plasmid in the E.coli is used to produced light chain of hTS11;

hTS12: *Escherichia coli* Rosetta™(DE3)pLysS hTS12 VH was deposited at VKPM on July 10, 2024, having Accession Number B-14907, and the plasmid in the *E.coli* is used to produced heavy chain of hTS12, and *Escherichia coli* Rosetta™(DE3)pLysS hTS12 VL was deposited at VKPM on July 10, 2024, having Accession Number B-14906, and the plasmid in the E.coli is used to produced light chain of hTS12;

hTS13: *Escherichia coli* Rosetta™(DE3)pLysS hTS13 VH was deposited at VKPM on July 10, 2024, having Accession Number B-14909, and the plasmid in the *E.coli* is used to produced heavy chain of hTS13, and *Escherichia coli* Rosetta™(DE3)pLysS hTS13 VL was deposited at VKPM on July 10, 2024, having Accession Number B-14908, and the plasmid in the E.coli is used to produced light chain of hTS13;

hTS14: *Escherichia coli* Rosetta™(DE3)pLysS hTS14 VH was deposited at VKPM on July 10, 2024, having Accession Number B-14911, and the plasmid in the *E.coli* is used to produced heavy chain of hTS14, and *Escherichia coli* Rosetta™(DE3)pLysS hTS14 VL was deposited at VKPM on July 10, 2024, having Accession Number B-14910, and the plasmid in the E.coli is used to produced light chain of hTS14;

hTS15: *Escherichia coli* Rosetta™(DE3)pLysS hTS15 VH was deposited at VKPM on July 10, 2024, having Accession Number B-14913, and the plasmid in the *E.coli* is used to produced heavy chain of hTS15, and *Escherichia coli* Rosetta™(DE3)pLysS hTS15 VL was deposited at VKPM on July 10, 2024, having Accession Number B-14912, and the plasmid in the *E.coli* is used to produced light chain of hTS15;

hTS16: *Escherichia coli* Rosetta™(DE3)pLysS hTS16 VH was deposited at VKPM on July 10, 2024, having Accession Number B-14915, and the plasmid in the *E.coli* is used to produced heavy chain of hTS16, and *Escherichia coli* Rosetta™(DE3)pLysS hTS16 VL was deposited at VKPM on July 10, 2024, having Accession Number B-14914, and the plasmid in the *E.coli* is used to produced light chain of hTS16;

hTS17: *Escherichia coli* Rosetta™(DE3)pLysS hTS17 VH was deposited at VKPM on July 10, 2024, having Accession Number B-14917, and the plasmid in the *E.coli* is used to produced heavy chain of hTS17, and *Escherichia coli* Rosetta™(DE3)pLysS hTS17 VL was deposited at VKPM on July 10, 2024, having Accession Number B-14916, and the plasmid in the *E.coli* is used to produced light chain of hTS17.

Example 2: Detection of monoclonal antibody specificity

The specificity of the monoclonal antibody was determined by ELISA using TSH free β-subunit, free glycoprotein hormone common α-subunit, TSH dimer, FSH, LH and hCG. All proteins used for cross-reactivity studies were human, recombinant and highly purified. The specific method was as follows.

30 ng/0.05 ml of either protein in PBS was added to each well and incubated for 30 minutes. The plate was washed twice with PBST. The plate was then incubated with antibody solution (10 μg/ml) in PBST for 30 minutes and washed twice with PBST. Antibodies bound to TSH were shown by incubation with 0.05 ml of HRP-labeled anti-human or anti-rabbit antibody for 30 minutes. After incubation with secondary antibody, the plate was washed six times with PBST and 0.05 ml of o-phenylenediamine dihydrochloride (OPD) HRP substrate was added. After incubation for 10 minutes, the reaction was terminated by adding 0.025 ml of 0.5 M sulfuric acid and the absorbance in the wells was measured at 490 nm.

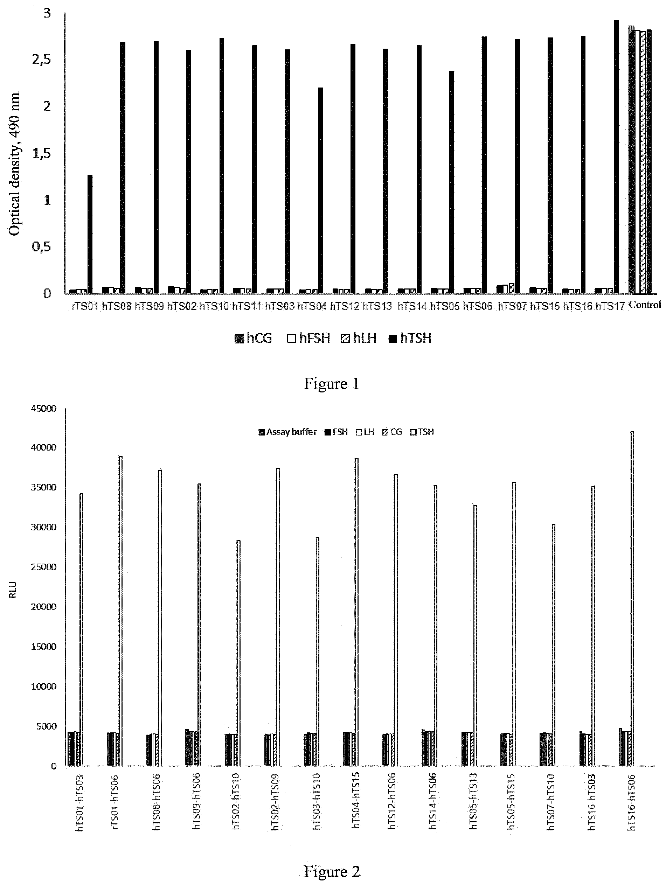

Figure 1 showed the results of the antibodies detected with glycoprotein hormones, and all antibodies did not recognize free α-subunit, FSH, LH, or hCG.

Based on the detection results of TSH dimer and free subunit, the antibodies could be divided into two groups. The first group of antibodies recognized TSH dimer and free TSH β-subunit (including rTS01, hTS08 to hTS17). We believed that the epitopes of these antibodies were located on the β-subunit. The second group of antibodies interacted only with TSH dimer and not with free α- and β-subunit (including hTS02 to hTS07). We believed that these antibodies were specific for TSH dimer. Table 4 summarized the specificity of the antibodies.

Table 4: Specificity detection results of anti-TSH antibodies

| Antibody | TSH dimer | TSH β-subunit |
|---|---|---|
| rTS01 | | + |
| hTS08 | | + |
| hTS09 | | + |
| hTS02 | + | |

(continued)

| Antibody | TSH dimer | TSH β-subunit |
|---|---|---|
| hTS10 | | + |
| hTS11 | | + |
| hTS03 | + | |
| hTS04 | + | |
| hTS12 | | + |
| hTS13 | | + |
| hTS14 | | + |
| hTS05 | + | |
| hTS06 | + | |
| hTS07 | + | |
| hTS15 | | + |
| hTS16 | | + |
| hTS17 | | + |
| Note: "+" indicates recognition of the corresponding antigen. | | |

Example 3: Establishment of sandwich immunoassay method

Using a plate-based sandwich immunoassay, rTS01 and hTS02 to hTS17 were detected in paired combinations to find a combination with the best properties. For chemiluminescent sandwich immunoassay, antibodies were labeled with alkaline phosphatase (ALP). The specific assay method was as follows.

The capture antibody in PBS at 50 ng/0.05 ml per well in a 96-well plate (Greiner) was incubated for 30 minutes. The plate was washed twice with Tris buffer containing 0.05% Tween-20 (TBST) and 10 ng/0.025 ml ALP-labeled detection antibody. At the same time, 0.025 ml of human TSH diluted to a concentration of 0.05 μIU/ml with TBST (containing 1% bovine serum albumin (assay buffer)) was added to the well. The detection buffer of the same volume was used as a blank. After incubation for 30 minutes, the plate was washed six times with TBST and added with 0.05 ml of AMPPD chemiluminescent substrate. The plate was incubated at 37°C for 10 minutes, and the signal was quantified using a plate reader capable of luminescence detection. The antibody pair that exhibited the highest signal-to-noise ratio was selected for further evaluation.

Example 4: Establishment of fully automated microparticle chemiluminescent immunoassay (sandwich method)

The antibody pairs selected in Example 3 were retested in the automated CLIA. The antibody labeled with ALP was used as the detection antibody. The capture antibody was biotinylated and bound to the streptavidin-coated magnetic beads. The magnetic beads immobilized with the capture antibody were used for the assay at a concentration of 0.25 mg/ml; the detection antibody was used for the detection at a concentration of 0.5μg/ml. Human natural TSH (WHO International Standard NIBSC code 81/565) was used as the antigen. TSH serial diluents were prepared with assay buffer (0.05, 0.016, 0.0055 μIU/ml). The assay buffer was used as the blank. 50 μL of TSH solution was mixed with 50 μL of magnetic bead suspension and 50 μL of detection antibody solution and incubated at 37°C for 16 minutes. After the reaction, the magnetic beads were washed with TBST. AMPPD chemiluminescent substrate was used to detect chemiluminescence. Finally, the 20 antibody pairs that showed the highest signal-to-noise ratio in this experiment were selected (Table 5).

Table 5: Best antibody pairs obtained by fully automated microparticle chemiluminescent immunoassay (sandwich method)

| No. | Capture antibody | Detection antibody |
|---|---|---|
| 1 | rTS01 | hTS03 |
| 2 | rTS01 | hTS06 |

(continued)

| No. | Capture antibody | Detection antibody |
|---|---|---|
| 3 | hTS08 | hTS06 |
| 4 | hTS09 | hTS06 |
| 5 | hTS02 | hTS10 |
| 6 | hTS02 | hTS09 |
| 7 | hTS03 | hTS10 |
| 8 | hTS04 | hTS15 |
| 9 | hTS12 | hTS06 |
| 10 | hTS14 | hTS06 |
| 11 | hTS05 | hTS13 |
| 12 | hTS05 | hTS15 |
| 13 | hTS07 | hTS10 |
| 14 | hTS16 | hTS03 |
| 15 | hTS16 | hTS06 |
| 16 | hTS06 | hTS09 |
| 17 | hTS07 | hTS08 |
| 18 | hTS05 | hTS09 |
| 19 | hTS04 | hTS11 |
| 20 | hTS04 | hTS08 |

Example 5: Characterization of selected antibody pairs by fully automated microparticle chemiluminescent immunoassay (sandwich method)

1. Determination of limit of detection (LoD)

We used human native TSH (WHO International Standard NIBSC code 81/565), which was diluted in assay buffer to concentrations of 0.0024, 0.0018, 0.0014, 0.001, 0.00077, and 0.00059 $\mu$IU/ml, to determine the LoD of the selected antibody pairs. In the corresponding chemiluminescent sandwich immunoassay, 20 replicates of assay buffer and TSH diluents were detected in each experiment. The limit of blank (LoB) and limit of detection (LoD) were calculated using the following formulas:

$$\text{LoB} = \text{Mean}_{\text{Blank}} + 1.645(\text{SD}_{\text{Blank}})$$

$$\text{LoD} = \text{LoB} + 1.645(\text{SD}_{\text{Low concentration sample}})$$

The LoD of TSH diluted in buffer solution was $0.00077 \mu$ IU/ml, and was the same for all antibody pairs. The coefficient of variation CV was <2% for all TSH diluents tested. This data indicated that the selected antibody pairs exhibited a sensitivity equivalent to that of the fourth generation TSH assay.

2. Cross-reactivity test with LH, FSH, CG

Recombinant human LH, FSH, and CG were diluted in assay buffer to a concentration of 10 IU/ml (LH, FSH) or 1000 IU/ml (CG), and tested in the corresponding chemiluminescent sandwich immunoassay. TSH (WHO International Standard NIBSC code 81/565) at a concentration of 0.137 $\mu$IU/ml was used as a control. For all antibody pairs, the signals detected in samples containing LH, FSH, or CG were not significantly higher than those in the blank control (Figure 2). Therefore, we confirmed that neither the individual antibodies nor the antibody pairs consisting of these antibodies cross-reacted with other human glycoprotein hormones.

3. Cross-reactivity test of TSH variant R55G

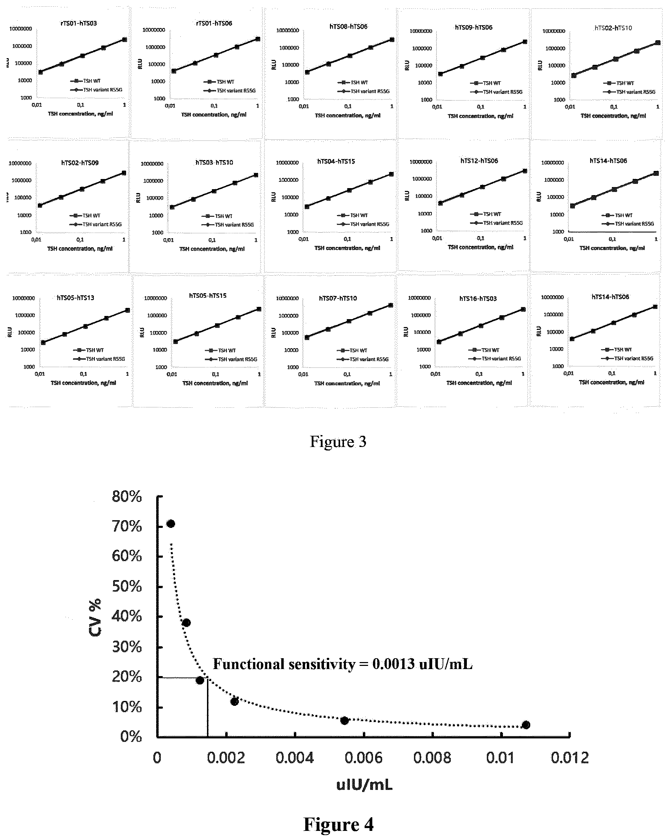

Recombinant human wild-type TSH and TSH variant R55G were serially diluted in assay buffer to concentrations of 1, 0.33, 0.11, 0.037, 0.012 ng/ml and tested in the corresponding chemiluminescent sandwich immunoassays. The selected antibody pairs recognized wild-type TSH and TSH variant R55G equally (Figure 3).

Example 6: Detection of antibody affinity

To determine the equilibrium dissociation constant, the analysis was performed using the Octet biolayer interferometry platform. Biotinylated antibodies were immobilized on a high-precision streptavidin biosensor (SAX). Human TSH was serially diluted to concentrations of 15, 10, 5, and 2.5 nmol in PBST containing 0.1% bovine serum albumin and 10 μg/ml biocytin. The reference biosensor without immobilized antibodies was incubated with TSH at the highest concentration (15 nmol). The analysis temperature was 30°C. The experimental protocol was as follows:

1. hydrating sensor with PBS

2. binding the sensor with antibody (4 μg/ml in PBS, 10 min)

3. washing the sensor (with PBS)

4. blocking the sensor (with PBST containing 0.1% BSA and 10 μg/ml biocytin)

5. binding (5 min for each tested antibody concentration)

6. dissociating (with PBS, 25 min)

The binding constant (Ka), equilibrium dissociation constant (KD), and dissociation constant (Kd) were calculated using Octet Analysis Studio software (the results were shown in Table 6). Data were analyzed using Global fitting and a 1:1 binding model.

Table 6: Binding constants of antibodies of the present application

| No. | Antibody | ka (1/Ms) | kdis (1/s) | KD (M) |
|-----|----------|-----------|-----------|--------|
| 1 | rTS01 | 2.32E+05 | 7.29E-05 | 3.14E-10 |
| 2 | hTS08 | 3.73E+05 | 3.09E-05 | 8.27E-11 |
| 3 | hTS09 | 3.18E+05 | 4.14E-05 | 1.31E-10 |
| 4 | hTS02 | 3.31E+05 | 1.31E-04 | 3.96E-10 |
| 5 | hTS10 | 3.25E+05 | 1.75E-05 | 5.39E-11 |
| 6 | hTS11 | 3.10E+05 | 2.98E-05 | 9.58E-11 |
| 7 | hTS03 | 3.38E+05 | 7.10E-05 | 2.10E-10 |
| 8 | hTS04 | 2.36E+05 | 1.08E-05 | 4.58E-11 |
| 9 | hTS12 | 3.21E+05 | 4.46E-05 | 1.39E-10 |
| 10 | hTS13 | 2.89E+05 | 1.83E-05 | 6.31E-11 |
| 11 | hTS14 | 3.32E+05 | 4.35E-05 | 1.31E-10 |
| 12 | hTS05 | 3.39E+05 | 8.20E-05 | 2.42E-10 |
| 13 | hTS06 | 2.57E+05 | 6.32E-05 | 2.46E-10 |
| 14 | hTS07 | 2.57E+05 | 1.05E-05 | 4.08E-11 |
| 15 | hTS15 | 3.45E+05 | 6.01E-05 | 1.74E-10 |
| 16 | hTS16 | 3.26E+05 | 3.36E-05 | 1.03E-10 |
| 17 | hTS17 | 2.60E+05 | 4.91E-05 | 1.89E-10 |

Example 7: Preparation and detection of TSH kit and calibrator

Step 1: Preparation of R1 reagent (containing capture antibody)

A specific TSH antibody and superparamagnetic particles were mixed in TBS buffer. After sufficient reaction, the mixture was placed on a magnetic separator until the supernatant was not turbid. The supernatant was discarded, and the superparamagnetic particles coated with TSH antibody was retained. The above operations were repeated with TBS buffer for 3 times. The superparamagnetic particles coated with TSH antibody were suspended with R1 diluent to prepare the R1 reagent, in which the content of superparamagnetic particles was 0.07% and the content of TSH antibody was 0.0007%. The R1 reagent was refrigerated at 2 to 8 °C for later use.

Step 2: Preparation of R2 reagent (containing detection antibody)

Another specific TSH antibody was conjugated with alkaline phosphatase, in which the molar ratio of alkaline phosphatase to the antibody was 15:1, then the conjugate was dissolved with R2 diluent to prepare the R2 reagent, in which the concentration of the conjugate was 0.5 $\mu$g/mL. The R2 reagent was refrigerated at 2 to 8 °C for later use. (R1 reagent and R2 reagent were combined to form a TSH kit).

Step 3: Preparation of TSH calibrators

TSH antigen was diluted to 0 $\mu$IU/mL, 0.05 $\mu$IU/mL, 0.5 $\mu$IU/mL, 1 $\mu$IU/mL, 2 $\mu$IU/mL, 4 $\mu$IU/mL, 10 $\mu$IU/mL, 20 $\mu$IU/mL, 40 $\mu$IU/mL, 60 $\mu$IU/mL, 80 $\mu$IU/mL, 100 $\mu$IU/mL, 120 $\mu$IU/mL, and other concentration points to prepare TSH calibrators.

Step 4: Measurement procedure of TSH kit

The TSH kit (containing R1 reagent and R2 reagent) was loaded into a Mindray CL series fully automatic chemiluminescence immunoassay analyzer, and measurement was carried out according to the operating steps in the instrument manual.

The matching TSH calibrators were used, and the calibration test was carried out according to the operating steps in the instrument manual. Through the calibration data, the system software used a weighted four-parameter logarithmic curve (4PLC) mathematical fitting model to fit the luminescence signals and concentrations. The final results were given in the form of $\mu$IU/mL concentration.

According to the above method, TSH kits 1 to 5 (the antibody and antigen compositions were shown in Table 7 below) were obtained and their performance was analyzed.

Table 7: TSH kits

| TSH kit | Capture antibody | Detection antibody |
|---------|------------------|--------------------|
| Kit 1 | hTS06 | hTS09 |
| Kit 2 | hTS07 | hTS08 |
| Kit 3 | hTS05 | hTS09 |
| Kit 4 | hTS04 | hTS11 |
| Kit 5 | hTS04 | hTS08 |

1. Test results of functional sensitivity:

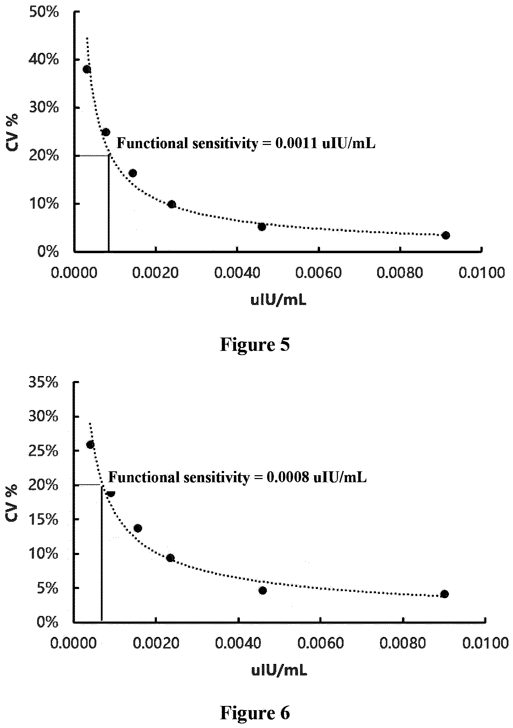

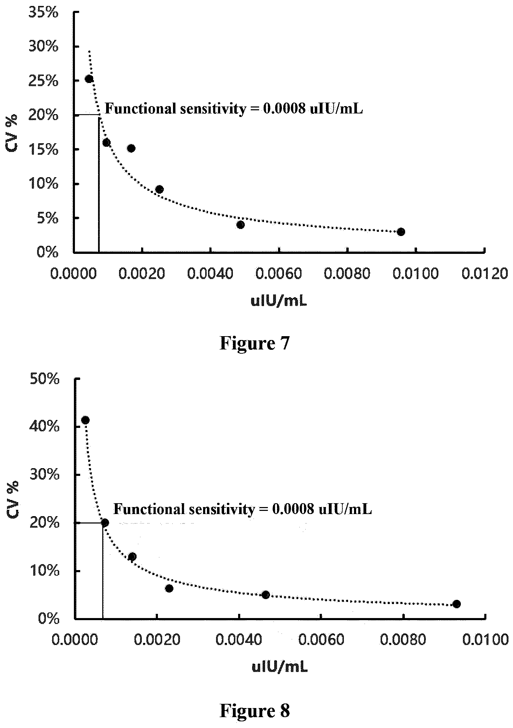

A series of low-concentration samples were prepared by gradient dilution, and these samples were tested multiple times for 10 consecutive days. The test was repeated twice a day to obtain 20 measurement results. The average value of each concentration was calculated, and the daily CV value of each concentration was calculated at the same time. A dot plot of the average value of each concentration and its daily CV was plotted. The system software used a weighted five-parameter logarithmic curve (5PLC) mathematical fitting model to fit the daily coefficient of variation CV% and the sample concentration value. The concentration value corresponding to the 20% CV value was calculated as the functional sensitivity of TSH. The results were shown in Figures 4 to 8.

The functional sensitivity of the current TSH detection kits could reach <0.0015 $\mu$IU/mL.

2. Test results of TSH kit analog cross-reaction:

TSH analog samples with target concentrations were prepared by adding three analog antigens to the TSH zero value calibrator, and the constructed TSH kits were used for detection, and the cross-reaction rates were calculated. The results were shown in Table 8.

The cross-reaction rates of the current TSH detection kits and the three analogs FSH, LH, and hCG were all <0.001%.

Table 8: TSH detection sensitivity and cross-reaction test results of the kits of the present application

| Analog | Concentration | Measured value of TSH Cross-reaction rate | Kit 1 | Kit 2 | Kit 3 | Kit 4 | Kit 5 |
|---|---|---|---|---|---|---|---|
| FSH | 10,000 mIU/mL | Measured value of TSH, $\mu$IU/mL | 0.109 | 0.107 | 0.105 | 0.104 | 0.111 |
| | | Cross-reaction rate, % | 0.000% | 0.000% | 0.000% | 0.000% | 0.000% |
| LH | 10,000 mIU/mL | Measured value of TSH, $\mu$IU/mL | 1.622 | 1.63 | 1.587 | 1.628 | 1.667 |
| | | Cross-reaction rate, % | 0.000% | 0.000% | 0.000% | 0.000% | 0.000% |
| hCG | 1,000,000 mIU/mL | Measured value of TSH, $\mu$IU/mL | 0.228 | 0.241 | 0.235 | 0.242 | 0.237 |
| | | Cross-reaction rate, % | 0.000% | 0.000% | 0.000% | 0.000% | 0.000% |

3. Methodological comparison data of TSH kits

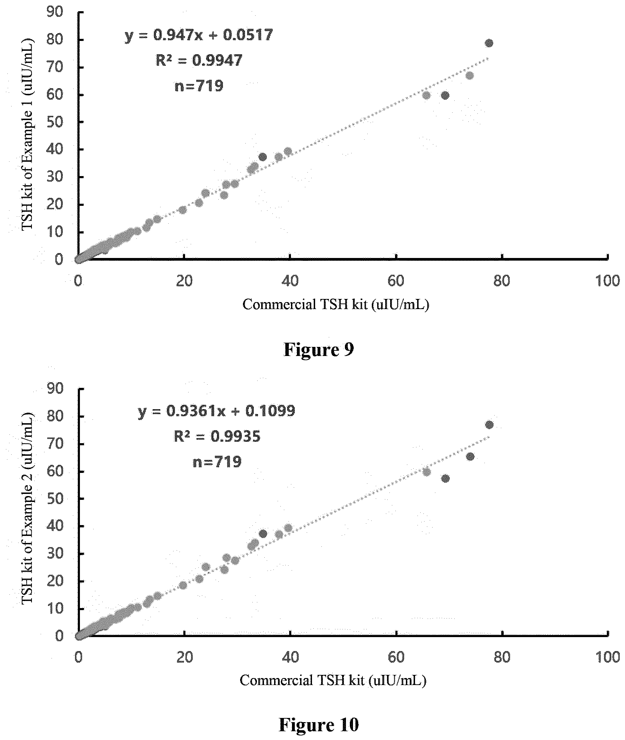

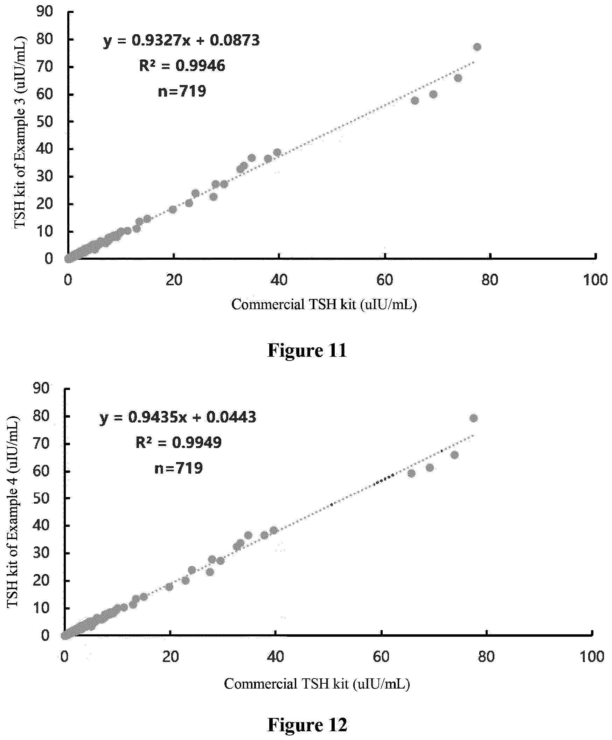

As shown in Figures 9 to 13, the TSH kits of the present application and the commercial TSH kit (thyroid stimulating hormone kit (electrochemiluminescence method), Roche, (240) 08429324190) had good methodological consistency, and the correlation coefficient $R^2$ was greater than 0.95, indicating that they had good clinical applicability.

Example 8: TSH kit being able to simultaneously detect wild-type TSH and mutant R55G TSH antigens

The samples to be tested were prepared with wild-type TSH recombinant antigen and mutant R55G TSH antigen at three different concentrations, respectively, and the Kits 1 to 3 in the above Example 7 were used to test these two types of samples on a Mindray chemiluminescent immunoassay analyzer CL8000i. At the same time, a commercial TSH detection kit was used on the corresponding instrument. The results were shown in the table below. It is shown that the kits of the present application can detect both wild-type TSH and mutant R55G TSH antigens, whereas the commercial kit could not detect mutant R55G TSH antigens.

| | Kit 1 | Kit 2 | Kit 3 | Commercial TSH |
|---|---|---|---|---|
| Wild-type TSH-Concentration 1 uIU/mL | 79.0788 | 79.1531 | 77.5309 | 78.166 |
| Wild-type TSH-Concentration 2 uIU/mL | 7.8243 | 7.7749 | 7.5948 | 7.472 |
| Wild-type TSH-Concentration 3 uIU/mL | 0.8726 | 0.8570 | 0.8405 | 0.844 |
| R55G TSH-Concentration 1 uIU/mL | 78.8602 | 77.9374 | 76.6014 | 0.026 |
| R55G TSH-Concentration 2 uIU/mL | 7.5229 | 7.5581 | 7.4789 | 0.012 |
| R55G TSH-Concentration 3 uIU/mL | 0.8460 | 0.8431 | 0.8400 | 0.013 |

The information of the amino acid sequences involved in the present application is described in the table below:

| SEQ ID NO: | Description | Sequence |
|---|---|---|
| 1 | Amino acid sequence of CDR-H1 of TS01/rTS01 | TYYYYMC |
| 2 | Amino acid sequence of CDR-H2 of TS01/rTS01 | CIEAGGSGTTYYANWAKG |

(continued)

| SEQ ID NO: | Description | Sequence |
|---|---|---|
| 3 | Amino acid sequence of CDR-H3 of TS01/rTS01 | FLGYASDGGAYINAFNL |
| 4 | Amino acid sequence of CDR-L1 of TS01/rTS01 | QASQNIGSNLA |
| 5 | Amino acid sequence of CDR-L2 of TS01/rTS01 | RASTLES |
| 6 | Amino acid sequence of CDR-L3 of TS01/rTS01 | QCTVYGSSDVFA |
| 7 | Amino acid sequence of CDR-H1 of TS02/rTS02 | SNAVG |
| 8 | Amino acid sequence of CDR-H2 of TS02/rTS02 | GMSSGGSTYYNPALKS |
| 9 | Amino acid sequence of CDR-H3 of TS02/rTS02 | VDATRPDDIGWIDY |
| 10 | Amino acid sequence of CDR-L1 of TS02/rTS02 | SGSDIGSSAVG |
| 11 | Amino acid sequence of CDR-L2 of TS02/rTS02 | GTVRRPS |
| 12 | Amino acid sequence of CDR-L3 of TS02/rTS02 | GGAAGSNYGD |
| 13 | Amino acid sequence of CDR-H1 of TS03/rTS03 | SNAVG |
| 14 | Amino acid sequence of CDR-H2 of TS03/rTS03 | GISSGGTAYYNPALKS |
| 15 | Amino acid sequence of CDR-H3 of TS03/rTS03 | VDATRPDDIGWIDY |
| 16 | Amino acid sequence of CDR-L1 of TS03/rTS03 | SGSDFGSSAVG |
| 17 | Amino acid sequence of CDR-L2 of TS03/rTS03 | GTTRRPS |
| 18 | Amino acid sequence of CDR-L3 of TS03/rTS03 | GGAAGSDHGD |
| 19 | Amino acid sequence of CDR-H1 of TS04/rTS04 | NYDVG |
| 20 | Amino acid sequence of CDR-H2 of TS04/rTS04 | DIYSGGGKSYNPALKS |
| 21 | Amino acid sequence of CDR-H3 of TS04/rTS04 | GVYSGNGIADAADV |
| 22 | Amino acid sequence of CDR-L1 of TS04/rTS04 | SGSYIDSRHVG |
| 23 | Amino acid sequence of CDR-L2 of TS04/rTS04 | YSTNRP |
| 24 | Amino acid sequence of CDR-L3 of TS04/rTS04 | GSYAASIDEGI |
| 25 | Amino acid sequence of CDR-H1 of TS05/rTS05 | MYSIT |
| 26 | Amino acid sequence of CDR-H2 of TS05/rTS05 | DIWGDGSTLYNPALKS |
| 27 | Amino acid sequence of CDR-H3 of TS05/rTS05 | GVGTTVTEVDD |
| 28 | Amino acid sequence of CDR-L1 of TS05/rTS05 | QGDLLDDQYTA |
| 29 | Amino acid sequence of CDR-L2 of TS05/rTS05 | KDTERPS |
| 30 | Amino acid sequence of CDR-L3 of TS05/rTS05 | LSLDSSRMGV |
| 31 | Amino acid sequence of CDR-H1 of TS06/rTS06 | SNAVG |
| 32 | Amino acid sequence of CDR-H2 of TS06/rTS06 | GISSGGSAYYNPALKS |
| 33 | Amino acid sequence of CDR-H3 of TS06/rTS06 | VDATRPDDIGWVDF |
| 34 | Amino acid sequence of CDR-L1 of TS06/rTS06 | SGSDFFSNAVG |
| 35 | Amino acid sequence of CDR-L2 of TS06/rTS06 | GTTRRPS |
| 36 | Amino acid sequence of CDR-L3 of TS06/rTS06 | GSTARSNYGD |
| 37 | Amino acid sequence of CDR-H1 of TS07/rTS07 | SNAVG |
| 38 | Amino acid sequence of CDR-H2 of TS07/rTS07 | GISSGGTTYYNPALKS |
| 39 | Amino acid sequence of CDR-H3 of TS07/rTS07 | VDATRPDDIGWIDY |
| 40 | Amino acid sequence of CDR-L1 of TS07/rTS07 | SGSDFGSSAVG |
| 41 | Amino acid sequence of CDR-L2 of TS07/rTS07 | GTTRRPS |

(continued)

| SEQ ID NO: | Description | Sequence |
|---|---|---|
| 42 | Amino acid sequence of CDR-L3 of TS07/rTS07 | GGAAGTNHGD |
| 43 | Amino acid sequence of CDR-H1 of TS08/rTS08 | SYSVN |
| 44 | Amino acid sequence of CDR-H2 of TS08/rTS08 | DISTYGNTVYNPTLES |
| 45 | Amino acid sequence of CDR-H3 of TS08/rTS08 | LHLTDSSHTRNGVDV |
| 46 | Amino acid sequence of CDR-L1 of TS08/rTS08 | QGDIGSSYVA |
| 47 | Amino acid sequence of CDR-L2 of TS08/rTS08 | QNSKRPS |
| 48 | Amino acid sequence of CDR-L3 of TS08/rTS08 | LSADSRFV |
| 49 | Amino acid sequence of CDR-H1 of TS09/rTS09 | SYNVV |
| 50 | Amino acid sequence of CDR-H2 of TS09/rTS09 | EIEPGGSTFSNPALKS |
| 51 | Amino acid sequence of CDR-H3 of TS09/rTS09 | ASYTDALR |
| 52 | Amino acid sequence of CDR-L1 of TS09/rTS09 | SGSSSNIGGGYYVG |
| 53 | Amino acid sequence of CDR-L2 of TS09/rTS09 | QNSKRPT |
| 54 | Amino acid sequence of CDR-L3 of TS09/rTS09 | STYDSSISASV |
| 55 | Amino acid sequence of CDR-H1 of TS10/rTS10 | SYSVN |
| 56 | Amino acid sequence of CDR-H2 of TS10/rTS10 | DISTYGNTVYNPALES |
| 57 | Amino acid sequence of CDR-H3 of TS10/rTS10 | LRLSEGGHTRNGVDV |
| 58 | Amino acid sequence of CDR-L1 of TS10/rTS10 | QGDDIGRSYVA |
| 59 | Amino acid sequence of CDR-L2 of TS10/rTS10 | QNSKRPS |
| 60 | Amino acid sequence of CDR-L3 of TS10/rTS10 | LSAGSRFV |
| 61 | Amino acid sequence of CDR-H1 of TS11/rTS11 | SNAVH |
| 62 | Amino acid sequence of CDR-H2 of TS11/rTS11 | VIVSTGSTAYNPALKS |
| 63 | Amino acid sequence of CDR-H3 of TS11/rTS11 | TIGGDDWTSDVEHIDY |
| 64 | Amino acid sequence of CDR-L1 of TS11/rTS11 | SGSSRNVGEYGVG |
| 65 | Amino acid sequence of CDR-L2 of TS11/rTS11 | GTSSRPS |
| 66 | Amino acid sequence of CDR-L3 of TS11/rTS11 | ATTDSSRRNVV |
| 67 | Amino acid sequence of CDR-H1 of TS12/rTS12 | SDAVT |
| 68 | Amino acid sequence of CDR-H2 of TS12/rTS12 | IIYSGGSTSYNQTLKS |
| 69 | Amino acid sequence of CDR-H3 of TS12/rTS12 | SNFGYDSDLDY |
| 70 | Amino acid sequence of CDR-L1 of TS12/rTS12 | SGSSSNVGYGNYVG |
| 71 | Amino acid sequence of CDR-L2 of TS12/rTS12 | GATNRAS |
| 72 | Amino acid sequence of CDR-L3 of TS12/rTS12 | ASYDSSSRIV |
| 73 | Amino acid sequence of CDR-H1 of TS13/rTS13 | TKAVH |
| 74 | Amino acid sequence of CDR-H2 of TS13/rTS13 | VIASSGSTAYNPALKS |
| 75 | Amino acid sequence of CDR-H3 of TS13/rTS13 | TIGGDDWRSDVEHIDY |
| 76 | Amino acid sequence of CDR-L1 of TS13/rTS13 | SGSSRNVGEYGVA |
| 77 | Amino acid sequence of CDR-L2 of TS13/rTS13 | GTSNRPS |
| 78 | Amino acid sequence of CDR-L3 of TS13/rTS13 | AATDSSRRDVV |
| 79 | Amino acid sequence of CDR-H1 of TS14/rTS14 | SYNLV |
| 80 | Amino acid sequence of CDR-H2 of TS14/rTS14 | EIEPGGSTFYNPALKS |

(continued)

| SEQ ID NO: | Description | Sequence |
|---|---|---|
| 81 | Amino acid sequence of CDR-H3 of TS14/rTS14 | ASHTDFLR |
| 82 | Amino acid sequence of CDR-L1 of TS14/rTS14 | SGSSSNIGGGYYVG |
| 83 | Amino acid sequence of CDR-L2 of TS14/rTS14 | QNSKRPT |
| 84 | Amino acid sequence of CDR-L3 of TS14/rTS14 | STYDSSISTTV |
| 85 | Amino acid sequence of CDR-H1 of TS15/rTS15 | SNAVH |
| 86 | Amino acid sequence of CDR-H2 of TS15/rTS15 | GIVSSGSTAYNPALKS |
| 87 | Amino acid sequence of CDR-H3 of TS15/rTS15 | TIGGDDWTSNVEHIDY |
| 88 | Amino acid sequence of CDR-L1 of TS15/rTS15 | SGSSRNVGEYGVG |
| 89 | Amino acid sequence of CDR-L2 of TS15/rTS15 | GTRSRPS |
| 90 | Amino acid sequence of CDR-L3 of TS15/rTS15 | ATTDSSRRNVV |
| 91 | Amino acid sequence of CDR-H1 of TS16/rTS16 | TYAVT |
| 92 | Amino acid sequence of CDR-H2 of TS16/rTS16 | IIYSGGSTSYNQTLKS |
| 93 | Amino acid sequence of CDR-H3 of TS16/rTS16 | SNFGYDSDLDY |
| 94 | Amino acid sequence of CDR-L1 of TS16/rTS16 | SGSSSNVGYGNYVG |
| 95 | Amino acid sequence of CDR-L2 of TS16/rTS16 | GATNRAS |
| 96 | Amino acid sequence of CDR-L3 of TS16/rTS16 | ASYDINSRII |
| 97 | Amino acid sequence of CDR-H1 of TS17/rTS17 | NDGVG |
| 98 | Amino acid sequence of CDR-H2 of TS17/rTS17 | AIYSSGGTYYNPALKS |
| 99 | Amino acid sequence of CDR-H3 of TS17/rTS17 | HFYRDWASGSDMVSFDY |
| 100 | Amino acid sequence of CDR-L1 of TS17/rTS17 | SGSSGNVGFGDYVA |
| 101 | Amino acid sequence of CDR-L2 of TS17/rTS17 | RATSRAS |
| 102 | Amino acid sequence of CDR-L3 of TS17/rTS17 | ASFDSSASGI |
| 103 | TSH α-subunit | APDVQDCPECTLQENPFFSQPG APILQCMGCCFSRAYPTPLRSKK TMLVQKNVTSESTCCVAKSYNR VTVMGGFKVENHTACHCSTCY YHKS |
| 104 | TSH α-subunit containing R55G mutation | FCIPTEYTMHIERRECAYCLTINT TICAGYCMTRDINGKLFLPKYA LSQDVCTYGDFIYRTVEIPGCPL HVAPYFSYPVALSCKCGKCNTD YSDCIHEAIKTNYCTKPQKSYLV GFSV |
| 105 | TS01/rTS01 CDR-H1 nucleotide sequence | acctactactactacatgtgc |
| 106 | TS01/rTS01 CDR-H2 nucleotide sequence | tgtattgaggctggtggtagtggtaccacttactac gcgaactgggcgaaaggc |
| 107 | TS01/rTS01 CDR-H3 nucleotide sequence | tttttggggttacgctagtgatggtggtgcttatattaa cgccttcaatttg |
| 108 | TS02/hTS02 CDR-H1 nucleotide sequence | agcaatgctgtaggc |

(continued)

| SEQ ID NO: | Description | Sequence |
|---|---|---|
| 109 | TS02/hTS02 CDR-H2 nucleotide sequence | ggcatgagtagtggtggaagtacatactataaccc ggccctgaaatcc |
| 110 | TS02/hTS02 CDR-H3 nucleotide sequence | gttgatgcgactcgtcctgatgatattggttggatc gactac |
| 111 | TS03/hTS03 CDR-H1 nucleotide sequence | agcaatgctgtcggc |
| 112 | TS03/hTS03 CDR-H2 nucleotide sequence | ggcataagtagtggtggcacagcatactataatcc ggccctgaaatcc |
| 113 | TS03/hTS03 CDR-H3 nucleotide sequence | gttgacgcgactcgtcctgatgatattggttggatc gactat |
| 114 | TS04/hTS04 CDR-H1 nucleotide sequence | aattatgatgtaggc |
| 115 | TS04/hTS04 CDR-H2 nucleotide sequence | gacatctatagtggtggaggtaaaagctataaccc ggccctgaaatcc |
| 116 | TS04/hTS04 CDR-H3 nucleotide sequence | ggggtctatagtggtaatggtattgctgatgctgct gatgtc |
| 117 | TS05/hTS05 CDR-H1 nucleotide sequence | atgtatagtataacc |
| 118 | TS05/hTS05 CDR-H2 nucleotide sequence | gatatatgggggtgatggaagtacgctctataaccc ggccctcaaatcc |
| 119 | TS05/hTS05 CDR-H3 nucleotide sequence | ggtgttgggacgactgtgacagaagtcgacgac |
| 120 | TS06/hTS06 CDR-H1 nucleotide sequence | agcaatgctgtaggc |
| 121 | TS06/hTS06 CDR-H2 nucleotide sequence | ggcataagtagtggtggaagtgcatactataaccc ggccctgaaatcc |
| 122 | TS06/hTS06 CDR-H3 nucleotide sequence | gttgatgcgactcgtcctgatgatattggttgggtc gacttc |
| 123 | TS07/hTS07 CDR-H1 nucleotide sequence | agcaatgctgtcggc |
| 124 | TS07/hTS07 CDR-H2 nucleotide sequence | ggcataagtagtggtggcactacatactataatcc ggccctgaaatcc |
| 125 | TS07/hTS07 CDR-H3 nucleotide sequence | gttgatgcgactcgtcctgatgatattggttggatc gactat |
| 126 | TS08/hTS08 CDR-H1 nucleotide sequence | agctatagtgtaaac |
| 127 | TS08/hTS08 CDR-H2 nucleotide sequence | gatataagtacttatggaaacacagtctataacccg accctggaatcc |
| 128 | TS08/hTS08 CDR-H3 nucleotide sequence | cttcacttgactgatagtagtcatactcgaaacggt gtggatgtc |
| 129 | TS09/hTS09 CDR-H1 nucleotide sequence | agctataatgtagtc |
| 130 | TS09/hTS09 CDR-H2 nucleotide sequence | gaaatagaacctggtggaagtacattcagtaaccc ggccctgaaatcc |

(continued)

| SEQ ID NO: | Description | Sequence |
|---|---|---|
| 131 | TS09/hTS09 CDR-H3 nucleotide sequence | gcttcgtataccgatgcactgcga |
| 132 | TS10/hTS10 CDR-H1 nucleotide sequence | agctatagtgtaaac |
| 133 | TS10/hTS10 CDR-H2 nucleotide sequence | gatataagcacttatggaaacacagtctataaccc ggccctggaatcc |
| 134 | TS10/hTS10 CDR-H3 nucleotide sequence | cttcgcttgagtgaaggtggtcacactcgaaacgg tgtagatgtc |
| 135 | TS11/hTS11 CDR-H1 nucleotide sequence | agcaatgctgtacac |
| 136 | TS11/hTS11 CDR-H2 nucleotide sequence | gttatagttagtactgggagcacagcctataaccc ggccctgaaatcc |
| 137 | TS11/hTS11 CDR-H3 nucleotide sequence | acaataggtggtgatgactggactagtgatgtaga acatatcgactac |
| 138 | TS12/hTS12 CDR-H1 nucleotide sequence | agcgatgctgtaact |
| 139 | TS12/hTS12 CDR-H2 nucleotide sequence | atcatatatagtggtggaagtacatcttataatcaga ccctgaaatcc |
| 140 | TS12/hTS12 CDR-H3 nucleotide sequence | agtaattttggttatgattccgatctcgactac |
| 141 | TS13/hTS13 CDR-H1 nucleotide sequence | accaaggcggtacac |
| 142 | TS13/hTS13 CDR-H2 nucleotide sequence | gttattgctagtagtgggagcacagcctataaccc ggccctgaaatcc |
| 143 | TS13/hTS13 CDR-H3 nucleotide sequence | acaataggtggtgatgactggcgtagtgatgtgga gcatatcgactac |
| 144 | TS14/hTS14 CDR-H1 nucleotide sequence | agctataatttagtc |
| 145 | TS14/hTS14 CDR-H2 nucleotide sequence | gaaatagaacctggtggaagtacattctataaccc ggccctgaaatcc |
| 146 | TS14/hTS14 CDR-H3 nucleotide sequence | gcttcgcataccgatttcctgcga |
| 147 | TS15/hTS15 CDR-H1 nucleotide sequence | agcaatgctgtacac |
| 148 | TS15/hTS15 CDR-H2 nucleotide sequence | ggtatagttagtagtggaagcacagcctataaccc ggccctgaaatcc |
| 149 | TS15/hTS15 CDR-H3 nucleotide sequence | acaataggtggtgatgactggactagtaatgtaga gcatatcgactac |
| 150 | TS16/hTS16 CDR-H1 nucleotide sequence | acctatgctgtaact |
| 151 | TS16/hTS16 CDR-H2 nucleotide sequence | atcatatatagtggtggaagtacatcttataaccag accctgaaatcc |
| 152 | TS16/hTS16 CDR-H3 nucleotide sequence | agtaattttggttatgattccgatctcgactac |
| 153 | TS17/hTS17 CDR-H1 nucleotide sequence | aacgatggtgtaggc |
| 154 | TS17/hTS17 CDR-H2 nucleotide sequence | gcgatttacagtagtggaggtacatactataatccg gccctgaaatcc |

(continued)

| SEQ ID NO: | Description | Sequence |
|---|---|---|
| 155 | TS17/hTS17 CDR-H3 nucleotide sequence | cacttttaccgtgattgggctagtgggtctgatatg gtttctttcgactac |
| 156 | TS01/rTS01 CDR-L1 nucleotide sequence | caggccagtcagaacattggtagtaatttagcc |
| 157 | TS01/rTS01 CDR-L2 nucleotide sequence | agggcatccactctggaatct |
| 158 | TS01/rTS01 CDR-L3 nucleotide sequence | caatgtactgtttatggtagtagtgatgtttttgct |
| 159 | TS02/hTS02 CDR-L1 nucleotide sequence | tctggaagcgacatcggtagtagtgcagtaggc |
| 160 | TS02/hTS02 CDR-L2 nucleotide sequence | ggtactgtccgtcgaccctca |
| 161 | TS02/hTS02 CDR-L3 nucleotide sequence | ggaggtgctgctggtagtaactatggtgat |
| 162 | TS03/hTS03 CDR-L1 nucleotide sequence | tctggaagcgacttcggtagtagtgcagtaggc |
| 163 | TS03/hTS03 CDR-L2 nucleotide sequence | ggtactaccaggcgaccctca |
| 164 | TS03/hTS03 CDR-L3 nucleotide sequence | ggaggtgctgctggttctgaccatggtgat |
| 165 | TS04/hTS04 CDR-L1 nucleotide sequence | tctggaagctacatcgatagtaggcatgtaggc |
| 166 | TS04/hTS04 CDR-L2 nucleotide sequence | tatagtaccaatcgaccctca |
| 167 | TS04/hTS04 CDR-L3 nucleotide sequence | ggaagttatgctgcgagtatcgatgagggtatt |
| 168 | TS05/hTS05 CDR-L1 nucleotide sequence | cagggagacctgctggacgatcaatatacagct |
| 169 | TS05/hTS05 CDR-L2 nucleotide sequence | aaagacactgagcggccttca |
| 170 | TS05/hTS05 CDR-L3 nucleotide sequence | ctgtcacttgacagcagcagaatgggtgtt |
| 171 | TS06/hTS06 CDR-L1 nucleotide sequence | tctggaagcgacttctttagtaatgcagtaggc |
| 172 | TS06/hTS06 CDR-L2 nucleotide sequence | ggtactacccgtcgaccctca |
| 173 | TS06/hTS06 CDR-L3 nucleotide sequence | ggaagtactgcccgtagtaactatggtgat |
| 174 | TS07/hTS07 CDR-L1 nucleotide sequence | tctggaagcgacttcggtagtagtgcagtaggc |
| 175 | TS07/hTS07 CDR-L2 nucleotide sequence | ggtactactaggcgaccctca |
| 176 | TS07/hTS07 CDR-L3 nucleotide sequence | ggaggtgctgctggtactaaccatggtgat |
| 177 | TS08/hTS08 CDR-L1 nucleotide sequence | cagggagacataggaagctcttatgttgcg |
| 178 | TS08/hTS08 CDR-L2 nucleotide sequence | caaaatagtaagaggccctcg |
| 179 | TS08/hTS08 CDR-L3 nucleotide sequence | ctgtcagctgacagccgttttgtt |
| 180 | TS09/hTS09 CDR-L1 nucleotide sequence | tctggaagcagcagcaacatcggggggtggttatt atgtgggc |
| 181 | TS09/hTS09 CDR-L2 nucleotide sequence | caaaacagcaaacgaccgaca |
| 182 | TS09/hTS09 CDR-L3 nucleotide sequence | tcaacttatgacagcagtattagtgctagtgtt |
| 183 | TS10/hTS10 CDR-L1 nucleotide sequence | cagggagacgacataggaaggtcttatgttgcg |
| 184 | TS10/hTS10 CDR-L2 nucleotide sequence | caaaatagtaagaggccctcg |
| 185 | TS10/hTS10 CDR-L3 nucleotide sequence | ctgtcagctggcagcaggtttgtt |
| 186 | TS11/hTS11 CDR-L1 nucleotide sequence | tctggaagcagcaggaacgttggtgaatatggtgt aggc |
| 187 | TS 11/hTS11 CDR-L2 nucleotide sequence | ggtactagtagtcgaccctcg |
| 188 | TS11/hTS11 CDR-L3 nucleotide sequence | gcaactactgacagcagtagaaggaatgttgtt |

(continued)

| SEQ ID NO: | Description | Sequence |
|---|---|---|
| 189 | TS12/hTS12 CDR-L1 nucleotide sequence | tctggaagcagcagcaacgttggatatggtaattatgtgggc |
| 190 | TS12/hTS12 CDR-L2 nucleotide sequence | ggtgcaaccaatcgagcctcg |
| 191 | TS12/hTS12 CDR-L3 nucleotide sequence | gcatcttatgacagcagtagtagaattgtt |
| 192 | TS13/hTS13 CDR-L1 nucleotide sequence | tctggaagcagcaggaacgttggtgaatatggtgtagct |
| 193 | TS13/hTS13 CDR-L2 nucleotide sequence | ggtactagcaatcgaccctcg |
| 194 | TS13/hTS13 CDR-L3 nucleotide sequence | gcagctactgatagcagtaggcgggatgttgtt |
| 195 | TS14/hTS14 CDR-L1 nucleotide sequence | tctggaagcagcagcaacatcggggggtggttattatgtgggc |
| 196 | TS14/hTS14 CDR-L2 nucleotide sequence | caaaacagcaaacgaccgaca |
| 197 | TS14/hTS14 CDR-L3 nucleotide sequence | tcaacttatgacagcagtattagtactactgtt |
| 198 | TS15/hTS15 CDR-L1 nucleotide sequence | tctggaagcagcaggaacgttggtgaatatggtgtaggc |
| 199 | TS15/hTS15 CDR-L2 nucleotide sequence | ggtactcgtagtcgaccctcg |
| 200 | TS15/hTS15 CDR-L3 nucleotide sequence | gcaactactgacagcagtagaaggaatgttgtt |
| 201 | TS16/hTS16 CDR-L1 nucleotide sequence | tcgggaagcagcagcaacgttggatatggtaattatgtgggc |
| 202 | TS16/hTS16 CDR-L2 nucleotide sequence | ggtgcaaccaatcgagcctcg |
| 203 | TS16/hTS16 CDR-L3 nucleotide sequence | gcatcttatgacatcaacagtaggattatt |
| 204 | TS17/hTS17 CDR-L1 nucleotide sequence | tctggaagcagcggcaacgttggatttggtgattatgtggcc |
| 205 | TS17/hTS17 CDR-L2 nucleotide sequence | cgtgcaacgagtcgagcctcg |
| 206 | TS17/hTS17 CDR-L3 nucleotide sequence | gcctcttttgacagcagtgccagtggtatt |

Although the specific embodiments of the present application have been described in detail, those skilled in the art will understand that various modifications and substitutions can be made to those details based on all the teachings disclosed, and these changes are within the scope of protection of the present application. The full scope of the present application is given by the attached claims and any equivalents thereof.

We would like to claim:

1. An antibody or antigen-binding fragment thereof that specifically binds to TSH (thyroid stimulating hormone) or a mutant thereof, comprising:

a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or,

a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof;

wherein each of the variants comprises an amino acid mutation as compared to the amino acid sequence from which it is derived, and the amino acid mutation is a substitution, deletion or addition of one or several amino acids (e.g., a substitution, deletion or addition of 1, 2, 3, 4 or 5 amino acids); preferably, the substitution is a conservative substitution;

preferably, each of the variants has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which it is derived.

2. The antibody or antigen-binding fragment thereof according to claim 1, comprising:

(1a) a heavy chain variable region comprising the following three CDRs: CDR-H1 having an amino acid sequence of SEQ ID NO: 1 or a variant thereof, CDR-H2 having an amino acid sequence of SEQ ID NO: 2 or a variant thereof, and CDR-H3 having an amino acid sequence of SEQ ID NO: 3 or a variant thereof; and, a light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;

(1b) a heavy chain variable region comprising the following three CDRs: CDR-H1 having an amino acid sequence of SEQ ID NO: 7 or a variant thereof, CDR-H2 having an amino acid sequence of SEQ ID NO: 8 or a variant thereof, and CDR-H3 having an amino acid sequence of SEQ ID NO: 9 or a variant thereof; and, a light chain variable region comprising the following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;

(1c) a heavy chain variable region comprising the following three CDRs: CDR-H1 having an amino acid sequence of SEQ ID NO: 13 or a variant thereof, CDR-H2 having an amino acid sequence of SEQ ID NO: 14 or a variant thereof, and CDR-H3 having an amino acid sequence of SEQ ID NO: 15 or a variant thereof; and, a light chain variable region comprising the following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;

(1d) a heavy chain variable region comprising the following three CDRs: CDR-H1 having an amino acid sequence of SEQ ID NO: 19 or a variant thereof, CDR-H2 having an amino acid sequence of SEQ ID NO: 20 or a variant thereof, and CDR-H3 having an amino acid sequence of SEQ ID NO: 21 or a variant thereof; and, a light chain variable region comprising the following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;

(1e) a heavy chain variable region comprising the following three CDRs: CDR-H1 having an amino acid sequence of SEQ ID NO: 25 or a variant thereof, CDR-H2 having an amino acid sequence of SEQ ID NO: 26 or a variant thereof, and CDR-H3 having an amino acid sequence of SEQ ID NO: 27 or a variant thereof; and, a light chain variable region comprising the following three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof;

(1f) a heavy chain variable region comprising the following three CDRs: CDR-H1 having an amino acid sequence of SEQ ID NO: 31 or a variant thereof, CDR-H2 having an amino acid sequence of SEQ ID NO: 32 or a variant thereof, and CDR-H3 having an amino acid sequence of SEQ ID NO: 33 or a variant thereof; and, a light chain variable region comprising the following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof;

(1g) a heavy chain variable region comprising the following three CDRs: CDR-H1 having an amino acid sequence of SEQ ID NO: 37 or a variant thereof, CDR-H2 having an amino acid sequence of SEQ ID NO: 38 or a variant thereof, and CDR-H3 having an amino acid sequence of SEQ ID NO: 39 or a variant thereof; and, a light chain variable region comprising the following three CDRs: CDR-L1 having an amino acid sequence of SEQ ID NO: 40 or a variant thereof, CDR-L2 having an amino acid sequence of SEQ ID NO: 41 or a variant thereof, and CDR-L3 having an amino acid sequence of SEQ ID NO: 42 or a variant thereof;

(1h) a heavy chain variable region comprising the following three CDRs: CDR-H1 having an amino acid sequence of SEQ ID NO: 43 or a variant thereof, CDR-H2 having an amino acid sequence of SEQ ID NO: 44 or a variant thereof, and CDR-H3 having an amino acid sequence of SEQ ID NO: 45 or a variant thereof; and, a light chain variable region comprising the following three CDRs: CDR-L1 having an amino acid sequence of SEQ ID NO: 46 or a variant thereof, CDR-L2 having an amino acid sequence of SEQ ID NO: 47 or a variant thereof, and CDR-L3 having an amino acid sequence of SEQ ID NO: 48 or a variant thereof;

(1i) a heavy chain variable region comprising the following three CDRs: CDR-H1 having an amino acid sequence of SEQ ID NO: 49 or a variant thereof, CDR-H2 having an amino acid sequence of SEQ ID NO: 50 or a variant thereof, and CDR-H3 having an amino acid sequence of SEQ ID NO: 51 or a variant thereof; and, a light chain variable region comprising the following three CDRs: CDR-L1 having an amino acid sequence of SEQ ID NO: 52 or a variant thereof, CDR-L2 having an amino acid sequence of SEQ ID NO: 53 or a variant thereof, and CDR-L3 having an amino acid sequence of SEQ ID NO: 54 or a variant thereof;

(1j) a heavy chain variable region comprising the following three CDRs: CDR-H1 having an amino acid sequence of SEQ ID NO: 55 or a variant thereof, CDR-H2 having an amino acid sequence of SEQ ID NO: 56 or a variant thereof, and CDR-H3 having an amino acid sequence of SEQ ID NO: 57 or a variant thereof; and, a light chain variable region comprising the following three CDRs: CDR-L1 having an amino acid sequence of SEQ ID NO: 58 or a variant thereof, CDR-L2 having an amino acid sequence of SEQ ID NO: 59 or a variant thereof, and CDR-L3 having an amino acid sequence of SEQ ID NO: 60 or a variant thereof;

(1k) a heavy chain variable region comprising the following three CDRs: CDR-H1 having an amino acid sequence of SEQ ID NO: 61 or a variant thereof, CDR-H2 having an amino acid sequence of SEQ ID NO: 62 or a variant thereof, and CDR-H3 having an amino acid sequence of SEQ ID NO: 63 or a variant thereof; and, a light chain variable region comprising the following three CDRs: CDR-L1 having an amino acid sequence of SEQ ID NO: 64 or a variant thereof, CDR-L2 having an amino acid sequence of SEQ ID NO: 65 or a variant thereof, and CDR-L3 having an amino acid sequence of SEQ ID NO: 66 or a variant thereof;

(11) a heavy chain variable region comprising the following three CDRs: CDR-H1 having an amino acid sequence of SEQ ID NO: 67 or a variant thereof, CDR-H2 having an amino acid sequence of SEQ ID NO: 68 or a variant thereof, and CDR-H3 having an amino acid sequence of SEQ ID NO: 69 or a variant thereof; and, a light chain variable region comprising the following three CDRs: CDR-L1 having an amino acid sequence of SEQ ID NO: 70 or a variant thereof, CDR-L2 having an amino acid sequence of SEQ ID NO: 71 or a variant thereof, and CDR-L3 having an amino acid sequence of SEQ ID NO: 72 or a variant thereof;

(1m) a heavy chain variable region comprising the following three CDRs: CDR-H1 having an amino acid sequence of SEQ ID NO: 73 or a variant thereof, CDR-H2 having an amino acid sequence of SEQ ID NO: 74 or a variant thereof, and CDR-H3 having an amino acid sequence of SEQ ID NO: 75 or a variant thereof; and, a light chain variable region comprising the following three CDRs: CDR-L1 having an amino acid sequence of SEQ ID NO: 76 or a variant thereof, CDR-L2 having an amino acid sequence of SEQ ID NO: 77 or a variant thereof, and CDR-L3 having an amino acid sequence of SEQ ID NO: 78 or a variant thereof;

(1n) a heavy chain variable region comprising the following three CDRs: CDR-H1 having an amino acid sequence of SEQ ID NO: 79 or a variant thereof, CDR-H2 having an amino acid sequence of SEQ ID NO: 80 or a variant thereof, and CDR-H3 having an amino acid sequence of SEQ ID NO: 81 or a variant thereof; and, a light chain variable region comprising the following three CDRs: CDR-L1 having an amino acid sequence of SEQ ID NO: 82 or a variant thereof, CDR-L2 having an amino acid sequence of SEQ ID NO: 83 or a variant thereof, and CDR-L3 with an amino acid sequence of SEQ ID NO: 84 or a variant thereof;

(1o) a heavy chain variable region comprising the following three CDRs: CDR-H1 having an amino acid sequence of SEQ ID NO: 85 or a variant thereof, CDR-H2 having an amino acid sequence of SEQ ID NO: 86 or a variant thereof, and CDR-H3 having an amino acid sequence of SEQ ID NO: 87 or a variant thereof; and, a light chain variable region comprising the following three CDRs: CDR-L1 having an amino acid sequence of SEQ ID NO: 88 or a variant thereof, CDR-L2 having an amino acid sequence of SEQ ID NO: 89 or a variant thereof, and CDR-L3 having an amino acid sequence of SEQ ID NO: 90 or a variant thereof;

(1p) a heavy chain variable region comprising the following three CDRs: CDR-H1 having an amino acid sequence of SEQ ID NO: 91 or a variant thereof, CDR-H2 having an amino acid sequence of SEQ ID NO: 92 or a variant thereof, and CDR-H3 having an amino acid sequence of SEQ ID NO: 93 or a variant thereof; and, a light chain variable region comprising the following three CDRs: CDR-L1 having an amino acid sequence of SEQ ID NO: 94 or a variant thereof, CDR-L2 having an amino acid sequence of SEQ ID NO: 95 or a variant thereof, and CDR-L3 having an amino acid sequence of SEQ ID NO: 96 or a variant thereof; or

(1q) a heavy chain variable region comprising the following three CDRs: CDR-H1 having an amino acid sequence of SEQ ID NO: 97 or a variant thereof, CDR-H2 having an amino acid sequence of SEQ ID NO: 98 or a variant thereof, and CDR-H3 having an amino acid sequence of SEQ ID NO: 99 or a variant thereof; and, a light chain variable region comprising the following three CDRs: CDR-L1 having an amino acid sequence of SEQ ID NO: 100 or a variant thereof, CDR-L2 having an amino acid sequence of SEQ ID NO: 101 or a variant thereof, and CDR-L3 having an amino acid sequence of SEQ ID NO: 102 or a variant thereof.

3. The antibody or antigen-binding fragment thereof according to claim 1 or 2, which further comprises a heavy chain constant region (CH) and a light chain constant region (CL);

preferably, the heavy chain constant region is a rabbit or sheep heavy chain constant region, and the light chain constant region is a rabbit or sheep light chain constant region;

preferably, the heavy chain constant region is a human heavy chain constant region, and the light chain constant region is a human light chain constant region;

preferably, the antibody or antigen-binding fragment thereof is an IgG, IgM, IgE, IgD or IgA antibody;

preferably, the heavy chain constant region is an IgG heavy chain constant region, such as an IgG1, IgG2, IgG3 or IgG4 heavy chain constant region;

preferably, the light chain constant region is a $\kappa$ or $\lambda$ light chain constant region (e.g., a human $\lambda$ light chain constant region).

4. The antibody or antigen-binding fragment thereof according to any one of claims 1 to 3, wherein the antigen-binding fragment is selected from the group consisting of scFv, Fab, Fab', (Fab')$_2$, Fd, Fv, CDR fragment, nanobody, disulfide bond-linked Fv (dsFv), diabody, bispecific antibody and multispecific antibody; and/or, the antibody is a rabbit or sheep antibody, a chimeric antibody or a humanized antibody.

5. The antibody or antigen-binding fragment thereof according to any one of claims 1 to 4, which has a detectable label; preferably, the label is selected from the group consisting of fluorescein, chemiluminescence (e.g., acridinium esters), enzymes (e.g., horseradish peroxidase, alkaline phosphatase), radioisotope, biotin, colloidal gold and magnetic particle.

6. An antibody or antigen-binding fragment thereof that specifically binds to TSH or a mutant thereof, wherein the antibody or antigen-binding fragment thereof is selected from the group consisting of:

produced based on a plasmid in *Escherichia coli* Rosetta™ (DE3) pLysS hTS01 VH deposited at the All-Russian National Collection of Industrial Microorganisms (VKPM) with Accession Number B-14885 and a plasmid in Escherichia coli RosettaTM (DE3) pLysS hTS01 VL deposited at VKPM with Accession Number B-14884;

produced based on a plasmid in *Escherichia coli* Rosetta™ (DE3) pLysS hTS02 VH deposited at the VKPM with Accession Number B-14887 and a plasmid in Escherichia coli RosettaTM (DE3) pLysS hTS02 VL deposited at VKPM with Accession Number B-14886;

produced based on a plasmid in *Escherichia coli* Rosetta™ (DE3) pLysS hTS03 VH deposited at the VKPM with Accession Number B-14889 and a plasmid in Escherichia coli RosettaTM (DE3) pLysS hTS03 VL deposited at VKPM with Accession Number B-14888;

produced based on a plasmid in *Escherichia coli* Rosetta™ (DE3) pLysS hTS04 VH deposited at the VKPM with Accession Number B-14891 and a plasmid in Escherichia coli RosettaTM (DE3) pLysS hTS04 VL deposited at VKPM with Accession Number B-14890;

produced based on a plasmid in *Escherichia coli* Rosetta™ (DE3) pLysS hTS05 VH deposited at the VKPM with Accession Number B-14893 and a plasmid in Escherichia coli RosettaTM (DE3) pLysS hTS05 VL deposited at VKPM with Accession Number B-14892;

produced based on a plasmid in *Escherichia coli* Rosetta™ (DE3) pLysS hTS06 VH deposited at the VKPM with Accession Number B-14895 and a plasmid in Escherichia coli RosettaTM (DE3) pLysS hTS06 VL deposited at VKPM with Accession Number B-14896;

produced based on a plasmid in *Escherichia coli* Rosetta™ (DE3) pLysS hTS07 VH deposited at the VKPM with Accession Number B-14897 and a plasmid in Escherichia coli RosettaTM (DE3) pLysS hTS07 VL deposited at VKPM with Accession Number B-14896;

produced based on a plasmid in *Escherichia coli* Rosetta™ (DE3) pLysS hTS08 VH deposited at the VKPM with Accession Number B-14899 and a plasmid in Escherichia coli RosettaTM (DE3) pLysS hTS08 VL deposited at VKPM with Accession Number B-14898;

produced based on a plasmid in *Escherichia coli* Rosetta™ (DE3) pLysS hTS09 VH deposited at the VKPM with Accession Number B-14901 and a plasmid in Escherichia coli RosettaTM (DE3) pLysS hTS09 VL deposited at VKPM with Accession Number B-14900;

produced based on a plasmid in *Escherichia coli* Rosetta™ (DE3) pLysS hTS10 VH deposited at the VKPM with Accession Number B-14903 and a plasmid in Escherichia coli RosettaTM (DE3) pLysS hTS10 VL deposited at VKPM with Accession Number B-14902;

produced based on a plasmid in *Escherichia coli* Rosetta™ (DE3) pLysS hTS11 VH deposited at the VKPM with Accession Number B-14905 and a plasmid in Escherichia coli RosettaTM (DE3) pLysS hTS11 VL deposited at VKPM with Accession Number B-14904;

produced based on a plasmid in *Escherichia coli* Rosetta™ (DE3) pLysS hTS12 VH deposited at the VKPM with Accession Number B-14907 and a plasmid in Escherichia coli RosettaTM (DE3) pLysS hTS13 VL deposited at VKPM with Accession Number B-14906;

produced based on a plasmid in *Escherichia coli* Rosetta™ (DE3) pLysS hTS13 VH deposited at the VKPM with Accession Number B-14909 and a plasmid in Escherichia coli RosettaTM (DE3) pLysS hTS13 VL deposited at VKPM with Accession Number B-14908;

produced based on a plasmid in *Escherichia coli* Rosetta™ (DE3) pLysS hTS14 VH deposited at the VKPM with Accession Number B-14911 and a plasmid in Escherichia coli RosettaTM (DE3) pLysS hTS14 VL deposited at VKPM with Accession Number B-14910;

produced based on a plasmid in *Escherichia coli* Rosetta™ (DE3) pLysS hTS15 VH deposited at the VKPM with Accession Number B-14913 and a plasmid in Escherichia coli RosettaTM (DE3) pLysS hTS15 VL deposited at VKPM with Accession Number B-14912;

produced based on a plasmid in *Escherichia coli* Rosetta™ (DE3) pLysS hTS16 VH deposited at the VKPM with Accession Number B-14915 and a plasmid in Escherichia coli RosettaTM (DE3) pLysS hTS16 VL deposited at VKPM with Accession Number B-14914; and

produced based on a plasmid in *Escherichia coli* Rosetta™ (DE3) pLysS hTS17 VH deposited at the VKPM with Accession Number B-14917 and a plasmid in Escherichia coli RosettaTM (DE3) pLysS hTS17 VL deposited at VKPM with Accession Number B-14916.

7. The antibody or antigen-binding fragment thereof according to any one of claims 1 to 6, which specifically recognizes a TSH dimer or a β subunit of TSH, and optionally an R55G mutant of TSH.

8. An isolated nucleic acid molecule, which encodes the antibody or antigen-binding fragment thereof according to any one of claims 1 to 7.

9. An expression vector, which comprises the isolated nucleic acid molecule according to claim 8;
preferably, the vector is a plasmid, a virus, a bacteriophage, a bacterium or a viroid.

10. A host cell, which comprises the isolated nucleic acid molecule according to claim 8 or the expression vector according to claim 9;

preferably, the host cell is a eukaryotic cell, preferably a mammalian cell;

preferably, the host cell is a prokaryotic cell, preferably *Escherichia coli.*

11. A TSH detection kit, which is used for detecting the presence or level of TSH or a mutant thereof in a sample;

preferably, the detection functional sensitivity of the detection kit for the TSH or mutant thereof is ≤0.0015 μIU/mL, preferably ≤0.001 μIU/mL;

preferably, the TSH or mutant thereof is selected from the group consisting of TSH dimer, β subunit of TSH and/or R55G mutant of TSH.

12. The detection kit according to claim 11, which comprises a first reagent and a second reagent, wherein the first reagent comprises magnetic beads coated with a first antibody capable of recognizing the TSH or a mutant thereof; and the second reagent comprises a second antibody connected with a chemiluminescent label, wherein a sandwich complex of the first antibody-an antigen-the second antibody can be formed after the sample is mixed with the first reagent and the second reagent, and the antigen is the TSH or mutant thereof.

13. The detection kit according to claim 12, wherein the first antibody and/or the second antibody is selected from an antibody or antigen-binding fragment thereof that specifically binds to TSH or a mutant thereof, wherein the antibody or antigen-binding fragment thereof comprises:

a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or,

a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof;

preferably, the first antibody and/or the second antibody comprises:

a heavy chain variable region comprising the following three CDRs: CDR-H1 having an amino acid sequence of SEQ ID NO: 1 or a variant thereof, CDR-H2 having an amino acid sequence of SEQ ID NO: 2 or a variant thereof, and CDR-H3 having an amino acid sequence of SEQ ID NO: 3 or a variant thereof; and, a light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;

a heavy chain variable region comprising the following three CDRs: CDR-H1 having an amino acid sequence of SEQ ID NO: 7 or a variant thereof, CDR-H2 having an amino acid sequence of SEQ ID NO: 8 or a variant thereof, and CDR-H3 having an amino acid sequence of SEQ ID NO: 9 or a variant thereof; and, a light chain variable region comprising the following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;

a heavy chain variable region comprising the following three CDRs: CDR-H1 having an amino acid sequence of SEQ ID NO: 13 or a variant thereof, CDR-H2 having an amino acid sequence of SEQ ID NO: 14 or a variant thereof, and CDR-H3 having an amino acid sequence of SEQ ID NO: 15 or a variant thereof; and, a light chain variable region comprising the following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;

a heavy chain variable region comprising the following three CDRs: CDR-H1 having an amino acid sequence of SEQ ID NO: 19 or a variant thereof, CDR-H2 having an amino acid sequence of SEQ ID NO: 20 or a variant thereof, and CDR-H3 having an amino acid sequence of SEQ ID NO: 21 or a variant thereof; and, a light chain variable region comprising the following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;

a heavy chain variable region comprising the following three CDRs: CDR-H1 having an amino acid sequence of SEQ ID NO: 25 or a variant thereof, CDR-H2 having an amino acid sequence of SEQ ID NO: 26 or a variant thereof, and CDR-H3 having an amino acid sequence of SEQ ID NO: 27 or a variant thereof; and, a light chain variable region comprising the following three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof;

a heavy chain variable region comprising the following three CDRs: CDR-H1 having an amino acid sequence of SEQ ID NO: 31 or a variant thereof, CDR-H2 having an amino acid sequence of SEQ ID NO: 32 or a variant thereof, and CDR-H3 having an amino acid sequence of SEQ ID NO: 33 or a variant thereof; and, a light chain variable region comprising the following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof;

a heavy chain variable region comprising the following three CDRs: CDR-H1 having an amino acid sequence of SEQ ID NO: 37 or a variant thereof, CDR-H2 having an amino acid sequence of SEQ ID NO: 38 or a variant thereof, CDR-H3 having an amino acid sequence of SEQ ID NO: 39 or a variant thereof; and, a light chain variable region comprising the following three CDRs: CDR-L1 having an amino acid sequence of SEQ ID NO: 40 or a variant thereof, CDR-L2 having an amino acid sequence of SEQ ID NO: 41 or a variant thereof, and CDR-L3 having an amino acid sequence of SEQ ID NO: 42 or a variant thereof;

a heavy chain variable region comprising the following three CDRs: CDR-H1 having an amino acid sequence of SEQ ID NO: 43 or a variant thereof, CDR-H2 having an amino acid sequence of SEQ ID NO: 44 or a variant thereof, and CDR-H3 having an amino acid sequence of SEQ ID NO: 45 or a variant thereof; and, a light chain variable region comprising the following three CDRs: CDR-L1 having an amino acid sequence of SEQ ID NO: 46 or a variant thereof, CDR-L2 having an amino acid sequence of SEQ ID NO: 47 or a variant thereof, and CDR-L3 having an amino acid sequence of SEQ ID NO: 48 or a variant thereof;

a heavy chain variable region comprising the following three CDRs: CDR-H1 having an amino acid sequence of SEQ ID NO: 49 or a variant thereof, CDR-H2 having an amino acid sequence of SEQ ID NO: 50 or a variant thereof, and CDR-H3 having an amino acid sequence of SEQ ID NO: 51 or a variant thereof; and, a light chain variable region comprising the following three CDRs: CDR-L1 having an amino acid sequence of SEQ ID NO: 52 or a variant thereof, CDR-L2 having an amino acid sequence of SEQ ID NO: 53 or a variant thereof, and CDR-L3 having an amino acid sequence of SEQ ID NO: 54 or a variant thereof;

a heavy chain variable region comprising the following three CDRs: CDR-H1 having an amino acid sequence of SEQ ID NO: 55 or a variant thereof, CDR-H2 having an amino acid sequence of SEQ ID NO: 56 or a variant thereof, and CDR-H3 having an amino acid sequence of SEQ ID NO: 57 or a variant thereof; and, a light chain variable region comprising the following three CDRs: CDR-L1 having an amino acid sequence of SEQ ID NO: 58 or a variant thereof, CDR-L1 having an amino acid sequence of SEQ ID NO: 59 or a variant thereof, and CDR-L3 having an amino acid sequence of SEQ ID NO: 60 or a variant thereof;

a heavy chain variable region comprising the following three CDRs: CDR-H1 having an amino acid sequence of SEQ ID NO: 61 or a variant thereof, CDR-H2 having an amino acid sequence of SEQ ID NO: 62 or a variant thereof, and CDR-H3 having an amino acid sequence of SEQ ID NO: 63 or a variant thereof; and, a light chain variable region comprising the following three CDRs: CDR-L1 having an amino acid sequence of SEQ ID NO: 64 or a variant thereof, CDR-L2 having an amino acid sequence of SEQ ID NO: 65 or a variant thereof, and CDR-L3 having an amino acid sequence of SEQ ID NO: 66 or a variant thereof;

a heavy chain variable region comprising the following three CDRs: CDR-H1 having an amino acid sequence of SEQ ID NO: 67 or a variant thereof, CDR-H2 having an amino acid sequence of SEQ ID NO: 68 or a variant thereof, CDR-H3 having an amino acid sequence of SEQ ID NO: 69 or a variant thereof; and, a light chain variable region comprising the following three CDRs: CDR-L1 having an amino acid sequence of SEQ ID NO: 70 or a variant thereof, CDR-L2 having an amino acid sequence of SEQ ID NO: 71 or a variant thereof, and CDR-L3 having an amino acid sequence of SEQ ID NO: 72 or a variant thereof;

a heavy chain variable region comprising the following three CDRs: CDR-H1 having an amino acid sequence of SEQ ID NO: 73 or a variant thereof, CDR-H2 having an amino acid sequence of SEQ ID NO: 74 or a variant thereof, and CDR-H3 having an amino acid sequence of SEQ ID NO: 75 or a variant thereof; and, a light chain variable region comprising the following three CDRs: CDR-L1 having an amino acid sequence of SEQ ID NO: 76 or a variant thereof, CDR-L2 having an amino acid sequence of SEQ ID NO: 77 or a variant thereof, and CDR-L3 having an amino acid sequence of SEQ ID NO: 78 or a variant thereof;

a heavy chain variable region comprising the following three CDRs: CDR-H1 having an amino acid sequence of SEQ ID NO: 79 or a variant thereof, CDR-H2 having an amino acid sequence of SEQ ID NO: 80 or a variant thereof, and CDR-H3 having an amino acid sequence of SEQ ID NO: 81 or a variant thereof; and, a light chain variable region comprising the following three CDRs: CDR-L1 having an amino acid sequence of SEQ ID NO: 82 or a variant thereof, CDR-L2 having an amino acid sequence of SEQ ID NO: 83 or a variant thereof, and CDR-L3 having an amino acid sequence of SEQ ID NO: 84 or a variant thereof;

a heavy chain variable region comprising the following three CDRs: CDR-H1 having an amino acid sequence of SEQ ID NO: 85 or a variant thereof, CDR-H2 having an amino acid sequence of SEQ ID NO: 86 or a variant thereof, and CDR-H3 having an amino acid sequence of SEQ ID NO: 87 or a variant thereof; and, a light chain variable region comprising the following three CDRs: CDR-L1 having an amino acid sequence of SEQ ID NO: 88 or a variant thereof, CDR-L2 having an amino acid sequence of SEQ ID NO: 89 or a variant thereof, and CDR-L3 having an amino acid sequence of SEQ ID NO: 90 or a variant thereof;

a heavy chain variable region comprising the following three CDRs: CDR-H1 having an amino acid sequence of SEQ ID NO: 91 or a variant thereof, CDR-H2 having an amino acid sequence of SEQ ID NO: 92 or a variant thereof, and CDR-H3 having an amino acid sequence of SEQ ID NO: 93 or a variant thereof; and, a light chain variable region comprising the following three CDRs: CDR-L1 having an amino acid sequence of SEQ ID NO: 94 or a variant thereof, CDR-L2 having an amino acid sequence of SEQ ID NO: 95 or a variant thereof, and CDR-L3 having an amino acid sequence of SEQ ID NO: 96 or a variant thereof; or

a heavy chain variable region comprising the following three CDRs: CDR-H1 having an amino acid sequence of SEQ ID NO: 97 or a variant thereof, CDR-H2 having an amino acid sequence of SEQ ID NO: 98 or a variant thereof, CDR-H3 having an amino acid sequence of SEQ ID NO: 99 or a variant thereof; and a light chain variable region comprising the following three CDRs: CDR-L1 having an amino acid sequence of SEQ ID NO: 100 or a variant thereof, CDR-L2 having an amino acid sequence of SEQ ID NO: 101 or a variant thereof, and CDR-L3 having an amino acid sequence of SEQ ID NO: 102 or a variant thereof.

14. The detection kit according to claim 13, wherein:

i) the first antibody is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof; and,
the second antibody is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof;

ii) the first antibody is an antibody or antigen-binding fragment thereof having a heavy chain variable region comprising the following three CDRs: CDR-H1 having an amino acid sequence selected from SEQ ID NO: 37 and variants thereof, CDR-H2 having an amino acid sequence selected from SEQ ID NO: 38 and variants thereof, and CDR-H3 having an amino acid sequence selected from SEQ ID NO: 39 and variants thereof; and, a light chain variable region comprising the following three CDRs: CDR-L1 having an amino acid sequence selected from SEQ ID NO: 40 and variants thereof, CDR-L2 having an amino acid sequence selected from SEQ ID NO: 41 and variants thereof, and CDR-L3 having an amino acid sequence selected from SEQ ID NO: 42 and variants thereof; and,

the second antibody is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-H3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof;

iii) the first antibody is an antibody or antigen-binding fragment thereof having a heavy chain variable region comprising the following three CDRs: CDR-H1 having an amino acid sequence selected from SEQ ID NO: 25 and variants thereof, CDR-H2 having an amino acid sequence selected from SEQ ID NO: 26 and variants thereof, and CDR-L3 having an amino acid sequence selected from SEQ ID NO: 27 and variants thereof; and, a light chain variable region comprising the following three CDRs: CDR-L1 having an amino acid sequence selected from SEQ ID NO: 28 and variants thereof, CDR-L2 having an amino acid sequence selected from SEQ ID NO: 29 and variants thereof, and CDR-L3 having an amino acid sequence selected from SEQ ID NO: 30 and variants thereof; and,

the second antibody is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof;

iv) the first antibody is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 having an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: 23 and variants thereof, and CDR-L3 having an amino acid sequence selected from SEQ ID NO: 24 and variants thereof; and,

the second antibody is an antibody or antigen-binding fragment thereof having a heavy chain variable region comprising the following three CDRs: CDR-H1 having an amino acid sequence selected from SEQ ID NO: 61 and variants thereof, CDR-H2 having an amino acid sequence selected from SEQ ID NO: 62 and variants thereof, and CDR-H3 having an amino acid sequence selected from SEQ ID NO: 63 and variants thereof; and, a light chain variable region comprising the following three CDRs: CDR-L1 having an amino acid sequence selected from SEQ ID NO: 64 and variants thereof, CDR-L2 having an amino acid sequence selected from SEQ ID NO: 65 and variants thereof, and CDR-L3 having an amino acid sequence selected from SEQ ID NO: 66 and variants thereof; or,

v) the first antibody is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 with an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: ID NO: 23 and variants thereof, and CDR-L3 having an amino acid sequence selected from SEQ ID NO: 24 and variants thereof; and,

the second antibody is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-H3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof.

15. A composition or TSH detection kit, which comprises the antibody or antigen-binding fragment thereof according to any one of claims 1 to 7.

16. A composition or TSH detection kit, which comprises:

a first antibody, which is an antibody or antigen-binding fragment thereof having a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or, a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof, and,

a second antibody, which is an antibody or antigen-binding fragment thereof having a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or, a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof;

wherein, the first antibody and the second antibody are directed to different epitopes of TSH or a mutant thereof, respectively.

17. The composition or immunoassay kit according to claim 16, wherein:

the first antibody is an antibody or antigen-binding fragment thereof having a heavy chain variable region comprising the following three CDRs: CDR-H1 having an amino acid sequence selected from SEQ ID NOs: 7, 13, 19, 25, 31, 37 and variants thereof, CDR-H2 having an amino acid sequence selected from SEQ ID NOs: 8, 14, 20, 26, 32, 38 and variants thereof, and/or CDR-H3 having an amino acid sequence selected from SEQ ID NOs: 9, 15, 21, 27, 33, 39 and variants thereof; and/or a light chain variable region comprising the following three CDRs: CDR-L1 having an amino acid sequence selected from SEQ ID NOs: 10, 16, 22, 28, 34, 40 and variants thereof, CDR-L2 having an amino acid sequence selected from SEQ ID NOs: 11, 17, 23, 29, 35, 41 and variants thereof, and/or CDR-L3 having an amino acid sequence selected from SEQ ID NOs: 12, 18, 24, 30, 36, 42 and variants thereof, and,

the second antibody is an antibody or antigen-binding fragment thereof having a heavy chain variable region comprising the following three CDRs: CDR-H1 having an amino acid sequence selected from SEQ ID NOs: 1, 43, 49, 55, 61, 67, 73, 79, 85, 91, 97 and variants thereof, CDR-H2 having an amino acid sequence selected from SEQ ID NOs: 2, 44, 50, 56, 62, 68, 74, 80, 86, 92, 98 and variants thereof, and/or CDR-H3 having an amino acid sequence selected from SEQ ID NOs: 3, 45, 51, 57, 63, 69, 75, 81, 87, 93, 99 and variants thereof; and/or, a light chain variable region comprising the following three CDRs: CDR-L1 having an amino acid sequence selected from SEQ ID NOs: 4, 46, 52, 58, 64, 70, 76, 82, 88, 94, 100 and variants thereof, CDR-L2 having an amino acid sequence selected from SEQ ID NOs: 5, 47, 53, 59, 65, 71, 77, 83, 89, 95, 101 and variants thereof, and/or CDR-L3 having an amino acid sequence selected from SEQ ID NOs: 6, 48, 54, 60, 66, 72, 78, 84, 90, 96, 102 and variants thereof.

18. The composition or TSH detection kit according to claim 15 or 16, which comprises:

i) a first antibody, which is an antibody or antigen-binding fragment thereof having a heavy chain variable region comprising the following three CDRs: CDR-H1 having an amino acid sequence selected from SEQ ID NO: 1 and variants thereof, CDR-H2 having an amino acid sequence selected from SEQ ID NO: 2 and variants thereof, and CDR-H3 having an amino acid sequence selected from SEQ ID NO: 3 and variants thereof; and, a light chain variable region comprising the following three CDRs: CDR-L1 having an amino acid sequence selected from SEQ ID NO: 4 and variants thereof, CDR-L2 having an amino acid sequence selected from SEQ ID NO: 5 and variants thereof, and CDR-L3 having an amino acid sequence selected from SEQ ID NO: 6 and variants thereof, and, a second antibody, which is an antibody or antigen-binding fragment thereof having a heavy chain variable region comprising the following three CDRs: CDR-H1 having an amino acid sequence selected from SEQ ID NO: 13 and variants thereof, CDR-H2 having an amino acid sequence selected from SEQ ID NO: 14 and variants thereof, and CDR-L3 having an amino acid sequence selected from SEQ ID NO: 15 and variants thereof; and, a light chain variable region comprising the following three CDRs: CDR-L1 having an amino acid sequence selected from SEQ ID NO: 16 and variants thereof, CDR-L2 having an amino acid sequence selected from SEQ ID NO: 17 and variants thereof, and CDR-L3 having an amino acid sequence selected from SEQ ID NO: 18 and variants thereof;

ii) a first antibody, which is an antibody or antigen-binding fragment thereof having a heavy chain variable region comprising the following three CDRs: CDR-H1 having an amino acid sequence selected from SEQ ID NO: 1 and variants thereof, CDR-H2 having an amino acid sequence selected from SEQ ID NO: 2 and variants thereof, and CDR-H3 having an amino acid sequence selected from SEQ ID NO: 3 and variants thereof; and, a light chain variable region comprising the following three CDRs: CDR-L1 having an amino acid sequence selected from SEQ ID NO: 4 and variants thereof, CDR-L2 having an amino acid sequence selected from SEQ ID NO: 5 and variants thereof, and CDR-L3 having an amino acid sequence selected from SEQ ID NO: 6 and variants thereof, and, a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

iii) a first antibody, which is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-L3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof; and, a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

iv) a first antibody, which is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof, and,

a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-L3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

v) a first antibody, which is an antibody or antigen-binding fragment thereof having a heavy chain variable region comprising the following three CDRs: CDR-H1 having an amino acid sequence selected from SEQ ID NO: 7 and variants thereof, CDR-H2 having an amino acid sequence selected from SEQ ID NO: 8 and variants thereof, and CDR-H3 having an amino acid sequence selected from SEQ ID NO: 9 and variants thereof; and, a light chain variable region comprising the following three CDRs: CDR-L1 having an amino acid sequence selected from SEQ ID NO: 10 and variants thereof, CDR-L2 having an amino acid sequence selected from SEQ ID NO: 11 and variants thereof, and CDR-L3 having an amino acid sequence selected from SEQ ID NO: 12 and variants thereof CDR-L3, and,

a second antibody, which is an antibody or antigen-binding fragment thereof having a heavy chain variable region comprising the following three CDRs: CDR-H1 having an amino acid sequence selected from SEQ ID NO: 55 and variants thereof, CDR-H2 having an amino acid sequence selected from SEQ ID NO: 56 and variants thereof, CDR-H3 having an amino acid sequence selected from SEQ ID NO: 57 and variants thereof; and, a light chain variable region comprising the following three CDRs: CDR-L1 having an amino acid sequence selected from SEQ ID NO: 58 and variants thereof, CDR-L2 having an amino acid sequence selected from SEQ ID NO: 59 and variants thereof, and CDR-L3 having an amino acid sequence selected from SEQ ID NO: 60 and variants thereof;

vi) a first antibody, which is an antibody or antigen-binding fragment thereof having a heavy chain variable region comprising the following three CDRs: CDR-H1 having an amino acid sequence selected from SEQ ID NO: 7 and variants thereof, CDR-H2 having an amino acid sequence selected from SEQ ID NO: 8 and variants thereof, and CDR-H3 having an amino acid sequence selected from SEQ ID NO: 9 and variants thereof; and, a light chain variable region comprising the following three CDRs: CDR-L1 having an amino acid sequence selected from SEQ ID NO: 10 and variants thereof, CDR-L2 having an amino acid sequence selected from SEQ ID NO: 11 and variants thereof, and CDR-L3 having an amino acid sequence selected from SEQ ID NO: 12 and variants thereof, and,

a second antibody, which is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof;

vii) a first antibody, which is an antibody or antigen-binding fragment thereof having a heavy chain variable region comprising the following three CDRs: CDR-H1 having an amino acid sequence selected from SEQ ID NO: 13 and variants thereof, CDR-H2 having an amino acid sequence selected from SEQ ID NO: 14 and variants thereof, and CDR-H3 having an amino acid sequence selected from SEQ ID NO: 15 and variants thereof; and, a light chain variable region comprising the following three CDRs: CDR-L1 having an amino acid sequence selected from SEQ ID NO: 16 and variants thereof, CDR-L2 having an amino acid sequence selected from SEQ ID NO: 17 and variants thereof, and CDR-L3 having an amino acid sequence selected from SEQ ID NO: 18 and variants thereof; and,

a second antibody, which is an antibody or antigen-binding fragment thereof having a heavy chain variable region comprising the following three CDRs: CDR-H1 having an amino acid sequence selected from SEQ ID NO: 55 and variants thereof, CDR-H2 having an amino acid sequence selected from SEQ ID NO: 56 and variants thereof, and CDR-H3 having an amino acid sequence selected from SEQ ID NO: 57 and variants thereof; and, a light chain variable region comprising the following three CDRs: CDR-L1 having an amino acid sequence selected from SEQ ID NO: 58 and variants thereof, CDR-L2 having an amino acid sequence selected from SEQ ID NO: 59 and variants thereof, and CDR-L3 having an amino acid sequence selected from SEQ ID NO: 60 and variants thereof;

viii) a first antibody, which is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 having an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: 23 and variants thereof, and CDR-L3 having an amino acid sequence selected from SEQ ID NO: 24 and variants thereof; and,
a second antibody, which is an antibody or antigen-binding fragment thereof having a heavy chain variable region comprising the following three CDRs: CDR-H1 having an amino acid sequence selected from SEQ ID NO: 85 and variants thereof, CDR-H2 having an amino acid sequence selected from SEQ ID NO: 86 and variants thereof, and CDR-H3 having an amino acid sequence selected from SEQ ID NO: 87 and variants thereof; and, a light chain variable region comprising the following three CDRs: CDR-L1 having an amino acid sequence selected from SEQ ID NO: 88 and variants thereof, CDR-L2 having an amino acid sequence selected from SEQ ID NO: 89 and variants thereof, and CDR-L3 having an amino acid sequence selected from SEQ ID NO: 90 and variants thereof;

ix) a first antibody, which is an antibody or antigen-binding fragment thereof having a heavy chain variable region comprising the following three CDRs: CDR-H1 having an amino acid sequence selected from SEQ ID NO: 67 and variants thereof, CDR-H2 having an amino acid sequence selected from SEQ ID NO: 68 and variants thereof, and CDR-H3 having an amino acid sequence selected from SEQ ID NO: 69 and variants thereof; and, a light chain variable region comprising the following three CDRs: CDR-L1 having an amino acid sequence selected from SEQ ID NO: 70 and variants thereof, CDR-L2 having an amino acid sequence selected from SEQ ID NO: 71 and variants thereof, and CDR-L3 having an amino acid sequence selected from SEQ ID NO: 72 and variants thereof; and,
a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

x) a first antibody, which is an antibody or antigen-binding fragment thereof having a heavy chain variable region comprising the following three CDRs: CDR-H1 having an amino acid sequence selected from SEQ ID NO: 79 and variants thereof, CDR-H2 having an amino acid sequence selected from SEQ ID NO: 80 and variants thereof, and CDR-H3 having an amino acid sequence selected from SEQ ID NO: 81 and variants thereof; and, a light chain variable region comprising the following three CDRs: CDR-L1 having an amino acid sequence selected from SEQ ID NO: 82 and variants thereof, CDR-L2 having an amino acid sequence selected from SEQ ID NO: 83 and variants thereof, and CDR-L3 having an amino acid sequence selected from SEQ ID NO: 84 and variants thereof; and,
a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

xi) a first antibody, which is an antibody or antigen-binding fragment thereof having a heavy chain variable region comprising the following three CDRs: CDR-H1 having an amino acid sequence selected from SEQ ID NO: 25 and variants thereof, CDR-H2 having an amino acid sequence selected from SEQ ID NO: 26 and variants thereof, and CDR-H3 having an amino acid sequence selected from SEQ ID NO: 27 and variants thereof; and, a light chain variable region comprising the following three CDRs: CDR-L1 having an amino acid sequence selected from SEQ ID NO: 28 and variants thereof, CDR-L2 having an amino acid sequence selected from SEQ ID NO: 29 and variants thereof, and CDR-L3 having an amino acid sequence selected from SEQ ID NO: 30 and variants thereof; and,

a second antibody, which is an antibody or antigen-binding fragment thereof having a heavy chain variable region comprising the following three CDRs: CDR-H1 having an amino acid sequence selected from SEQ ID NO: 73 and variants thereof, CDR-H2 having an amino acid sequence selected from SEQ ID NO: 74 and variants thereof, and CDR-L3 having an amino acid sequence selected from SEQ ID NO: 75 and variants thereof; and, a light chain variable region comprising the following three CDRs: CDR-L1 having an amino acid sequence selected from SEQ ID NO: 76 and variants thereof, CDR-L2 having an amino acid sequence selected from SEQ ID NO: 77 and variants thereof, and CDR-L3 having an amino acid sequence selected from SEQ ID NO: 78 and variants thereof;

xii) a first antibody, which is an antibody or antigen-binding fragment thereof having a heavy chain variable region comprising the following three CDRs: CDR-H1 having an amino acid sequence selected from SEQ ID NO: 25 and variants thereof, CDR-H2 having an amino acid sequence selected from SEQ ID NO: 26 and variants thereof, and CDR-H3 having an amino acid sequence selected from SEQ ID NO: 27 and variants thereof; and, a light chain variable region comprising the following three CDRs: CDR-L1 having an amino acid sequence selected from SEQ ID NO: 28 and variants thereof, CDR-L2 having an amino acid sequence selected from SEQ ID NO: 29 and variants thereof, and CDR-L3 having an amino acid sequence selected from SEQ ID NO: 30 and variants thereof; and,

a second antibody, which is an antibody or antigen-binding fragment thereof having a heavy chain variable region comprising the following three CDRs: CDR-H1 having an amino acid sequence selected from SEQ ID NO: 85 and variants thereof, CDR-H2 having an amino acid sequence selected from SEQ ID NO: 86 and variants thereof, and CDR-H3 having an amino acid sequence selected from SEQ ID NO: 87 and variants thereof; and, a light chain variable region comprising the following three CDRs: CDR-L1 having an amino acid sequence selected from SEQ ID NO: 88 and variants thereof, CDR-L2 having an amino acid sequence selected from SEQ ID NO: 89 and variants thereof, and CDR-L3 having an amino acid sequence selected from SEQ ID NO: 90 and variants thereof;

xiii) a first antibody, which is an antibody or antigen-binding fragment thereof having a heavy chain variable region comprising the following three CDRs: CDR-H1 having an amino acid sequence selected from SEQ ID NO: 37 and variants thereof, CDR-H2 having an amino acid sequence selected from SEQ ID NO: 38 and variants thereof, and CDR-H3 having an amino acid sequence selected from SEQ ID NO: 39 and variants thereof; and, a light chain variable region comprising the following three CDRs: CDR-L1 having an amino acid sequence selected from SEQ ID NO: 40 and variants thereof, CDR-L2 having an amino acid sequence selected from SEQ ID NO: 41 and variants thereof, and CDR-L3 having an amino acid sequence selected from SEQ ID NO: 42 and variants thereof; and,

a second antibody, which is an antibody or antigen-binding fragment thereof having a heavy chain variable region comprising the following three CDRs: CDR-H1 having an amino acid sequence selected from SEQ ID NO: 55 and variants thereof, CDR-H2 having an amino acid sequence selected from SEQ ID NO: 56 and variants thereof, and CDR-H3 having an amino acid sequence selected from SEQ ID NO: 57 and variants thereof; and, a light chain variable region comprising the following three CDRs: CDR-L1 having an amino acid sequence selected from SEQ ID NO: 58 and variants thereof, CDR-L2 having an amino acid sequence selected from SEQ ID NO: 59 and variants thereof, and CDR-L3 having an amino acid sequence selected from SEQ ID NO: 60 and variants thereof;

xiv) a first antibody, which is an antibody or antigen-binding fragment thereof having a heavy chain variable region comprising the following three CDRs: CDR-H1 having an amino acid sequence selected from SEQ ID NO: 91 and variants thereof, CDR-H2 having an amino acid sequence selected from SEQ ID NO: 92 and variants thereof, and CDR-H3 having an amino acid sequence selected from SEQ ID NO: 93 and variants thereof; and, a light chain variable region comprising the following three CDRs: CDR-L1 having an amino acid sequence selected from SEQ ID NO: 94 and variants thereof, CDR-L2 having an amino acid sequence selected from SEQ ID NO: 95 and variants thereof, and CDR-L3 having an amino acid sequence selected from SEQ ID NO: 96 and variants thereof, and,

a second antibody, which is an antibody or antigen-binding fragment thereof having a heavy chain variable region comprising the following three CDRs: CDR-H1 having an amino acid sequence selected from SEQ ID NO: 13 and variants thereof, CDR-H2 having an amino acid sequence selected from SEQ ID NO: 14 and variants thereof, and CDR-H3 having an amino acid sequence selected from SEQ ID NO: 15 and variants thereof; and, a light chain variable region comprising the following three CDRs: CDR-L1 having an amino acid sequence selected from SEQ ID NO: 16 and variants thereof, CDR-L2 having an amino acid sequence selected from SEQ ID NO: 17 and variants thereof, and CDR-L3 having an amino acid sequence selected from SEQ ID NO: 18 and variants thereof; or,

xv) a first antibody, which is an antibody or antigen-binding fragment thereof having a heavy chain variable region comprising the following three CDRs: CDR-H1 having an amino acid sequence selected from SEQ ID NO: 91 and variants thereof, CDR-H2 having an amino acid sequence selected from SEQ ID NO: 92 and variants thereof, and CDR-H3 having an amino acid sequence selected from SEQ ID NO: 93 and variants thereof; and, a light chain variable region comprising the following three CDRs: CDR-L1 having an amino acid sequence selected from SEQ ID NO: 94 and variants thereof, CDR-L2 having an amino acid sequence selected from SEQ ID NO: 95 and variants thereof, and CDR-L3 having an amino acid sequence selected from SEQ ID NO: 96 and variants thereof, and,
a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof.

19. The composition or TSH detection kit according to claim 15 or 16, which comprises:

i) a first antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof; and
a second antibody, which is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof;

ii) a first antibody, which is an antibody or antigen-binding fragment thereof having a heavy chain variable region comprising the following three CDRs: CDR-H1 having an amino acid sequence selected from SEQ ID NO: 37 and variants thereof, CDR-H2 having an amino acid sequence selected from SEQ ID NO: 38 and variants thereof, and CDR-H3 having an amino acid sequence selected from SEQ ID NO: 39 and variants thereof; and, a light chain variable region comprising the following three CDRs: CDR-L1 having an amino acid sequence selected from SEQ ID NO: 40 and variants thereof, CDR-L2 having an amino acid sequence selected from SEQ ID NO: 41 and variants thereof, and CDR-L3 having an amino acid sequence selected from SEQ ID NO: 42 and variants thereof; and,
a second antibody, which is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-H3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof;

iii) a first antibody, which is an antibody or antigen-binding fragment thereof having a heavy chain variable region comprising the following three CDRs: CDR-H1 having an amino acid sequence selected from SEQ ID NO: 25 and variants thereof, CDR-H2 having an amino acid sequence selected from SEQ ID NO: 26 and variants thereof, and CDR-L3 having an amino acid sequence selected from SEQ ID NO: 27 and variants thereof; and, a light chain variable region comprising the following three CDRs: CDR-L1 having an amino acid sequence selected from SEQ ID NO: 28 and variants thereof, CDR-L2 having an amino acid sequence selected from SEQ ID NO: 29 and variants thereof, and CDR-L3 having an amino acid sequence selected from SEQ ID NO: 30 and variants thereof; and,

a second antibody, which is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof;

iv) a first antibody, which is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 having an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: 23 and variants thereof, and CDR-L3 having an amino acid sequence selected from SEQ ID NO: 24 and variants thereof; and,

a second antibody, which is an antibody or antigen-binding fragment thereof having a heavy chain variable region comprising the following three CDRs: CDR-H1 having an amino acid sequence selected from SEQ ID NO: 61 and variants thereof, CDR-H2 having an amino acid sequence selected from SEQ ID NO: 62 and variants thereof, and CDR-H3 having an amino acid sequence selected from SEQ ID NO: 63 and variants thereof; and, a light chain variable region comprising the following three CDRs: CDR-L1 having an amino acid sequence selected from SEQ ID NO: 64 and variants thereof, CDR-L2 having an amino acid sequence selected from SEQ ID NO: 65 and variants thereof, and CDR-L3 having an amino acid sequence selected from SEQ ID NO: 66 and variants thereof; or,

v) the first antibody, which is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 having an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: 23 and variants thereof, and CDR-L3 having an amino acid sequence selected from SEQ ID NO: 24 and variants thereof; and,

a second antibody, which is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-H3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof.

20. The composition or TSH detection kit according to any one of claims 16 to 19, wherein the first antibody is a capture antibody and the second antibody is a detection antibody.

21. Use of the antibody or antigen-binding fragment thereof according to any one of claims 1 to 7 in the manufacture of a kit or use of the immunoassay kit according to any one of claims 11 to 20, wherein the kit is used for:

(1) detection of the presence or level of TSH or a mutant thereof in a sample;

(2) diagnosis or auxiliary diagnosis of a thyroid dysfunction (e.g., hypothyroidism or hyperthyroidism);

(3) diagnosis or auxiliary diagnosis of a thyroid-related disease; and/or

(4) evaluation or auxiliary evaluation of a therapeutic effect on a thyroid-related disease; preferably, the thyroid-related disease is a hyperthyroidism or a thyroid cancer.

22. A sample analyzer, comprising:

a loading device, which is configured to load a sample to be tested and a reagent required for chemiluminescent reaction into a reaction container, wherein the reagent comprises an antibody or antigen-binding fragment thereof that specifically binds to TSH or a mutant thereof;

a reaction incubation device, which is configured to provide a reaction and incubation site for the reaction container containing the sample to be tested and the reagent required for chemiluminescent reaction, so that the sample to be tested and the reagent required for chemiluminescent reaction in the reaction container form a sample liquid to be tested; and

a photometric device, which is configured to perform photometry on the sample liquid to be tested; wherein,

the antibody or the antigen-binding fragment thereof comprises:

a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or,

a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof;

the photometric device comprises:

a receiving component, which is configured to receive a photosignal emitted by the sample to be tested through the chemiluminescent reaction and convert the photosignal into a corresponding electrical signal; and

a processing component, which is configured to be electrically connected to the receiving component and receive the electrical signal from the receiving component, wherein the processing component comprises a first photon counting module and a second photon counting module, the first photon counting module is configured to detect the number of pulses of the electrical signal using a pulse recognition method to obtain a first photon counting result, the second photon counting module is configured to process the electrical signal to obtain a parameter for characterizing photon number, and calculate a second photon counting result according to the parameter for characterizing photon number and a preset calibration function, wherein the calibration function represents a mapping relationship between the parameter for characterizing photon number and the photon counting result, and the processing component is further configured to output a final photon counting result of the sample to be tested based on the first photon counting result and the second photon counting result.

23. The sample analyzer according to claim 22, characterized in that the processing component is further configured to: when the first photon counting result is lower than a first threshold, output the first photon counting result as the final photon counting result; when the first photon counting result is higher than a second threshold, output the second photon counting result as the final photon counting result, wherein the second threshold is greater than or equal to the first threshold.

24. The sample analyzer according to claim 22 or 23, characterized in that the second photon counting module comprises an integration circuit and a second counting circuit electrically connected to each other, the integration circuit is configured to integrate the electrical signal within a predetermined time period to obtain a DC component signal, the parameter for characterizing photon number comprises a parameter related to the DC component signal, and the second counting circuit is configured to calculate the second photon counting result according to the parameter related to the DC component signal and a preset calibration function.

25. A sample analyzer, comprising:

a loading device, which is configured to load a sample to be tested and a reagent required for chemiluminescent reaction into a reaction container, wherein the reagent comprises an antibody or antigen-binding fragment thereof;

a reaction incubation device, which is configured to provide a reaction and incubation site for the reaction container containing the sample to be tested and the reagent required for chemiluminescent reaction, so that the sample to be tested and the reagent required for chemiluminescent reaction in the reaction container form a sample liquid to be tested; and

a photometric device, which is configured to perform photometry on the sample liquid to be tested, wherein the linear detection range of the photometric device is [A1, A2] photons/second, wherein the linear range represents the range of the output photon number of the photometric device, within which the intensity of the luminescent signal generated in the chemiluminescent reaction is linear with the output photon number of the detection device, wherein A1 is less than or equal to 2000 and A2 is greater than or equal to $10^8$;

the antibody or antigen-binding fragment thereof comprises: a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or a light chain variable region comprising the following three CDRs: CDR-L1 having an amino acid sequence selected from the group consisting of SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NO: 6, 12, 18, 24, 30, 36, 42, 48, 54, 60, 66, 72, 78, 84, 90, 96, 102 and variants thereof.

## Claims

1. An antibody or antigen-binding fragment thereof that specifically binds to TSH (thyroid stimulating hormone) or a mutant thereof, comprising:

a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or,
a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof;
wherein each of the variants comprises an amino acid mutation as compared to the amino acid sequence from which it is derived, and the amino acid mutation is a substitution, deletion or addition of one or several amino acids (e.g., a substitution, deletion or addition of 1, 2, 3, 4 or 5 amino acids); preferably, the substitution is a conservative substitution;
preferably, each of the variants has an identity of at least 80%, at least 85%, at least 90%, at least 91%, at least 92%, at least 93%, at least 94%, at least 95%, at least 96%, at least 97%, at least 98%, at least 99%, or 100% as compared to the amino acid sequence from which it is derived.

2. The antibody or antigen-binding fragment thereof according to claim 1, comprising:

(1a) a heavy chain variable region comprising the following three CDRs: CDR-H1 having an amino acid sequence of SEQ ID NO: 1 or a variant thereof, CDR-H2 having an amino acid sequence of SEQ ID NO: 2 or a variant thereof, and CDR-H3 having an amino acid sequence of SEQ ID NO: 3 or a variant thereof; and, a light chain variable region comprising the following three CDRs: CDR-L1 having an amino acid sequence of SEQ ID NO: 4 or a variant thereof, CDR-L2 having an amino acid sequence of SEQ ID NO: 5 or a variant thereof, and CDR-L3 having an amino acid sequence of SEQ ID NO: 6 or a variant thereof;

(1b) a heavy chain variable region comprising the following three CDRs: CDR-H1 having an amino acid sequence of SEQ ID NO: 7 or a variant thereof, CDR-H2 having an amino acid sequence of SEQ ID NO: 8 or a variant thereof, and CDR-H3 having an amino acid sequence of SEQ ID NO: 9 or a variant thereof; and, a light chain variable region comprising the following three CDRs: CDR-L1 having an amino acid sequence of SEQ ID NO: 10 or a variant thereof, CDR-L2 having an amino acid sequence of SEQ ID NO: 11 or a variant thereof, and CDR-L3 having an amino acid sequence of SEQ ID NO: 12 or a variant thereof;

(1c) a heavy chain variable region comprising the following three CDRs: CDR-H1 having an amino acid sequence of SEQ ID NO: 13 or a variant thereof, CDR-H2 having an amino acid sequence of SEQ ID NO: 14 or a variant thereof, and CDR-H3 having an amino acid sequence of SEQ ID NO: 15 or a variant thereof; and, a light chain variable region comprising the following three CDRs: CDR-L1 having an amino acid sequence of SEQ ID NO: 16 or a variant thereof, CDR-L2 having an amino acid sequence of SEQ ID NO: 17 or a variant thereof, and CDR-L3 having an amino acid sequence of SEQ ID NO: 18 or a variant thereof;

(1d) a heavy chain variable region comprising the following three CDRs: CDR-H1 having an amino acid sequence of SEQ ID NO: 19 or a variant thereof, CDR-H2 having an amino acid sequence of SEQ ID NO: 20 or a variant thereof, and CDR-H3 having an amino acid sequence of SEQ ID NO: 21 or a variant thereof; and, a light chain variable region comprising the following three CDRs: CDR-L1 having an amino acid sequence of SEQ ID NO: 22 or a variant thereof, CDR-L2 having an amino acid sequence of SEQ ID NO: 23 or a variant thereof, and CDR-L3 having an amino acid sequence of SEQ ID NO: 24 or a variant thereof;

(1e) a heavy chain variable region comprising the following three CDRs: CDR-H1 having an amino acid sequence of SEQ ID NO: 25 or a variant thereof, CDR-H2 having an amino acid sequence of SEQ ID NO: 26 or a variant thereof, and CDR-H3 having an amino acid sequence of SEQ ID NO: 27 or a variant thereof; and, a light chain variable region comprising the following three CDRs: CDR-L1 having an amino acid sequence of SEQ ID NO: 28 or a variant thereof, CDR-L2 having an amino acid sequence of SEQ ID NO: 29 or a variant thereof, and CDR-L3 having an amino acid sequence of SEQ ID NO: 30 or a variant thereof;

(1f) a heavy chain variable region comprising the following three CDRs: CDR-H1 having an amino acid sequence of SEQ ID NO: 31 or a variant thereof, CDR-H2 having an amino acid sequence of SEQ ID NO: 32 or a variant thereof, and CDR-H3 having an amino acid sequence of SEQ ID NO: 33 or a variant thereof; and, a light chain variable region comprising the following three CDRs: CDR-L1 having an amino acid sequence of SEQ ID NO: 34 or a variant thereof, CDR-L2 having an amino acid sequence of SEQ ID NO: 35 or a variant thereof, and CDR-L3 having an amino acid sequence of SEQ ID NO: 36 or a variant thereof;

(1g) a heavy chain variable region comprising the following three CDRs: CDR-H1 having an amino acid sequence of SEQ ID NO: 37 or a variant thereof, CDR-H2 having an amino acid sequence of SEQ ID NO: 38 or a variant thereof, and CDR-H3 having an amino acid sequence of SEQ ID NO: 39 or a variant thereof; and, a light chain variable region comprising the following three CDRs: CDR-L1 having an amino acid sequence of SEQ ID NO: 40 or a variant thereof, CDR-L2 having an amino acid sequence of SEQ ID NO: 41 or a variant thereof, and CDR-L3 having an amino acid sequence of SEQ ID NO: 42 or a variant thereof;

(1h) a heavy chain variable region comprising the following three CDRs: CDR-H1 having an amino acid sequence of SEQ ID NO: 43 or a variant thereof, CDR-H2 having an amino acid sequence of SEQ ID NO: 44 or a variant thereof, and CDR-H3 having an amino acid sequence of SEQ ID NO: 45 or a variant thereof; and, a light chain variable region comprising the following three CDRs: CDR-L1 having an amino acid sequence of SEQ ID NO: 46 or a variant thereof, CDR-L2 having an amino acid sequence of SEQ ID NO: 47 or a variant thereof, and CDR-L3 having an amino acid sequence of SEQ ID NO: 48 or a variant thereof;

(1i) a heavy chain variable region comprising the following three CDRs: CDR-H1 having an amino acid sequence of SEQ ID NO: 49 or a variant thereof, CDR-H2 having an amino acid sequence of SEQ ID NO: 50 or a variant thereof, and CDR-H3 having an amino acid sequence of SEQ ID NO: 51 or a variant thereof; and, a light chain variable region comprising the following three CDRs: CDR-L1 having an amino acid sequence of SEQ ID NO: 52 or a variant thereof, CDR-L2 having an amino acid sequence of SEQ ID NO: 53 or a variant thereof, and CDR-L3 having an amino acid sequence of SEQ ID NO: 54 or a variant thereof;

(1j) a heavy chain variable region comprising the following three CDRs: CDR-H1 having an amino acid sequence of SEQ ID NO: 55 or a variant thereof, CDR-H2 having an amino acid sequence of SEQ ID NO: 56 or a variant thereof, and CDR-H3 having an amino acid sequence of SEQ ID NO: 57 or a variant thereof; and, a light chain variable region comprising the following three CDRs: CDR-L1 having an amino acid sequence of SEQ ID NO: 58 or a variant thereof, CDR-L2 having an amino acid sequence of SEQ ID NO: 59 or a variant thereof, and CDR-L3 having an amino acid sequence of SEQ ID NO: 60 or a variant thereof;

(1k) a heavy chain variable region comprising the following three CDRs: CDR-H1 having an amino acid sequence of SEQ ID NO: 61 or a variant thereof, CDR-H2 having an amino acid sequence of SEQ ID NO: 62 or a variant thereof, and CDR-H3 having an amino acid sequence of SEQ ID NO: 63 or a variant thereof; and, a light chain variable region comprising the following three CDRs: CDR-L1 having an amino acid sequence of SEQ ID NO: 64 or a variant thereof, CDR-L2 having an amino acid sequence of SEQ ID NO: 65 or a variant thereof, and CDR-L3 having an amino acid sequence of SEQ ID NO: 66 or a variant thereof;

(1l) a heavy chain variable region comprising the following three CDRs: CDR-H1 having an amino acid sequence of SEQ ID NO: 67 or a variant thereof, CDR-H2 having an amino acid sequence of SEQ ID NO: 68 or a variant thereof, and CDR-H3 having an amino acid sequence of SEQ ID NO: 69 or a variant thereof; and, a light chain variable region comprising the following three CDRs: CDR-L1 having an amino acid sequence of SEQ ID NO: 70 or a variant thereof, CDR-L2 having an amino acid sequence of SEQ ID NO: 71 or a variant thereof, and CDR-L3 having an amino acid sequence of SEQ ID NO: 72 or a variant thereof;

(1m) a heavy chain variable region comprising the following three CDRs: CDR-H1 having an amino acid sequence of SEQ ID NO: 73 or a variant thereof, CDR-H2 having an amino acid sequence of SEQ ID NO: 74 or a variant thereof, and CDR-H3 having an amino acid sequence of SEQ ID NO: 75 or a variant thereof; and, a light chain variable region comprising the following three CDRs: CDR-L1 having an amino acid sequence of SEQ ID NO: 76 or a variant thereof, CDR-L2 having an amino acid sequence of SEQ ID NO: 77 or a variant thereof, and CDR-L3 having an amino acid sequence of SEQ ID NO: 78 or a variant thereof;

(1n) a heavy chain variable region comprising the following three CDRs: CDR-H1 having an amino acid sequence of SEQ ID NO: 79 or a variant thereof, CDR-H2 having an amino acid sequence of SEQ ID NO: 80 or a variant thereof, and CDR-H3 having an amino acid sequence of SEQ ID NO: 81 or a variant thereof; and, a light chain variable region comprising the following three CDRs: CDR-L1 having an amino acid sequence of SEQ ID NO: 82 or a variant thereof, CDR-L2 having an amino acid sequence of SEQ ID NO: 83 or a variant thereof, and CDR-L3 with an amino acid sequence of SEQ ID NO: 84 or a variant thereof;

(1o) a heavy chain variable region comprising the following three CDRs: CDR-H1 having an amino acid sequence of SEQ ID NO: 85 or a variant thereof, CDR-H2 having an amino acid sequence of SEQ ID NO: 86 or a variant thereof, and CDR-H3 having an amino acid sequence of SEQ ID NO: 87 or a variant thereof; and, a light chain variable region comprising the following three CDRs: CDR-L1 having an amino acid sequence of SEQ ID NO: 88 or a variant thereof, CDR-L2 having an amino acid sequence of SEQ ID NO: 89 or a variant thereof, and CDR-L3 having an amino acid sequence of SEQ ID NO: 90 or a variant thereof;

(1p) a heavy chain variable region comprising the following three CDRs: CDR-H1 having an amino acid sequence of SEQ ID NO: 91 or a variant thereof, CDR-H2 having an amino acid sequence of SEQ ID NO: 92 or a variant thereof, and CDR-H3 having an amino acid sequence of SEQ ID NO: 93 or a variant thereof; and, a light chain variable region comprising the following three CDRs: CDR-L1 having an amino acid sequence of SEQ ID NO: 94 or a variant thereof, CDR-L2 having an amino acid sequence of SEQ ID NO: 95 or a variant thereof, and CDR-L3 having an amino acid sequence of SEQ ID NO: 96 or a variant thereof; or

(1q) a heavy chain variable region comprising the following three CDRs: CDR-H1 having an amino acid sequence of SEQ ID NO: 97 or a variant thereof, CDR-H2 having an amino acid sequence of SEQ ID NO: 98 or a variant thereof, and CDR-H3 having an amino acid sequence of SEQ ID NO: 99 or a variant thereof; and, a light chain variable region comprising the following three CDRs: CDR-L1 having an amino acid sequence of SEQ ID NO: 100 or a variant thereof, CDR-L2 having an amino acid sequence of SEQ ID NO: 101 or a variant thereof, and CDR-L3 having an amino acid sequence of SEQ ID NO: 102 or a variant thereof.

3. The antibody or antigen-binding fragment thereof according to claim 1 or 2, which further comprises a heavy chain constant region (CH) and a light chain constant region (CL);

preferably, the heavy chain constant region is a rabbit or sheep heavy chain constant region, and the light chain constant region is a rabbit or sheep light chain constant region;
preferably, the heavy chain constant region is a human heavy chain constant region, and the light chain constant region is a human light chain constant region;
preferably, the antibody or antigen-binding fragment thereof is an IgG, IgM, IgE, IgD or IgA antibody;
preferably, the heavy chain constant region is an IgG heavy chain constant region, such as an IgG1, IgG2, IgG3 or IgG4 heavy chain constant region;
preferably, the light chain constant region is a κ or λ light chain constant region (e.g., a human λ light chain constant region).

4. An antibody or antigen-binding fragment thereof that specifically binds to TSH or a mutant thereof, wherein the antibody or antigen-binding fragment thereof is selected from the group consisting of:

produced based on a plasmid in *Escherichia coli* Rosetta™ (DE3) pLysS hTS01 VH deposited at the All-Russian National Collection of Industrial Microorganisms (VKPM) with Accession Number B-14885 and a plasmid in Escherichia coli RosettaTM (DE3) pLysS hTS01 VL deposited at VKPM with Accession Number B-14884;

produced based on a plasmid in *Escherichia coli* Rosetta™ (DE3) pLysS hTS02 VH deposited at the VKPM with Accession Number B-14887 and a plasmid in Escherichia coli RosettaTM (DE3) pLysS hTS02 VL deposited at VKPM with Accession Number B-14886;

produced based on a plasmid in *Escherichia coli* Rosetta™ (DE3) pLysS hTS03 VH deposited at the VKPM with Accession Number B-14889 and a plasmid in Escherichia coli RosettaTM (DE3) pLysS hTS03 VL deposited at VKPM with Accession Number B-14888;

produced based on a plasmid in *Escherichia coli* Rosetta™ (DE3) pLysS hTS04 VH deposited at the VKPM with Accession Number B-14891 and a plasmid in Escherichia coli RosettaTM (DE3) pLysS hTS04 VL deposited at VKPM with Accession Number B-14890;

produced based on a plasmid in *Escherichia coli* Rosetta™ (DE3) pLysS hTS05 VH deposited at the VKPM with Accession Number B-14893 and a plasmid in Escherichia coli RosettaTM (DE3) pLysS hTS05 VL deposited at VKPM with Accession Number B-14892;

produced based on a plasmid in *Escherichia coli* Rosetta™ (DE3) pLysS hTS06 VH deposited at the VKPM with Accession Number B-14895 and a plasmid in Escherichia coli RosettaTM (DE3) pLysS hTS06 VL deposited at VKPM with Accession Number B-14896;

produced based on a plasmid in *Escherichia coli* Rosetta™ (DE3) pLysS hTS07 VH deposited at the VKPM with Accession Number B-14897 and a plasmid in Escherichia coli RosettaTM (DE3) pLysS hTS07 VL deposited at VKPM with Accession Number B-14896;

produced based on a plasmid in *Escherichia coli* Rosetta™ (DE3) pLysS hTS08 VH deposited at the VKPM with Accession Number B-14899 and a plasmid in Escherichia coli RosettaTM (DE3) pLysS hTS08 VL deposited at VKPM with Accession Number B-14898;

produced based on a plasmid in *Escherichia coli* Rosetta™ (DE3) pLysS hTS09 VH deposited at the VKPM with Accession Number B-14901 and a plasmid in Escherichia coli RosettaTM (DE3) pLysS hTS09 VL deposited at VKPM with Accession Number B-14900;

produced based on a plasmid in *Escherichia coli* Rosetta™ (DE3) pLysS hTS10 VH deposited at the VKPM with Accession Number B-14903 and a plasmid in Escherichia coli RosettaTM (DE3) pLysS hTS10 VL deposited at VKPM with Accession Number B-14902;

produced based on a plasmid in *Escherichia coli* Rosetta™ (DE3) pLysS hTS11 VH deposited at the VKPM with Accession Number B-14905 and a plasmid in Escherichia coli RosettaTM (DE3) pLysS hTS11 VL deposited at VKPM with Accession Number B-14904;

produced based on a plasmid in *Escherichia coli* Rosetta™ (DE3) pLysS hTS12 VH deposited at the VKPM with Accession Number B-14907 and a plasmid in Escherichia coli RosettaTM (DE3) pLysS hTS13 VL deposited at VKPM with Accession Number B-14906;

produced based on a plasmid in *Escherichia coli* Rosetta™ (DE3) pLysS hTS13 VH deposited at the VKPM with Accession Number B-14909 and a plasmid in Escherichia coli RosettaTM (DE3) pLysS hTS13 VL deposited at VKPM with Accession Number B-14908;

produced based on a plasmid in *Escherichia coli* Rosetta™ (DE3) pLysS hTS14 VH deposited at the VKPM with Accession Number B-14911 and a plasmid in Escherichia coli RosettaTM (DE3) pLysS hTS14 VL deposited at VKPM with Accession Number B-14910;

produced based on a plasmid in *Escherichia coli* Rosetta™ (DE3) pLysS hTS15 VH deposited at the VKPM with Accession Number B-14913 and a plasmid in Escherichia coli RosettaTM (DE3) pLysS hTS15 VL deposited at VKPM with Accession Number B-14912;

produced based on a plasmid in *Escherichia coli* Rosetta™ (DE3) pLysS hTS16 VH deposited at the VKPM with Accession Number B-14915 and a plasmid in Escherichia coli RosettaTM (DE3) pLysS hTS16 VL deposited at VKPM with Accession Number B-14914; and

produced based on a plasmid in *Escherichia coli* Rosetta™ (DE3) pLysS hTS17 VH deposited at the VKPM with Accession Number B-14917 and a plasmid in Escherichia coli RosettaTM (DE3) pLysS hTS17 VL deposited at VKPM with Accession Number B-14916.

5. The antibody or antigen-binding fragment thereof according to any one of claims 1 to 4, which specifically recognizes a TSH dimer or a β subunit of TSH, and optionally an RSSG mutant of TSH.

6. A TSH detection kit, which is used for detecting the presence or level of TSH or a mutant thereof in a sample;

preferably, the detection functional sensitivity of the detection kit for the TSH or mutant thereof is ≤0.0015 μIU/mL,

preferably ≤0.001 μIU/mL;
preferably, the TSH or mutant thereof is selected from the group consisting of TSH dimer, β subunit of TSH and/or R55G mutant of TSH.

7. The detection kit according to claim 6, which comprises a first reagent and a second reagent, wherein the first reagent comprises magnetic beads coated with a first antibody capable of recognizing the TSH or a mutant thereof; and the second reagent comprises a second antibody connected with a chemiluminescent label, wherein a sandwich complex of the first antibody-an antigen-the second antibody can be formed after the sample is mixed with the first reagent and the second reagent, and the antigen is the TSH or mutant thereof.

8. A composition or TSH detection kit, which comprises the antibody or antigen-binding fragment thereof according to any one of claims 1 to 5.

9. A composition or TSH detection kit, which comprises:

a first antibody, which is an antibody or antigen-binding fragment thereof having a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or, a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof, and,
a second antibody, which is an antibody or antigen-binding fragment thereof having a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or, a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof;
wherein, the first antibody and the second antibody are directed to different epitopes of TSH or a mutant thereof, respectively.

10. The composition or immunoassay kit according to claim 9, wherein:

the first antibody is an antibody or antigen-binding fragment thereof having a heavy chain variable region comprising the following three CDRs: CDR-H1 having an amino acid sequence selected from SEQ ID NOs: 7, 13, 19, 25, 31, 37 and variants thereof, CDR-H2 having an amino acid sequence selected from SEQ ID NOs: 8, 14, 20, 26, 32, 38 and variants thereof, and/or CDR-H3 having an amino acid sequence selected from SEQ ID NOs: 9, 15, 21, 27, 33, 39 and variants thereof; and/or a light chain variable region comprising the following three CDRs: CDR-L1 having an amino acid sequence selected from SEQ ID NOs: 10, 16, 22, 28, 34, 40 and variants thereof, CDR-L2 having an amino acid sequence selected from SEQ ID NOs: 11, 17, 23, 29, 35, 41 and variants thereof, and/or CDR-L3 having an amino acid sequence selected from SEQ ID NOs: 12, 18, 24, 30, 36, 42 and variants thereof, and,
the second antibody is an antibody or antigen-binding fragment thereof having a heavy chain variable region comprising the following three CDRs: CDR-H1 having an amino acid sequence selected from SEQ ID NOs: 1, 43, 49, 55, 61, 67, 73, 79, 85, 91, 97 and variants thereof, CDR-H2 having an amino acid sequence selected from SEQ ID NOs: 2, 44, 50, 56, 62, 68, 74, 80, 86, 92, 98 and variants thereof, and/or CDR-H3 having an amino acid sequence selected from SEQ ID NOs: 3, 45, 51, 57, 63, 69, 75, 81, 87, 93, 99 and variants thereof; and/or, a light chain variable region comprising the following three CDRs: CDR-L1 having an amino acid sequence selected from SEQ ID NOs: 4, 46, 52, 58, 64, 70, 76, 82, 88, 94, 100 and variants thereof, CDR-L2 having an amino acid sequence selected from SEQ ID NOs: 5, 47, 53, 59, 65, 71, 77, 83, 89, 95, 101 and variants thereof, and/or CDR-L3 having an amino acid sequence selected from SEQ ID NOs: 6, 48, 54, 60, 66, 72, 78, 84, 90, 96, 102 and variants thereof.

**11.** The composition or TSH detection kit according to claim 8 or 9, which comprises:

i) a first antibody, which is an antibody or antigen-binding fragment thereof having a heavy chain variable region comprising the following three CDRs: CDR-H1 having an amino acid sequence selected from SEQ ID NO: 1 and variants thereof, CDR-H2 having an amino acid sequence selected from SEQ ID NO: 2 and variants thereof, and CDR-H3 having an amino acid sequence selected from SEQ ID NO: 3 and variants thereof; and, a light chain variable region comprising the following three CDRs: CDR-L1 having an amino acid sequence selected from SEQ ID NO: 4 and variants thereof, CDR-L2 having an amino acid sequence selected from SEQ ID NO: 5 and variants thereof, and CDR-L3 having an amino acid sequence selected from SEQ ID NO: 6 and variants thereof, and, a second antibody, which is an antibody or antigen-binding fragment thereof having a heavy chain variable region comprising the following three CDRs: CDR-H1 having an amino acid sequence selected from SEQ ID NO: 13 and variants thereof, CDR-H2 having an amino acid sequence selected from SEQ ID NO: 14 and variants thereof, and CDR-L3 having an amino acid sequence selected from SEQ ID NO: 15 and variants thereof; and, a light chain variable region comprising the following three CDRs: CDR-L1 having an amino acid sequence selected from SEQ ID NO: 16 and variants thereof, CDR-L2 having an amino acid sequence selected from SEQ ID NO: 17 and variants thereof, and CDR-L3 having an amino acid sequence selected from SEQ ID NO: 18 and variants thereof;

ii) a first antibody, which is an antibody or antigen-binding fragment thereof having a heavy chain variable region comprising the following three CDRs: CDR-H1 having an amino acid sequence selected from SEQ ID NO: 1 and variants thereof, CDR-H2 having an amino acid sequence selected from SEQ ID NO: 2 and variants thereof, and CDR-H3 having an amino acid sequence selected from SEQ ID NO: 3 and variants thereof; and, a light chain variable region comprising the following three CDRs: CDR-L1 having an amino acid sequence selected from SEQ ID NO: 4 and variants thereof, CDR-L2 having an amino acid sequence selected from SEQ ID NO: 5 and variants thereof, and CDR-L3 having an amino acid sequence selected from SEQ ID NO: 6 and variants thereof, and, a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

iii) a first antibody, which is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-L3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof; and,

a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

iv) a first antibody, which is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof, and,

a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-L3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

v) a first antibody, which is an antibody or antigen-binding fragment thereof having a heavy chain variable region comprising the following three CDRs: CDR-H1 having an amino acid sequence selected from SEQ ID NO: 7 and variants thereof, CDR-H2 having an amino acid sequence selected from SEQ ID NO: 8 and variants thereof, and CDR-H3 having an amino acid sequence selected from SEQ ID NO: 9 and variants thereof; and, a light chain variable region comprising the following three CDRs: CDR-L1 having an amino acid sequence selected from SEQ ID NO: 10 and variants thereof, CDR-L2 having an amino acid sequence selected from SEQ ID NO: 11 and variants thereof, and CDR-L3 having an amino acid sequence selected from SEQ ID NO: 12 and variants thereof CDR-L3, and,

a second antibody, which is an antibody or antigen-binding fragment thereof having a heavy chain variable region comprising the following three CDRs: CDR-H1 having an amino acid sequence selected from SEQ ID NO: 55 and variants thereof, CDR-H2 having an amino acid sequence selected from SEQ ID NO: 56 and variants thereof, CDR-H3 having an amino acid sequence selected from SEQ ID NO: 57 and variants thereof; and, a light chain variable region comprising the following three CDRs: CDR-L1 having an amino acid sequence selected from SEQ ID NO: 58 and variants thereof, CDR-L2 having an amino acid sequence selected from SEQ ID NO: 59 and variants thereof, and CDR-L3 having an amino acid sequence selected from SEQ ID NO: 60 and variants thereof;

vi) a first antibody, which is an antibody or antigen-binding fragment thereof having a heavy chain variable region comprising the following three CDRs: CDR-H1 having an amino acid sequence selected from SEQ ID NO: 7 and variants thereof, CDR-H2 having an amino acid sequence selected from SEQ ID NO: 8 and variants thereof, and CDR-H3 having an amino acid sequence selected from SEQ ID NO: 9 and variants thereof; and, a light chain variable region comprising the following three CDRs: CDR-L1 having an amino acid sequence selected from SEQ ID NO: 10 and variants thereof, CDR-L2 having an amino acid sequence selected from SEQ ID NO: 11 and variants thereof, and CDR-L3 having an amino acid sequence selected from SEQ ID NO: 12 and variants thereof, and,

a second antibody, which is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof;

vii) a first antibody, which is an antibody or antigen-binding fragment thereof having a heavy chain variable region comprising the following three CDRs: CDR-H1 having an amino acid sequence selected from SEQ ID NO: 13 and variants thereof, CDR-H2 having an amino acid sequence selected from SEQ ID NO: 14 and variants thereof, and CDR-H3 having an amino acid sequence selected from SEQ ID NO: 15 and variants thereof; and, a light chain variable region comprising the following three CDRs: CDR-L1 having an amino acid sequence selected from SEQ ID NO: 16 and variants thereof, CDR-L2 having an amino acid sequence selected from SEQ ID NO: 17 and variants thereof, and CDR-L3 having an amino acid sequence selected from SEQ ID NO: 18 and variants thereof; and,

a second antibody, which is an antibody or antigen-binding fragment thereof having a heavy chain variable region comprising the following three CDRs: CDR-H1 having an amino acid sequence selected from SEQ ID NO: 55 and variants thereof, CDR-H2 having an amino acid sequence selected from SEQ ID NO: 56 and variants thereof, and CDR-H3 having an amino acid sequence selected from SEQ ID NO: 57 and variants thereof; and, a light chain variable region comprising the following three CDRs: CDR-L1 having an amino acid sequence selected from SEQ ID NO: 58 and variants thereof, CDR-L2 having an amino acid sequence selected from SEQ ID NO: 59 and variants thereof, and CDR-L3 having an amino acid sequence selected from SEQ ID NO: 60 and variants thereof;

viii) a first antibody, which is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 having an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: 23 and variants thereof, and CDR-L3 having an amino acid sequence selected from SEQ ID NO: 24 and variants thereof;

and,

a second antibody, which is an antibody or antigen-binding fragment thereof having a heavy chain variable region comprising the following three CDRs: CDR-H1 having an amino acid sequence selected from SEQ ID NO: 85 and variants thereof, CDR-H2 having an amino acid sequence selected from SEQ ID NO: 86 and variants thereof, and CDR-H3 having an amino acid sequence selected from SEQ ID NO: 87 and variants thereof; and, a light chain variable region comprising the following three CDRs: CDR-L1 having an amino acid sequence selected from SEQ ID NO: 88 and variants thereof, CDR-L2 having an amino acid sequence selected from SEQ ID NO: 89 and variants thereof, and CDR-L3 having an amino acid sequence selected from SEQ ID NO: 90 and variants thereof;

ix) a first antibody, which is an antibody or antigen-binding fragment thereof having a heavy chain variable region comprising the following three CDRs: CDR-H1 having an amino acid sequence selected from SEQ ID NO: 67 and variants thereof, CDR-H2 having an amino acid sequence selected from SEQ ID NO: 68 and variants thereof, and CDR-H3 having an amino acid sequence selected from SEQ ID NO: 69 and variants thereof; and, a light chain variable region comprising the following three CDRs: CDR-L1 having an amino acid sequence selected from SEQ ID NO: 70 and variants thereof, CDR-L2 having an amino acid sequence selected from SEQ ID NO: 71 and variants thereof, and CDR-L3 having an amino acid sequence selected from SEQ ID NO: 72 and variants thereof; and,

a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

x) a first antibody, which is an antibody or antigen-binding fragment thereof having a heavy chain variable region comprising the following three CDRs: CDR-H1 having an amino acid sequence selected from SEQ ID NO: 79 and variants thereof, CDR-H2 having an amino acid sequence selected from SEQ ID NO: 80 and variants thereof, and CDR-H3 having an amino acid sequence selected from SEQ ID NO: 81 and variants thereof; and, a light chain variable region comprising the following three CDRs: CDR-L1 having an amino acid sequence selected from SEQ ID NO: 82 and variants thereof, CDR-L2 having an amino acid sequence selected from SEQ ID NO: 83 and variants thereof, and CDR-L3 having an amino acid sequence selected from SEQ ID NO: 84 and variants thereof; and,

a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof;

xi) a first antibody, which is an antibody or antigen-binding fragment thereof having a heavy chain variable region comprising the following three CDRs: CDR-H1 having an amino acid sequence selected from SEQ ID NO: 25 and variants thereof, CDR-H2 having an amino acid sequence selected from SEQ ID NO: 26 and variants thereof, and CDR-H3 having an amino acid sequence selected from SEQ ID NO: 27 and variants thereof; and, a light chain variable region comprising the following three CDRs: CDR-L1 having an amino acid sequence selected from SEQ ID NO: 28 and variants thereof, CDR-L2 having an amino acid sequence selected from SEQ ID NO: 29 and variants thereof, and CDR-L3 having an amino acid sequence selected from SEQ ID NO: 30 and variants thereof; and,

a second antibody, which is an antibody or antigen-binding fragment thereof having a heavy chain variable region comprising the following three CDRs: CDR-H1 having an amino acid sequence selected from SEQ ID NO: 73 and variants thereof, CDR-H2 having an amino acid sequence selected from SEQ ID NO: 74 and variants thereof, and CDR-L3 having an amino acid sequence selected from SEQ ID NO: 75 and variants thereof; and, a light chain variable region comprising the following three CDRs: CDR-L1 having an amino acid sequence selected from SEQ ID NO: 76 and variants thereof, CDR-L2 having an amino acid sequence selected from SEQ ID NO: 77 and variants thereof, and CDR-L3 having an amino acid sequence selected from SEQ ID NO: 78 and variants thereof;

xii) a first antibody, which is an antibody or antigen-binding fragment thereof having a heavy chain variable region comprising the following three CDRs: CDR-H1 having an amino acid sequence selected from SEQ ID NO: 25 and variants thereof, CDR-H2 having an amino acid sequence selected from SEQ ID NO: 26 and variants thereof, and CDR-H3 having an amino acid sequence selected from SEQ ID NO: 27 and variants thereof; and, a light chain variable region comprising the following three CDRs: CDR-L1 having an amino acid sequence selected from SEQ ID NO: 28 and variants thereof, CDR-L2 having an amino acid sequence selected from SEQ ID NO: 29 and variants thereof, and CDR-L3 having an amino acid sequence selected from SEQ ID NO: 30 and variants thereof; and,

a second antibody, which is an antibody or antigen-binding fragment thereof having a heavy chain variable region comprising the following three CDRs: CDR-H1 having an amino acid sequence selected from SEQ ID NO: 85 and variants thereof, CDR-H2 having an amino acid sequence selected from SEQ ID NO: 86 and variants thereof, and CDR-H3 having an amino acid sequence selected from SEQ ID NO: 87 and variants thereof; and, a light chain variable region comprising the following three CDRs: CDR-L1 having an amino acid sequence selected from SEQ ID NO: 88 and variants thereof, CDR-L2 having an amino acid sequence selected from SEQ ID NO: 89 and variants thereof, and CDR-L3 having an amino acid sequence selected from SEQ ID NO: 90 and variants thereof;

xiii) a first antibody, which is an antibody or antigen-binding fragment thereof having a heavy chain variable region comprising the following three CDRs: CDR-H1 having an amino acid sequence selected from SEQ ID NO: 37 and variants thereof, CDR-H2 having an amino acid sequence selected from SEQ ID NO: 38 and variants thereof, and CDR-H3 having an amino acid sequence selected from SEQ ID NO: 39 and variants thereof; and, a light chain variable region comprising the following three CDRs: CDR-L1 having an amino acid sequence selected from SEQ ID NO: 40 and variants thereof, CDR-L2 having an amino acid sequence selected from SEQ ID NO: 41 and variants thereof, and CDR-L3 having an amino acid sequence selected from SEQ ID NO: 42 and variants thereof; and,

a second antibody, which is an antibody or antigen-binding fragment thereof having a heavy chain variable region comprising the following three CDRs: CDR-H1 having an amino acid sequence selected from SEQ ID NO: 55 and variants thereof, CDR-H2 having an amino acid sequence selected from SEQ ID NO: 56 and variants thereof, and CDR-H3 having an amino acid sequence selected from SEQ ID NO: 57 and variants thereof; and, a light chain variable region comprising the following three CDRs: CDR-L1 having an amino acid sequence selected from SEQ ID NO: 58 and variants thereof, CDR-L2 having an amino acid sequence selected from SEQ ID NO: 59 and variants thereof, and CDR-L3 having an amino acid sequence selected from SEQ ID NO: 60 and variants thereof;

xiv) a first antibody, which is an antibody or antigen-binding fragment thereof having a heavy chain variable region comprising the following three CDRs: CDR-H1 having an amino acid sequence selected from SEQ ID NO: 91 and variants thereof, CDR-H2 having an amino acid sequence selected from SEQ ID NO: 92 and variants thereof, and CDR-H3 having an amino acid sequence selected from SEQ ID NO: 93 and variants thereof; and, a light chain variable region comprising the following three CDRs: CDR-L1 having an amino acid sequence selected from SEQ ID NO: 94 and variants thereof, CDR-L2 having an amino acid sequence selected from SEQ ID NO: 95 and variants thereof, and CDR-L3 having an amino acid sequence selected from SEQ ID NO: 96 and variants thereof, and,

a second antibody, which is an antibody or antigen-binding fragment thereof having a heavy chain variable region comprising the following three CDRs: CDR-H1 having an amino acid sequence selected from SEQ ID NO: 13 and variants thereof, CDR-H2 having an amino acid sequence selected from SEQ ID NO: 14 and variants thereof, and CDR-H3 having an amino acid sequence selected from SEQ ID NO: 15 and variants thereof; and, a light chain variable region comprising the following three CDRs: CDR-L1 having an amino acid sequence selected from SEQ ID NO: 16 and variants thereof, CDR-L2 having an amino acid sequence selected from SEQ ID NO: 17 and variants thereof, and CDR-L3 having an amino acid sequence selected from SEQ ID NO: 18 and variants thereof; or,

xv) a first antibody, which is an antibody or antigen-binding fragment thereof having a heavy chain variable region comprising the following three CDRs: CDR-H1 having an amino acid sequence selected from SEQ ID NO: 91 and variants thereof, CDR-H2 having an amino acid sequence selected from SEQ ID NO: 92 and variants thereof, and CDR-H3 having an amino acid sequence selected from SEQ ID NO: 93 and variants thereof; and, a light chain variable region comprising the following three CDRs: CDR-L1 having an amino acid sequence selected from SEQ ID NO: 94 and variants thereof, CDR-L2 having an amino acid sequence selected from SEQ ID NO: 95 and variants thereof, and CDR-L3 having an amino acid sequence selected from SEQ ID NO: 96 and variants thereof, and,

a second antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof.

12. The composition or TSH detection kit according to claim 8 or 9, which comprises:

i) a first antibody, which is an antibody or antigen-binding fragment thereof having a heavy chain variable region comprising the following three CDRs: CDR-H1 having an amino acid sequence selected from SEQ ID NO: 31 and variants thereof, CDR-H2 having an amino acid sequence selected from SEQ ID NO: 32 and variants thereof, and CDR-H3 having an amino acid sequence selected from SEQ ID NO: 33 and variants thereof; and, a light chain variable region comprising the following three CDRs: CDR-L1 having an amino acid sequence selected from SEQ ID NO: 34 and variants thereof, CDR-L2 having an amino acid sequence selected from SEQ ID NO: 35 and variants thereof, and CDR-L3 having an amino acid sequence selected from SEQ ID NO: 36 and variants thereof; and

a second antibody, which is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof;

ii) a first antibody, which is an antibody or antigen-binding fragment thereof having a heavy chain variable region comprising the following three CDRs: CDR-H1 having an amino acid sequence selected from SEQ ID NO: 37 and variants thereof, CDR-H2 having an amino acid sequence selected from SEQ ID NO: 38 and variants thereof, and CDR-H3 having an amino acid sequence selected from SEQ ID NO: 39 and variants thereof; and, a light chain variable region comprising the following three CDRs: CDR-L1 having an amino acid sequence selected from SEQ ID NO: 40 and variants thereof, CDR-L2 having an amino acid sequence selected from SEQ ID NO: 41 and variants thereof, and CDR-L3 having an amino acid sequence selected from SEQ ID NO: 42 and variants thereof; and,

a second antibody, which is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-H3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof;

iii) a first antibody, which is an antibody or antigen-binding fragment thereof having a heavy chain variable region comprising the following three CDRs: CDR-H1 having an amino acid sequence selected from SEQ ID NO: 25 and variants thereof, CDR-H2 having an amino acid sequence selected from SEQ ID NO: 26 and variants thereof, and CDR-L3 having an amino acid sequence selected from SEQ ID NO: 27 and variants thereof; and, a light chain variable region comprising the following three CDRs: CDR-L1 having an amino acid sequence selected from SEQ ID NO: 28 and variants thereof, CDR-L2 having an amino acid sequence selected from SEQ ID NO: 29 and variants thereof, and CDR-L3 having an amino acid sequence selected from SEQ ID NO: 30 and variants thereof; and,

a second antibody, which is an antibody or antigen-binding fragment thereof having a heavy chain variable region comprising the following three CDRs: CDR-H1 having an amino acid sequence selected from SEQ ID NO: 49 and variants thereof, CDR-H2 having an amino acid sequence selected from SEQ ID NO: 50 and variants thereof, and CDR-H3 having an amino acid sequence selected from SEQ ID NO: 51 and variants thereof; and, a light chain variable region comprising the following three CDRs: CDR-L1 having an amino acid sequence selected from SEQ ID NO: 52 and variants thereof, CDR-L2 having an amino acid sequence selected from SEQ ID NO: 53 and variants thereof, and CDR-L3 having an amino acid sequence selected from SEQ ID NO: 54 and variants thereof;

iv) a first antibody, which is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 having an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: 23 and variants thereof, and CDR-L3 having an amino acid sequence selected from SEQ ID NO: 24 and variants thereof; and,

a second antibody, which is an antibody or antigen-binding fragment thereof having a heavy chain variable region comprising the following three CDRs: CDR-H1 having an amino acid sequence selected from SEQ ID NO: 61 and variants thereof, CDR-H2 having an amino acid sequence selected from SEQ ID NO: 62 and variants thereof, and CDR-H3 having an amino acid sequence selected from SEQ ID NO: 63 and variants thereof; and, a light chain variable region comprising the following three CDRs: CDR-L1 having an amino acid sequence selected from SEQ ID NO: 64 and variants thereof, CDR-L2 having an amino acid sequence selected from SEQ ID NO: 65 and variants thereof, and CDR-L3 having an amino acid sequence selected from SEQ ID NO: 66 and variants thereof; or,

v) the first antibody, which is an antibody or antigen-binding fragment thereof having a heavy chain variable region comprising the following three CDRs: CDR-H1 having an amino acid sequence selected from SEQ ID NO: 19 and variants thereof, CDR-H2 having an amino acid sequence selected from SEQ ID NO: 20 and variants thereof, and CDR-H3 having an amino acid sequence selected from SEQ ID NO: 21 and variants thereof; and, a light chain variable region comprising the following three CDRs: CDR-L1 having an amino acid sequence selected from SEQ ID NO: 22 and variants thereof, CDR-L2 having an amino acid sequence selected from SEQ ID NO: 23 and variants thereof, and CDR-L3 having an amino acid sequence selected from SEQ ID NO: 24 and variants thereof; and,

a second antibody, which is an antibody or antigen-binding fragment thereof having a heavy chain variable region comprising the following three CDRs: CDR-H1 having an amino acid sequence selected from SEQ ID NO: 43 and variants thereof, CDR-H2 having an amino acid sequence selected from SEQ ID NO: 44 and variants thereof, and CDR-H3 having an amino acid sequence selected from SEQ ID NO: 45 and variants thereof; and, a light chain variable region comprising the following three CDRs: CDR-L1 having an amino acid sequence selected from SEQ ID NO: 46 and variants thereof, CDR-L2 having an amino acid sequence selected from SEQ ID NO: 47 and variants thereof, and CDR-L3 having an amino acid sequence selected from SEQ ID NO: 48 and variants thereof.

13. The composition or TSH detection kit according to any one of claims 9 to 12, wherein the first antibody is a capture antibody and the second antibody is a detection antibody.

14. Use of the antibody or antigen-binding fragment thereof according to any one of claims 1 to 7 in the manufacture of a kit or use of the immunoassay kit according to any one of claims 11 to 20, wherein the kit is used for:

(1) detection of the presence or level of TSH or a mutant thereof in a sample;
(2) diagnosis or auxiliary diagnosis of a thyroid dysfunction (e.g., hypothyroidism or hyperthyroidism);
(3) diagnosis or auxiliary diagnosis of a thyroid-related disease; and/or
(4) evaluation or auxiliary evaluation of a therapeutic effect on a thyroid-related disease; preferably, the thyroid-related disease is a hyperthyroidism or a thyroid cancer.

15. A sample analyzer, comprising:

a loading device, which is configured to load a sample to be tested and a reagent required for chemiluminescent reaction into a reaction container, wherein the reagent comprises an antibody or antigen-binding fragment thereof that specifically binds to TSH or a mutant thereof;
a reaction incubation device, which is configured to provide a reaction and incubation site for the reaction container containing the sample to be tested and the reagent required for chemiluminescent reaction, so that the sample to be tested and the reagent required for chemiluminescent reaction in the reaction container form a sample liquid to be tested; and
a photometric device, which is configured to perform photometry on the sample liquid to be tested; wherein, the antibody or the antigen-binding fragment thereof comprises:

a heavy chain variable region comprising the following three CDRs: CDR-H1 having an amino acid sequence selected from SEQ ID NOs: 1, 7, 13, 19, 25, 31, 37, 43, 49, 55, 61, 67, 73, 79, 85, 91, 97 and variants thereof, CDR-H2 having an amino acid sequence selected from SEQ ID NOs: 2, 8, 14, 20, 26, 32, 38, 44, 50, 56, 62, 68, 74, 80, 86, 92, 98 and variants thereof, and/or CDR-H3 having an amino acid sequence selected from SEQ ID NOs: 3, 9, 15, 21, 27, 33, 39, 45, 51, 57, 63, 69, 75, 81, 87, 93, 99 and variants thereof; and/or, a light chain variable region comprising the following three CDRs: CDR-L1 having an amino acid sequence selected from SEQ ID NOs: 4, 10, 16, 22, 28, 34, 40, 46, 52, 58, 64, 70, 76, 82, 88, 94, 100 and variants thereof, CDR-L2 having an amino acid sequence selected from SEQ ID NOs: 5, 11, 17, 23, 29, 35, 41, 47, 53, 59, 65, 71, 77, 83, 89, 95, 101 and variants thereof, and/or CDR-L3 having an amino acid sequence selected from SEQ ID NOs: 6, 12, 18, 24, 30, 36, 42, 48, 54, 60, 66, 72, 78, 84, 90, 96, 102 and variants thereof; the photometric device comprises:

a receiving component, which is configured to receive a photosignal emitted by the sample to be tested through the chemiluminescent reaction and convert the photosignal into a corresponding electrical signal; and
a processing component, which is configured to be electrically connected to the receiving component and receive the electrical signal from the receiving component, wherein the processing component comprises a first photon counting module and a second photon counting module, the first photon counting module is configured to detect the number of pulses of the electrical signal using a pulse recognition method to obtain a first photon counting result, the second photon counting module is configured to process the electrical signal to obtain a parameter for characterizing photon number, and calculate a second photon counting result according to the parameter for characterizing photon number and a preset calibration function, wherein the calibration function represents a mapping relationship between the parameter for characterizing photon number and the photon counting result, and the processing component is further configured to output a final photon counting result of the sample to be tested based on the first photon counting result and the second photon counting result.

16. The sample analyzer according to claim 15, **characterized in that** the processing component is further configured to: when the first photon counting result is lower than a first threshold, output the first photon counting result as the final photon counting result; when the first photon counting result is higher than a second threshold, output the second photon counting result as the final photon counting result, wherein the second threshold is greater than or equal to the first threshold.

17. The sample analyzer according to claim 15 or 16, **characterized in that** the second photon counting module comprises an integration circuit and a second counting circuit electrically connected to each other, the integration circuit is configured to integrate the electrical signal within a predetermined time period to obtain a DC component signal, the parameter for characterizing photon number comprises a parameter related to the DC component signal, and the second counting circuit is configured to calculate the second photon counting result according to the parameter related to the DC component signal and a preset calibration function.

Figure 1

Figure 2

Figure 3

Figure 4

Figure 5

Figure 6

Figure 7

Figure 8

$y = 0.947x + 0.0517$
$R^2 = 0.9947$
$n=719$

TSH kit of Example 1 (uIU/mL)

Commercial TSH kit (uIU/mL)

**Figure 9**

$y = 0.9361x + 0.1099$
$R^2 = 0.9935$
$n=719$

TSH kit of Example 2 (uIU/mL)

Commercial TSH kit (uIU/mL)

**Figure 10**

**Figure 11**

**Figure 12**

y = 1.2131x - 0.4228

R² = 0.9866

n=719

**Figure 13**

**Figure 14**

**Figure 15**

**Figure 16**

**Figure 17**

Figure 18

Figure 19

Figure 20

Figure 21

**Figure 22**

**Figure 23**

**Figure 24**

**Figure 25**

**Figure 26**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4816567 P, Cabilly
- US 5225539 A
- US 5530101 A, Queen
- US 5585089 A
- US 5693762 A
- US 6180370 B

### Non-patent literature cited in the description

- **DREES JC et al.** Falsely undetectable TSH in a cohort of South Asian euthyroid patients. *J Clin Endocrinol Metab.*, April 2014, vol. 99 (4), 1171-9
- Fundamental Immunology. Raven Press, 1989
- **KABAT**. Sequences of Proteins of Immunological Interest. National Institutes of Health, Bethesda, 1987
- **CHOTHIA** ; **LESK**. J. Mol. Biol.. 1987, vol. 196, 901-917
- **CHOTHIA et al.** *Nature*, 1989, vol. 342, 878-883
- **KABAT et al.** Sequences of Proteins of Immunological Interest. Public Health Service, National Institutes of Health, 1991
- **LEFRANC et al.** *Dev. Comparat. Immunol.*, 2003, vol. 27, 55-77
- **HOLLIGER et al.** *Nat Biotechnol*, 2005, vol. 23, 1126-1136
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **PLUCKTHUN**. The Pharmacology of Monoclonal Antibodies. Springer-Verlag, 1994, vol. 113, 269-315
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol*, 2001
- **HOLLIGER P. et al.** *Proc. Natl. Acad. Sci. USA*, 1993, vol. 90, 6444-6448
- **POLJAK R. J. et al.** *Structure*, 1994, vol. 2, 1121-1123
- **MORRISON et al.** *Proc. Natl. Acad. Sci. USA*, 1984, vol. 81, 6851-6855
- **KABAT, E.A. et al.** Sequences of Proteins of Immunological Interest. U.S. Department of Health and Human Services, 1991
- Antibody Engineering: Methods and Protocols. Humana Press, 2004, vol. 248
- **NEEDLEMAN et al.** *J. Mol. Biol.*, 1970, vol. 48, 443-453
- **E. MEYERS** ; **W. MILLER**. *Comput. Appl Biosci.*, 1988, vol. 4, 11-17
- **NEEDLEMAN** ; **WUNSCH**. *J Mol Biol.*, 1970, vol. 48, 444-453
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl Acad. Set USA*, 1997, vol. 94, 412-417
- Immunology-A Synthesis. Sinauer Associates, 1991